# EUROPÄISCHE PATENTANMELDUNG

(11) **EP 1 655 280 A1**
(43) Veröffentlichungstag der Anmeldung: **10.05.2006**
(21) Anmeldenummer: 06002721.6
(22) Anmeldetag: 31.08.2000
(51) Int. Cl.: C07C 69/76, C07C 69/34, C07C 69/738, C07C 69/734, C07C 69/94, C07C 255/57, C07C 309/73, C07C 323/62, C07C 65/28, C07C 63/66, C07C 65/24, C07C 63/331, C07F 9/54, A61K 31/194, A61K 31/235, A61K 31/277, A61K 31/66, A61P 9/00

(54) **Neuartige Dicarbonsäurederivate mit pharmazeutischen Eigenschaften**

(30) Priorität: 13.09.1999 DE 19943634
(62) Teilanmeldung aus: 00962413.1
(71) Anmelder: Bayer HealthCare AG, 51368 Leverkusen (DE)
(72) Erfinder: Alonso-Alija, Christina, Dr., 42781 Haan (DE); Heil, Markus, Dr., 42799 Leichlingen (DE); Flubacher, Dietmar, Dr., 79189 Krozingen (DE); Naab, Paul, Dr., 42287 Wuppertal (DE); Stasch, Johannes-Peter, Dr., 42651 Solingen (DE); Wunder, Frank, Dr., 42117 Wuppertal (DE); Dembowsky, Klaus, Dr., 81249 München (DE); Perzborn, Elisabeth, Dr., 42327 Wuppertal (DE); Stahl, Elke, Dr., 51467 Bergisch Gladbach (DE)

(57) **Zusammenfassung**

Die vorliegende Erfindung betrifft Verbindungen der Formel (I) 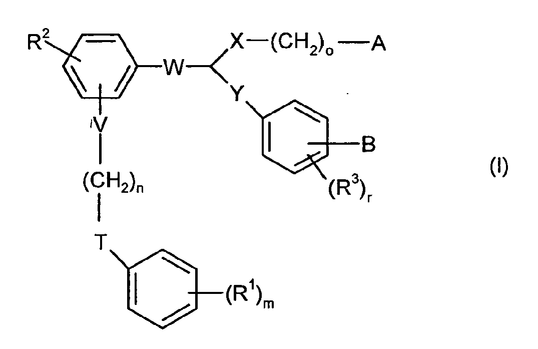 sowie deren Salze und Stereoisomere, zur Herstellung von Arzneimitteln zur Behandlung von Herz-Kreislauf-Erkrankungen.

## Beschreibung

Die vorliegende Erfindung betrifft neue chemische Verbindungen, welche die lösliche Guanylatcyclase auch über einen neuartigen, ohne Beteiligung der Häm-Gruppe des Enzyms verlaufenden Wirkmechanismus stimulieren, ihre Herstellung und ihre Verwendung als Arzneimittel, insbesondere als Arzneimittel zur Behandlung von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriposphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch CO ist in der Lage, am Eisen-Zentralatom des Häms anzugreifen, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion und der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Atherosklerose, Angina pectoris, Herzinsuffizienz, Thrombosen, Schlaganfall und Myokardinfarkt führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein viel versprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriffe am Eisenzentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxymethyl-2'-furyl)-1-benzylindazol (YC-1, Wu et al., Blood 84 (1994), 4226; Mülsch et al., Br.J.Pharmacol. 120 (1997), 681), Fettsäuren (Goldberg et al, J. Biol. Chem. 252 (1977), 1279), Diphenyliodonium-hexafluorophosphat (Pettibone et al., Eur. J. Pharmacol. 116 (1985), 307), Isoliquiritigenin (Yu et al., Brit. J. Pharmacol. 114 (1995), 1587), sowie verschiedene substituierte Pyrazolderivate (WO 98/16223, WO 98/16507 und WO 98/23619).

Die bisher bekannten Stimulatoren der löslichen Guanylatcyclase stimulieren das Enzym entweder direkt über die Häm-Gruppe (Kohlenmonoxid, Stickstoffmonoxid oder Diphenyliodoniumhexafluorophosphat) durch Interaktion mit dem Eisenzentrum der Häm-Gruppe und eine sich daraus ergebende, zur Erhöhung der Enzymaktivität führende Konformationsänderung (Gerzer et al., FEBS Lett. 132(1981), 71), oder über einen Häm-abhängigen Mechanismus, der unabhängig von NO ist, aber zu einer Potenzierung der stimulierenden Wirkung von NO oder CO führt (z.B. YC-1, Hoenicka et al., J. Mol. Med. (1999) 14; oder die in der WO 98/16223, WO 98/16507 und WO 98/23619 beschriebenen Pyrazolderivate).

Die in der Literatur behauptete stimulierende Wirkung von Isoliquiritigenin und von Fettsäuren, wie z. B. Arachidonsäure, Prostaglandinendoperoxide und Fettsäurehydroperoxide auf die lösliche Guanylatcyclase konnte nicht bestätigt werden (vgl. z.B. Hoenicka et al., J. Mol. Med. 77 (1999), 14).

Entfernt man von der löslichen Guanylatcyclase die Häm-Gruppe, zeigt das Enzym immer noch eine nachweisbare katalytische Basalaktivität, d.h. es wird nach wie vor cGMP gebildet. Die verbleibende katalytische Basalaktivität des Häm-freien Enzyms ist durch keinen der vorstehend genannten bekannten Stimulatoren stimulierbar.

Es wurde eine Stimulation von Häm-freier löslicher Guanylatcyclase durch Protoporphyrin IX beschrieben (Ignarro et al., Adv. Pharmacol. 26 (1994), 35). Allerdings kann Protoporphyrin IX als Mimik für das NO-Häm-Addukt angesehen werden, weshalb die Zugabe von Protoporphyrin IX zur löslichen Guanylatcyclase zur Bildung einer der durch NO stimulierten Häm-haltigen löslichen Guanylatcyclase entsprechenden Struktur des Enzyms führen dürfte. Dies wird auch durch die Tatsache belegt, dass die stimulierende Wirkung von Protoporphyrin IX durch den vorstehend beschriebenen NO-unabhängigen, aber Häm-abhängigen Stimulator YC-1 erhöht wird (Mülsch et al., Naunyn Schmiedebergs Arch. Pharmacol. 355, R47).

Bislang wurden somit keine Verbindungen beschrieben, welche die lösliche Guanylatcyclase unabhängig von der im Enzym befindlichen Häm-Gruppe stimulieren können.

Es war die Aufgabe der vorliegenden Erfindung, Arzneimittel zur Behandlung von Herz-Kreislauferkrankungen oder anderen über eine Beeinflussung des cGMP-Signalweges in Organismen therapierbaren Erkrankungen zu entwickeln.

Die vorstehende Aufgabe wird durch die Verwendung von Verbindungen zur Herstellung von Arzneimitteln gelöst, welche in der Lage sind, die lösliche Guanylatcyclase auch unabhängig von NO und von der im Enzym befindlichen Häm-Gruppe zu stimulieren.

Überraschend wurde gefunden, dass es Verbindungen gibt, welche die lösliche Guanylatcyclase auch unabhängig von der im Enzym befindlichen Häm-Gruppe stimulieren können. Die biologische Aktivität dieser Stimulatoren beruht auf einem völlig neuen Mechanismus der Stimulierung der löslichen Guanylatcyclase. Im Gegensatz zu den vorstehend beschriebenen, aus dem Stand der Technik als Stimulatoren der löslichen Guanylatcyclase bekannten Verbindungen sind die erfindungsgemäßen Verbindungen in der Lage, sowohl die Häm-haltige als auch die Häm-freie Form der löslichen Guanylatcyclase zu stimulieren. Die Stimulierung des Enzyms verläuft bei diesen neuen Stimulatoren also über einen Häm-unabhängigen Weg, was auch dadurch belegt wird, dass die neuen Stimulatoren am Häm-haltigen Enzym einerseits keine synergistische Wirkung mit NO zeigen und andererseits sich die Wirkung dieser neuartigen Stimulatoren nicht durch den Häm-abhängigen Inhibitor der löslichen Guanylatcyclase, 1*H*-1,2,4-Oxadiazol-(4,3a)-chinoxalin-1-on (ODQ), blockieren lässt.

Dies stellt einen neuen Therapieansatz zur Behandlung von Herz-Kreislauferkrankungen und anderen über eine Beeinflussung des cGMP-Signalweges in Organismen therapierbaren Erkrankungen dar.

Gemäß einer bevorzugten Ausführungsform der vorliegenden Erfindung werden zur von der im Enzym befindlichen Häm-Gruppe unabhängigen Stimulation der löslichen Guanylatcyclase Alkan- oder Alkensäurederivate eingesetzt.

In der EP-A-0 341 551 sind Alkan- und Alkensäurederivate wie beispielsweise (1) beschrieben, die potente Leukotrien-Antagonisten sind und daher beispielsweise als Medikamente zur Behandlung von Asthma oder Durchblutungsstörungen geeignet sind (S. 18, Z. 56-58). Eine stimulierende Wirkung dieser Verbindungen auf die lösliche Guanylatcyclase und die sich daraus ergebende Verwendung dieser Verbindungen zur Herstellung von Arzneimitteln, welche den cGMP-Signalweg beeinflussen können, ist jedoch nicht beschrieben.

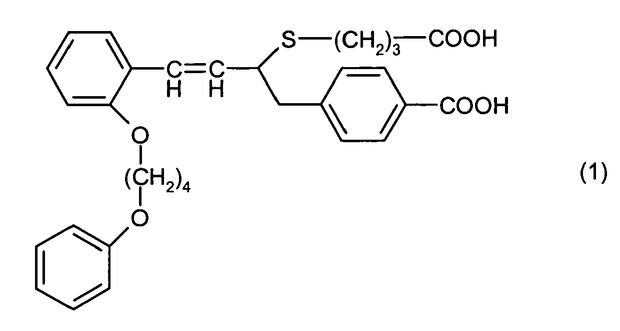

In der EP-A-0 410 241 sind weitere Alkan- und Alkensäurederivate wie beispielsweise (2) mit LTD₄-, LTC₄- oder LTE₄-antagonistischer Wirkung beschrieben.

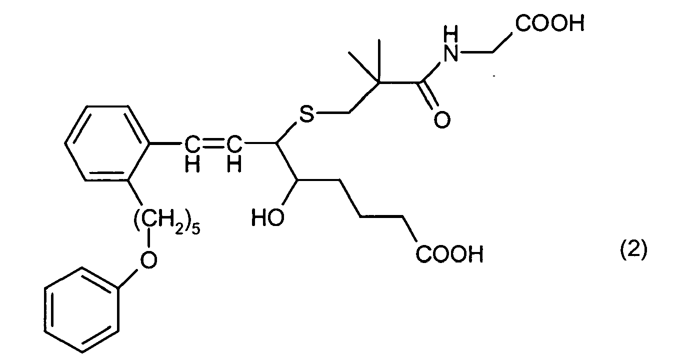

In der EP-A-0 494 621 sind schwefelhaltige Alkensäurederivate wie beispielsweise (3) beschrieben, welche bei allergischen Erkrankungen, Entzündungen und Herz-Kreislauf-Erkrankungen eingesetzt werden können.

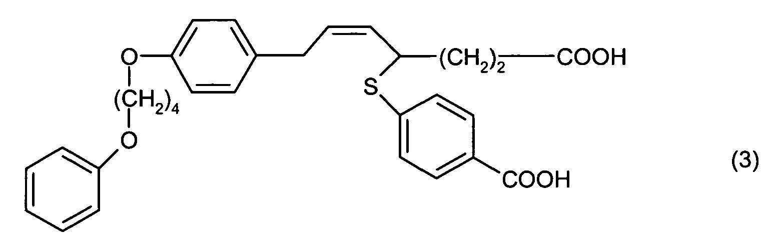

In der EP-A-0 791 576 sind Benzoesäurederivate wie beispielsweise (4) beschrieben, welche zur Behandlung von Atemwegserkrankungen verwendet werden können.

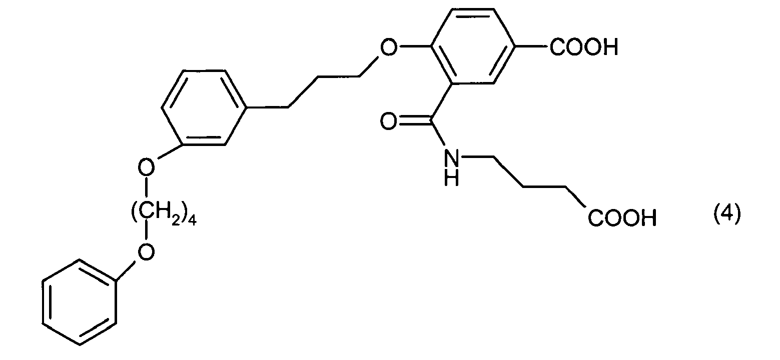

Es ist jedoch nicht beschrieben, dass irgendeine der vorstehend genannten, aus dem Stand der Technik bekannten Verbindungen eine stimulierende Wirkung auf die lösliche Guanylatcyclase besitzt und somit zur Behandlung von Erkrankungen eingesetzt werden könnte, welche durch Beeinflussung des cGMP-Spiegels therapierbar sind.

Die vorliegende Erfindung betrifft in einer bevorzugten Ausführungsform Verbindungen der allgemeinen Formel (I) 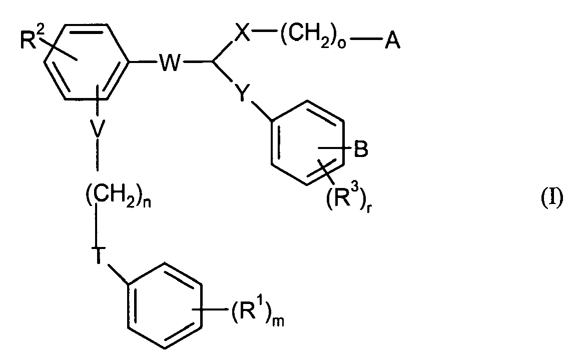 worin
- V: fehlt oder O bedeutet,
- n: eine ganze Zahl von 1 bis 10 bedeutet,
- T: fehlt oder O bedeutet,
- R¹: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,

- m: 1 oder 2 bedeutet,
- R²: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
- W: CH₂CH₂ oder CH=CH bedeutet, wenn W an dem Phenylring in ortho-Position zu dem Rest V-(CH₂)ₙ-T-Ph-(R¹)ₘ angeordnet ist,
mit der Maßgabe, dass W nicht CH=CH ist, wenn gleichzeitig T=V=O, R¹=R²=R³=H, n=4, Y=CH₂, A und B gleichzeitig COOH oder COOCH₃ sind, X fehlt oder S und o gleich 3 oder 4 ist,
beziehungsweise CH₂CH₂CH₂ oder CH₂CH=CH bedeutet, wenn W an dem Phenylring in meta-Position zu dem Rest V-(CH₂)ₙT-Ph-(R¹)ₘ angeordnet ist,
mit der Maßgabe, dass W nicht CH₂CH=CH ist, wenn entweder gleichzeitig T=V=O, R'=H oder F, m=1, R²=R³=H, n=3, Y=CH₂, A und B gleichzeitig COOH oder COOCH₃, X fehlt oder S und o gleich 3 oder 4 ist, oder gleichzeitig T fehlt oder O, V fehlt, R¹=R²=R³=H, n gleich 4 oder 5, Y=CH₂, A und B gleichzeitig COOH oder COOCH₂CH₃,
- X: fehlt, und o=4 ist,
X fehlt, geradkettiges oder verzweigtes Alkylen mit bis zu 6 Kohlenstoffatomen, O, SCH₂ oder S(O)p bedeutet,
worin
p 0, 1 oder 2 bedeutet
o eine ganze Zahl von 1 bis 5 bedeutet
A Tetrazolyl, Tetrazolylmethylen, COOH, CH₂COOH, COOR⁴, CH₂COOR⁵, CONR⁶R⁷ oder CN bedeutet,
worin
R⁴ und R⁵ unabhängig voneinander geradkettiges oder verzweigtes Alkyl mit bis zu 6 Kohlenstoffatomen bedeuten,
R⁶ und R⁷ unabhängig voneinander Wasserstoff, geradkettiges oder verzweigtes Alkyl mit bis zu 6 Kohlenstoffatomen, geradkettiges oder ver- zweigtes Alkylsulfonyl mit bis zu 12 Kohlenstoffatomen, Arylsulfonyl mit 6 bis 12 Kohlenstoffatomen bedeuten,
oder
R⁶ und R⁷ zusammen mit dem Stickstoffatom, an welches sie gebunden sind, einen 3- bis 8-gliedrigen gesättigten Heterocyclus bilden
Y fehlt, geradkettiges oder verzweigtes Alkylen mit bis zu 6 Kohlenstoffatomen, O, SCH₂ oder S(O)q bedeutet,
worin
q 0, 1 oder 2 bedeutet
B Tetrazolyl, Tetrazolylmethylen, COOH, CH₂COOH, COOR⁸, CH₂COOR⁹, CONR¹⁰R¹¹ oder CN bedeutet,
worin
R⁸ und R⁹ unabhängig voneinander geradkettiges oder verzweigtes Alkyl mit bis zu 6 Kohlenstoffatomen bedeuten,
R¹⁰ und R¹¹ unabhängig voneinander Wasserstoff, geradkettiges oder verzweigtes Alkyl mit bis zu 6 Kohlenstoffatomen, geradkettiges oder verzweigtes Alkylsulfonyl mit bis zu 12 Kohlenstoffatomen, Arylsulfonyl mit 6 bis 12 Kohlenstoffatomen bedeuten
oder
R¹⁰ und R¹¹ zusammen mit dem Stickstoffatom, an welches sie gebunden sind, einen 3- bis 8-gliedrigen gesättigten Heterocyclus bilden,
R³ Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
r 0, 1 oder 2 bedeutet,
sowie deren Salze und Stereoisomere.

Die erfindungsgemäßen Verbindungen der allgemeinen Formel (I) können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, p-Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweilige Mischungen. Die Racemformen lassen sich ebenso wie die Diastereomeren in bekannter Weise, beispielsweise durch chromatographische Trennung, in die stereoisomer einheitlichen Bestandteile trennen. In den erfindungsgemäßen Verbindungen vorhandene Doppelbindungen können in der cis- oder trans-Konfiguration (Z- oder E-Form) vorliegen.

Im Rahmen der vorliegenden Erfindung haben die Substituenten soweit nicht anders angegeben im allgemeinen die folgende Bedeutung:
Alkyl steht im allgemeinen für einen geradkettigen oder verzweigten Kohlenwasserstoffrest mit 1 bis 20 Kohlenstoffatomen. Beispielsweise seien Methyl, Ethyl, Propyl, Isopropyl, Butyl, Isobutyl, Pentyl, Isopentyl, Hexyl, Isohexyl, Heptyl, Isoheptyl, Octyl und Isooctyl, Nonyl, Decyl, Dodeyl, Eicosyl genannt.
Alkylen steht im allgemeinen für eine geradkettige oder verzweigte Kohlenwasserstoffbrücke mit 1 bis 20 Kohlenstoffatomen. Beispielsweise seien Methylen, Ethylen, Propylen, α-Methylethylen, β-Methylethylen, α-Ethylethylen, β-Ethylethylen, Butylen, α-Methylpropylen, β-Methylpropylen, γ-Methylpropylen, α-Ethylpropylen, β-Ethylpropylen, γ-Ethylpropylen, Pentylen, Hexylen, Heptylen, Octylen, Nonylen, Decylen, Dodeylen und Eicosylen genannt.
Alkenyl steht im allgemeinen für einen geradkettigen oder verzweigten Kohlenwasserstoffrest mit 2 bis 20 Kohlenstoffatomen und einer oder mehreren, bevorzugt mit einer oder zwei Doppelbindungen. Beispielsweise seien Allyl, Propenyl, Isopropenyl, Butenyl, Isobutenyl, Pentenyl, Isopentenyl, Hexenyl, Isohexenyl, Heptenyl, Isoheptenyl, Octenyl, Isooctenyl genannt.
Alkinyl steht im allgemeinen für einen geradkettigen oder verzweigten Kohlenwasserstoffrest mit 2 bis 20 Kohlenstoffatomen und einer oder mehreren, bevorzugt mit einer oder zwei Dreifachbindungen. Beispielsweise seien Ethinyl, 2-Butinyl, 2-Pentinyl und 2-Hexinyl benannt.
Acyl steht im allgemeinen für geradkettiges oder verzweigtes Niedrigalkyl mit 1 bis 9 Kohlenstoffatomen, das über eine Carbonylgruppe gebunden ist. Beispielsweise seien genannt: Acetyl, Ethylcarbonyl, Propylcarbonyl, Isopropylcarbonyl, Butylcarbonyl und Isobutylcarbonyl.
Alkoxy steht im allgemeinen für einen über einen Sauerstoffatom gebundenen geradkettigen oder verzweigten Kohlenwasserstoffrest mit 1 bis 14 Kohlenstoffatomen. Beispielsweise seien Methoxy, Ethoxy, Propoxy, Isopropoxy, Butoxy, Isobutoxy, Pentoxy Isopentoxy, Hexoxy, Isohexoxy, Heptoxy, Isoheptoxy, Octoxy oder Isooctoxy genannt. Die Begriffe "Alkoxy" und "Alkyloxy" werden synonym verwendet.
Alkoxyalkyl steht im allgemeinen für einen Alkylrest mit bis zu 8 Kohlenstoffatomen, der durch einen Alkoxyrest mit bis zu 8 Kohlenstoffatomen substituiert ist.
Alkoxycarbonyl kann beispielsweise durch die Formel 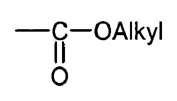 dargestellt werden.
Alkyl steht hierbei im allgemeinen für einen geradkettigen oder verzweigten Kohlenwasserstoffrest mit 1 bis 13 Kohlenstoffatomen. Beispielsweise seien die folgenden Alkoxycarbonylreste genannt: Methoxycarbonyl, Ethoxycarbonyl, Propoxycarbonyl, Isopropoxycarbonyl, Butoxycarbonyl oder Isobutoxycarbonyl.
Cycloalkyl steht im allgemeinen für einen cyclischen Kohlenwasserstoffrest mit 3 bis 8 Kohlenstoffatomen. Bevorzugt sind Cyclopropyl, Cyclopentyl und Cyclohexyl. Beispielsweise seien Cyclopentyl, Cyclohexyl, Cycloheptyl und Cyclooctyl genannt.
Cycloalkoxy steht im Rahmen der Erfindung für einen Alkoxyrest, dessen Kohlenwasserstoffrest ein Cycloalkylrest ist. Der Cycloalkylrest hat im allgemeinen bis zu 8 Kohlenstoffatome. Als Beispiele seien genannt: Cyclopropyloxy und Cyclohexyloxy. Die Begriffe "Cycloalkoxy" und "Cycloalkyloxy" werden synonym verwendet.
Aryl steht im allgemeinen für einen aromatischen Rest mit 6 bis 10 Kohlenstoffatomen. Bevorzugte Arylreste sind Phenyl und Naphthyl.
Halogen steht im Rahmen der Erfindung für Fluor, Chlor, Brom und Iod.
Heterocyclus steht im Rahmen der Erfindung im allgemeinen für einen gesättigten, ungesättigten oder aromatischen 3- bis 10-gliedrigen, beispielsweise 5- oder 6-gliedrigen Heterocyclus, der bis zu 3 Heteroatome aus der Reihe S, N und/oder O enthalten kann und der im Fall eines Stickstoffatoms auch über dieses gebunden sein kann. Beispielsweise seien genannt: Oxadiazolyl, Thiadiazolyl, Pyrazolyl, Pyridyl, Pyrimidinyl, Pyridazinyl, Pyrazinyl, Thienyl, Furyl, Pyrrolyl, Pyrrolidinyl, Piperazinyl, Tetrahydropyranyl, Tetrahydrofuranyl, 1,2,3 Triazolyl, Thiazolyl, Oxazolyl, Imidazolyl, Morpholinyl oder Piperidyl. Bevorzugt sind Thiazolyl, Furyl, Oxazolyl, Pyrazolyl, Triazolyl, Pyridyl, Pyrimidinyl, Pyridazinyl und Tetrahydropyranyl. Der Begriff "Heteroaryl" (bzw. "Hetaryl") steht für einen aromatischen heterocyclischen Rest.

Erfindungsgemäß bevorzugt sind Verbindungen der Formel (I), worin
- W: CH₂CH₂ oder CH=CH bedeutet und an dem Phenylring in ortho-Position zu dem Rest V-(CH₂)ₙ-T-Ph-(R¹)ₘ angeordnet ist,
mit der Maßgabe, dass W nicht CH=CH ist, wenn gleichzeitig T=V=O, R¹=R²=R³=H, n=4, Y=CH₂, A und B gleichzeitig COOH oder COOCH₃ sind, X fehlt oder S und o gleich 3 oder 4 ist,
und die anderen Substituenten wie vorstehend definiert sind.

Erfindungsgemäß weiterhin bevorzugt sind Verbindungen der Formel (I), worin
- W: CH₂CH₂CH₂ oder CH₂CH=CH bedeutet und an dem Phenylring in meta-Position zu dem Rest V-(CH₂)ₙ-T-Ph-(R¹)ₘ angeordnet ist,
mit der Maßgabe, dass W nicht CH₂CH=CH ist, wenn entweder gleichzeitig T=V=O, R'=H oder F, m=1, R²=R³=H, n=3, Y=CH₂, A und B gleichzeitig COOH oder COOCH₃, X fehlt oder S und o gleich 3 oder 4 ist, oder gleichzeitig T fehlt oder O, V fehlt,
R¹=R²=R³=H, n gleich 4 oder 5, Y=CH₂, A und B gleichzeitig COOH oder COOCH₂CH₃, X fehlt, und o=4 ist,
und die anderen Substituenten wie vorstehend definiert sind.

Besonders bevorzugt sind Verbindungen der Formel (I),
worin
- V: fehlt oder O bedeutet,
- n: eine ganze Zahl von 1 bis 10 bedeutet,
- T: fehlt oder O bedeutet,
- R¹: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
- m: 1 oder 2 bedeutet,
- R²: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
- W: CH₂CH₂ oder CH=CH bedeutet und an dem Phenylring in ortho-Position zu dem Rest V-(CH₂)ₙ-T-Ph-(R¹)ₘ angeordnet ist,
mit der Maßgabe, dass W nicht CH=CH ist, wenn gleichzeitig T=V=O, R¹=R²=H, n=4 und A und B gleichzeitig COOH oder COOCH₃ sind,
- X: fehlt,
- o: eine ganze Zahl von 1 bis 4 bedeutet,
- A: COOH oder COOR⁴ bedeutet,
worin
R⁴ Alkyl mit bis zu 2 Kohlenstoffatomen bedeuten,
- Y: O, S, SO, SO₂ oder CH₂ bedeutet,
- B: COOH , COOR⁸ oder CN bedeutet,
worin
R⁸ Alkyl mit bis zu 2 Kohlenstoffatomen bedeutet,
- R³: Wasserstoff, geradkettiges oder verzweigtes Alkoxy mit bis zu 6 Kohlenstoffatomen, F Cl, Br oder I bedeutet,
- r: 0, 1 oder 2 bedeutet.

Ebenfalls besonders bevorzugt sind Verbindungen der Formel (I),
worin
- V: fehlt oder O bedeutet,
- n: eine ganze Zahl von 1 bis 6 bedeutet,
- T: fehlt oder O bedeutet,
- R¹: Wasserstoff, geradkettiges oder verzweigtes Alkyl oder geradkettiges oder verzweigtes Alkoxy mit jeweils bis zu 6 Kohlenstoffatomen, F, Cl, Br oder CF₃ bedeutet,
- m: 1 oder 2 bedeutet,
- R²: Wasserstoff oder geradkettiges oder verzweigtes Alkyl mit bis zu 6 Kohlenstoffatomen bedeutet,
- W: CH₂CH₂ oder CH=CH bedeutet und an dem Phenylring in ortho-Position zu dem Rest V-(CH₂)ₙ-T-Ph-(R¹)ₘ angeordnet ist,
mit der Maßgabe, dass W nicht CH=CH ist, wenn gleichzeitig T=V=O, R¹=R²=H, n=4, und A und B gleichzeitig COOH oder COOCH₃ sind,
- X: fehlt,
- o: eine ganze Zahl von 1 bis 4 bedeutet,
- A: COOH oder COOR⁴ bedeutet,
worin
R⁴ Alkyl mit bis zu 2 Kohlenstoffatomen bedeuten,
- Y: O, S oder CH₂ bedeutet,
- B: COOH , COOR⁸ oder CN bedeutet,
worin
R⁸ Alkyl mit bis zu 2 Kohlenstoffatomen bedeutet,
- R³: Wasserstoff, geradkettiges oder verzweigtes Alkoxy mit bis zu 4 Kohlenstoffatomen, Cl oder Br bedeutet;
- r: 0, 1 oder 2 bedeutet.

Insbesondere bevorzugt sind Verbindungen der Formel (I),
worin
- V: fehlt oder O bedeutet,
- n: eine ganze Zahl von 1 bis 10 bedeutet,
- T: fehlt oder O bedeutet,
- R¹: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
- m: 1 oder 2 bedeutet,
- R²: Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
- W: CH₂CH₂CH₂ oder CH₂CH=CH bedeutet und an dem Phenylring in meta-Position zu dem Rest V-(CH₂)ₙ-T-Ph-(R¹)ₘ angeordnet ist,
mit der Maßgabe, dass W nicht CH₂CH=CH ist, wenn entweder gleichzeitig T=V=O, R'=H oder F, m=1, R²=H, n=3, und A und B gleichzeitig COOH oder COOCH₃ ist, oder gleichzeitig T fehlt oder O, V fehlt, R¹=R²=H, n gleich 4 oder 5, A und B gleichzeitig COOH oder COOCH₂CH₃, und o=4 ist,
- X: fehlt,
- o: 3 oder 4 bedeutet,
- A: COOH oder COOR⁴ bedeutet,
worin
R⁴ Alkyl mit bis zu 2 Kohlenstoffatomen bedeuten,
- Y: CH₂ bedeutet,
- B: COOH , COOR⁸ oder CN bedeutet,
worin
R⁸ Alkyl mit bis zu 2 Kohlenstoffatomen bedeutet,
- R³: Wasserstoff bedeutet.

Ebenfalls insbesondere bevorzugt sind Verbindungen der Formel (I),
worin
- V: fehlt oder O bedeutet,
- n: eine ganze Zahl von 1 bis 6 bedeutet,
- T: fehlt oder O bedeutet,
- R¹: Wasserstoff, geradkettiges oder verzweigtes Alkyl oder geradkettiges oder verzweigtes Alkoxy mit jeweils bis zu 6 Kohlenstoffatomen, F, Cl, Br oder CF₃ bedeutet,
- m: 1 oder 2 bedeutet,
- R²: Wasserstoff, geradkettiges oder verzweigtes Alkyl mit bis zu 6 Kohlenstoffatomen, F, Cl, Br oder CF₃ bedeutet,
- W: CH₂CH₂CH₂ oder CH₂CH=CH bedeutet und an dem Phenylring in meta-Position zu dem Rest V-(CH₂)ₙ-T-Ph-(R¹)ₘ angeordnet ist,
mit der Maßgabe, dass W nicht CH₂CH=CH ist, wenn entweder gleichzeitig T=V=O, R¹=H oder F, m=1, R²=H, n=3, und A und B gleichzeitig COOH oder COOCH₃ ist, oder gleichzeitig T fehlt oder O, V fehlt, R¹=R²=H, n gleich 4 oder 5, A und B gleichzeitig COOH oder COOCH₂CH₃, und o=4 ist,
- X: fehlt,
- o: 3 oder 4 bedeutet,
- A: COOH oder COOR⁴ bedeutet,
worin
R⁴ Alkyl mit bis zu 2 Kohlenstoffatomen bedeuten,
- Y: CH₂ bedeutet,
- B: COOH , COOR⁸ oder CN bedeutet,
worin
R⁸ Alkyl mit bis zu 2 Kohlenstoffatomen bedeutet,
- R³: Wasserstoff bedeutet.

Erfindungsgemäß ganz besonders bevorzugt sind Verbindungen der Formel (I), bei denen A und B jeweils für COOH stehen und die restlichen Substituenten wie in Anspruch 3 definiert sind.

Weiterhin sind erfindungsgemäß Verbindungen besonders bevorzugt, bei denen V für O steht und T fehlt und die restlichen Substituenten wie in Anspruch 3 definiert sind.

Weiterhin sind erfindungsgemäß Verbindungen bevorzugt, bei denen V und T fehlen, n für eine ganze Zahl von 1 bis 3 steht und die restlichen Substituenten wie in Anspruch 3 definiert sind.

Die vorliegende Erfindung betrifft weiterhin ein Verfahren zur Herstellung der erfindungsgemäßen Verbindungen der Formel (I) 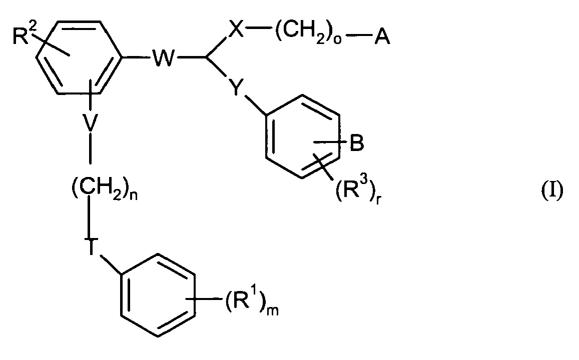 worin
R¹, R², R³, A,B, T, V, W, X, Y, m, n, o und r die vorstehend angegebene Bedeutung haben, umfassend

[α] die Umsetzung von Aldehyden der allgemeinen Formel (II) 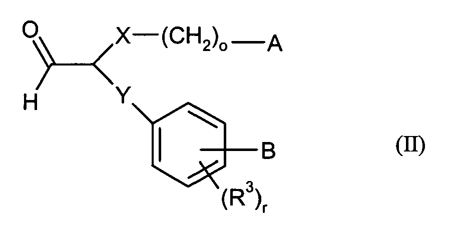 worin
- R³, A, B, X, Y, o und r: die vorstehend angegebene Bedeutung haben, mit der Maßgabe, dass A und B nicht für freie Carbonsäuregruppen stehen dürfen,
mit Phosphorverbindungen der allgemeinen Formel (III) 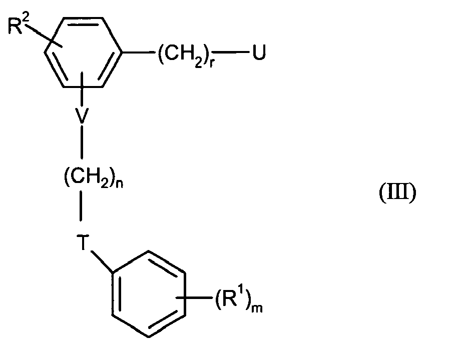 worin
- R¹, R², T, V, m und n: die vorstehend angegebenen Bedeutungen haben,
- r: 1 oder 2 bedeutet, und
- U: für einen Rest der Formel 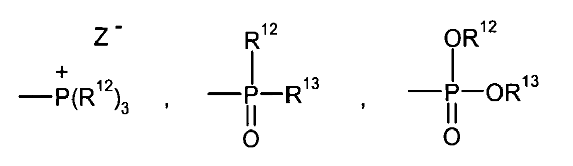
steht, worin
- R¹² und R¹³: unabhängig voneinander geradkettiges oder verzweigtes Alkyl mit bis zu 12 Kohlenstoffatomen oder Phenyl bedeuten, und
- Z: ein Halogenidanion oder Tosylatanion bedeutet,
in inerten Lösungsmitteln in Gegenwart einer Base,
und gegebenenfalls die anschließende teilweise oder vollständige Hydrolyse der Reste A und B zu freien Carbonsäuregruppen;
oder

[β] die Umsetzung von Aldehyden der Formel (i) 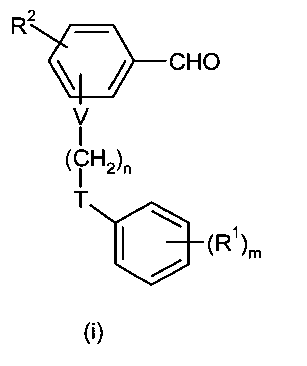 worin
R¹, R², T, V, m und n die vorstehend angegebenen Bedeutungen haben,
mit Phosphorverbindungen der Formel (ii) 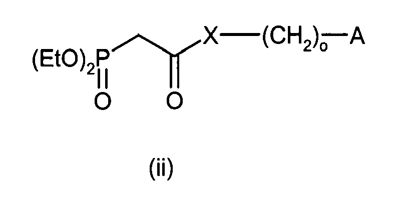 worin
- X, o und A: die vorstehend angegebenen Bedeutungen haben,
zu Verbindungen der Formel (iii) 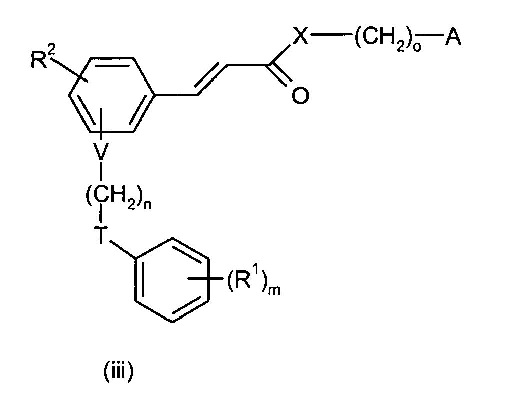 worin
R¹, R², T, V, m, n, X, o und A die vorstehend angegebenen Bedeutungen haben,
und die anschließende Überführung der Verbindungen der Formel (iii) in Verbindungen der Formel (iv) 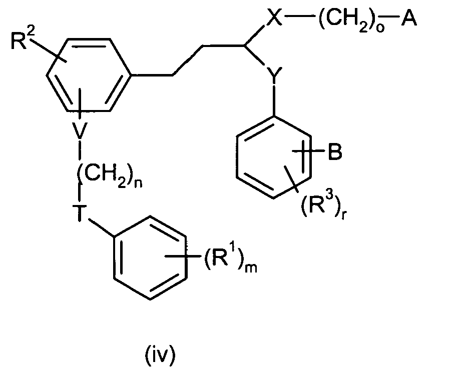 worin
- R¹, R², T, V, m, n, X, o, r, A, B und R³: die vorstehend angegebenen Bedeutungen haben,
- Y: O, SCH₂ oder S bedeutet,
durch aufeinander folgende Reduktion der Carbonylgruppe und der Alkengruppe und anschließende Substitution der durch Reduktion der Carbonylgruppe erzeugten Hydroxygruppe mit Alkoholen oder Thiolen sowie gegebenenfalls anschließende Oxidation zu den entsprechenden Sulfoxid- oder Sulfonverbindungen.

Gemäß der vorliegenden Erfindung bedeutet Z bevorzugt ein Halogenidanion, insbesondere bevorzugt Chlorid, Bromid oder Iodid.

Gemäß der vorliegenden Erfindung erfolgt die gegebenenfalls durchzuführende teilweise oder vollständige Hydrolyse zu den entsprechenden freien Carbonsäuregruppen vorzugsweise mit starken Säuren wie z.B. HCl oder mit starken Basen wie z.B. NaOH oder LiOH, die in wässriger Lösung oder Lösungsmittelgemischen aus Wasser mit Alkoholen wie z.B. Methanol oder Ethern vorliegen.

Für die erfindungsgemäßen Verfahren bevorzugte inerte Lösungsmittel sind herkömmliche organische Lösungsmittel, welche sich unter den Reaktionsbedingungen nicht verändern. Vorzugsweise können für das erfindungsgemäße Verfahren Ether wie Diethylether, Butylmethylether, Dioxan, Tetrahydrofuran, Glykoldimethylether oder Diethylenglykoldimethylether, oder Kohlenwasserstoffe wie Benzol, Toluol, Xylol oder Petrolether, oder Amide wie Dimethylformamid oder Hexamethylphosphortriamid, oder 1,3-Dimethyl-imidazolidin-2-on, 1,3-Dimethyl-tetrahydropyrimidin-2-on oder Dimethylsulfoxid verwendet werden. Es ist selbstverständlich auch möglich, Gemische der vorstehend genannten Lösungsmittel zu verwenden.

Für die erfindungsgemäßen Verfahren bevorzugte Basen umfassen herkömmlicherweise für basische Reaktionen eingesetzte basische Verbindungen. Vorzugsweise können Alkalimetallhydride wie beispielsweise Natriumhydrid oder Kaliumhydrid, oder Alkalimetallalkoholate wie Natriummethanolat, Natriumethanolat, Kaliummethanolat, Kaliumethanolat oder Kalium-t.-butylat, oder Amide wie Natriumamid oder Lithiumdiisopropylamid oder Natriumhexamethyldisilazan, oder Organolithium-Verbindungen wie Phenyllithium, Butyllithium oder Methyllithium verwendet werden. Gegebenenfalls kann beim erfindungsgemäßen Verfahren zur Optimierung der Reaktion ein herkömmlicher Kronenether wie 18-Krone-6 zugegeben werden.

Die Wahl des Lösungsmittels oder Base hängt von der Stabilität, Empfindlichkeit gegenüber Hydrolyse oder der CH-Aktivität der entsprechenden Phosphorverbindung ab. Ether wie Diethylether, Tetrahydrofuran, Dimethoxyethan oder Dioxan, zusammen mit einem Co-Lösungsmittel wie Dimethylformamid oder 1,3-Dimethyltetrahydropyridin-2-on oder 1,3-Dimethylimidazolidin-2-on, werden als Lösungsmittel besonders bevorzugt verwendet. Alkalimetallalkoholate wie Kalium-t-butylat oder Organolithiumverbindungen wie Phenyllithum oder Butyllithium oder Natriumhydrid werden als Basen besonders bevorzugt verwendet.

Die Reaktion kann im allgemeinen in einem Temperaturbereich von -80°C bis +70°C, vorzugsweise von -80°C bis +20°C ausgeführt werden.

Die Reaktion kann bei Normaldruck, erhöhtem oder verringertem Druck ausgeführt werden (beispielsweise in einem Bereich von 0,5 bis 5 bar). Im allgemeinen wird die Reaktion bei Normaldruck ausgeführt.

Bei der Durchführung der Reaktion werden die Phosphorverbindungen im allgemeinen in einer Menge von 1 bis 2 mol, bezogen auf 1 mol Aldehyd eingesetzt. Die Basen werden im allgemeinen in einer Menge von 1 bis 5 mol, vorzugsweise von 1 bis 2 mol, bezogen auf 1 mol Phosphorverbindung eingesetzt.

Das erfindungsgemäße Verfahren [α] kann beispielsweise durchgeführt werden, indem die Base und anschließend das Aldehyd, gegebenenfalls in einem Lösungsmittel, zu der in einem Lösungsmittel gelösten oder suspendierten Phosphorverbindung zugegeben werden, und anschließend gegebenenfalls das Gemisch erhitzt wird. Die Aufarbeitung erfolgt auf herkömmliche Weise, durch Extraktion, Chromatographie und/oder Kristallisation.

Bei der Durchführung des erfindungsgemäßen Verfahrens [α] ist es auch möglich, anstelle der vorstehend genannten Phosphoniumsalze die entsprechenden Phosphorane (U ist gleich - P(R¹²)₃=CHR) zu verwenden, die vorher in einer getrennten Reaktion aus den entsprechenden Phosphoniumsalzen im basischen Milieu dargestellt wurden. Es hat sich aber als vorteilhaft erwiesen, die Reaktion mit den Phosphorverbindungen in Gegenwart von Basen als Eintopf-Verfahren durchzuführen.

Die Phosphorverbindungen der allgemeinen Formel (III) können auf folgenden verschiedenen Wegen hergestellt werden.

### Verfahren A - 1. Variante

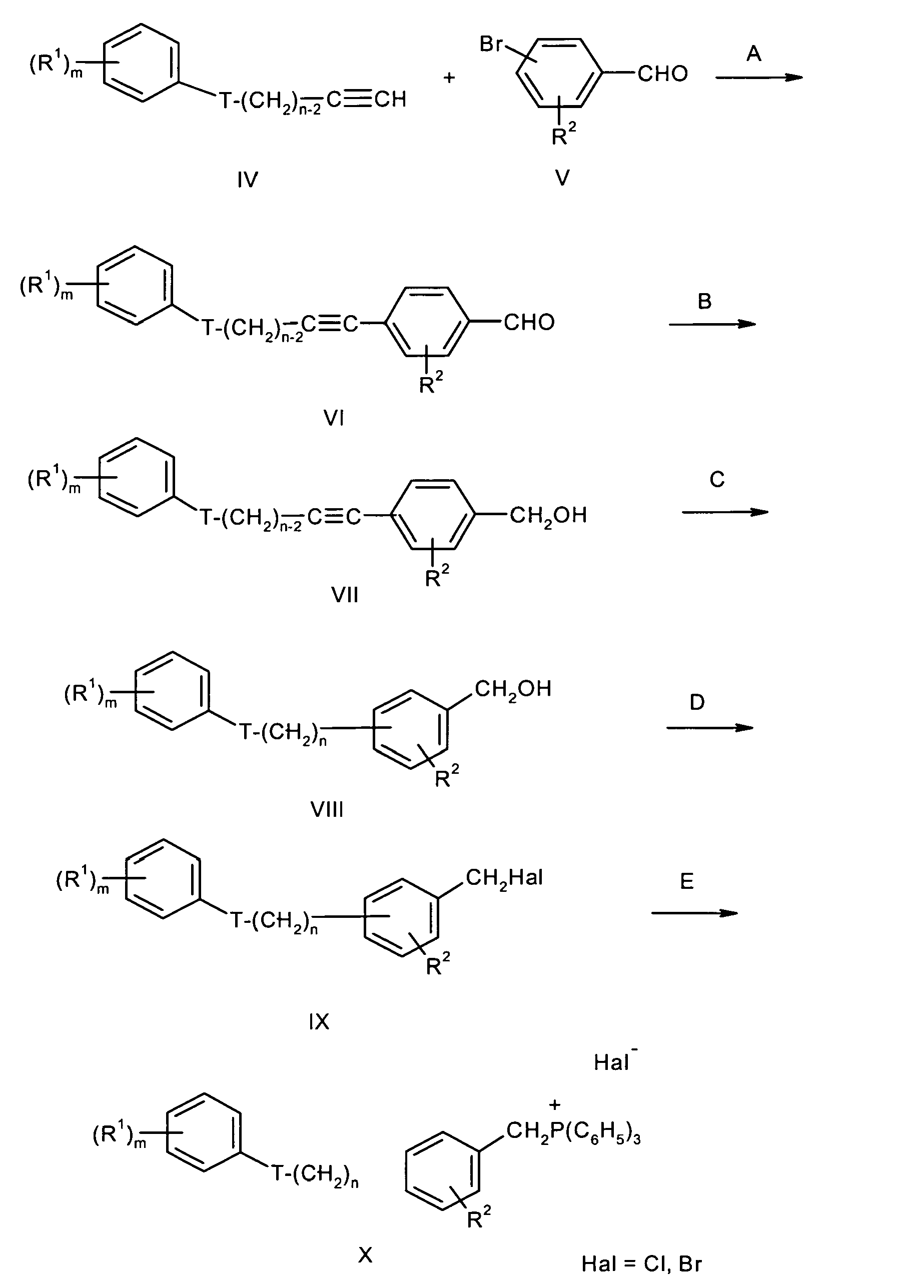

Beim ersten Reaktionsschritt [A] dieser Variante werden die Acetylenverbindungen (IV) mit den Brombenzaldehyden (V) in Lösungsmitteln wie Triethylamin, Acetonitril, Pyridin oder Gemischen davon, vorzugsweise in Triethylamin, in Gegenwart von Kupfer-(I)-Salzen und Palladium-(0)-Verbindungen, vorzugsweise in Gegenwart von Kupfer-(I)-Halogeniden wie beispielsweise Kupferiodid, und bis-(Triphenylphosphan)-Palladium-(II)-chlorid in einem Temperaturbereich von -40°C bis +80°C, vorzugsweise von 0°C bis +40°C umgesetzt.

Im zweiten Reaktionsschritt [B] wird die Formylverbindung (VI) in Lösungsmitteln wie Alkoholen, beispielsweise Methanol, Ethanol, Propanol oder Isopropanol, oder Ethern wie Diethylether, Tetrahydrofuran oder Dioxan, oder in basischen Lösungsmitteln wie Triethylamin, Pyridin oder Dimethylformamid, oder in Wasser oder in Gemischen aus den vorstehend genannten Lösungsmitteln unter Verwendung komplexierter Hydride wie beispielsweise Borhydriden oder Aluminiumhydriden, vorzugsweise Natriumborhydrid oder Lithiumaluminiumhydrid, als Reduktionsmittel in einem Temperaturbereich von -40°C bis +60°C, vorzugsweise von 0°C bis +40°C, zu den Hydroxylverbindungen (VII) reduziert.

Im dritten Reaktionsschritt [C] werden die Verbindungen (VII) in inerten Lösungsmitteln wie Alkoholen, beispielsweise Methanol, Ethanol, Propanol oder Isopropanol, oder Kohlenwasserstoffen wie Benzol, Toluol oder Xylol oder in Ethern wie Diethylether oder Tetrahydrofuran, oder in Ethylacetat, insbesondere bevorzugt in Methanol, in Gegenwart von Edelmetall-Katalysatoren wie Palladium oder Platin in einem Temperaturbereich von -30°C bis +80°C, vorzugsweise von 0°C bis +40°C, unter einem Druck von 1 bar bis 50 bar, vorzugsweise von 1 bar bis 20 bar hydriert.

Die Schritte B und C können auch in umgekehrter Reihenfolge ausgeführt werden.

Im vierten Schritt [D] werden die hydrierten Verbindungen (VIII) durch Umsetzung mit Bromierungsmitteln wie beispielsweise Phosphortribromid, Sulfonylbromid, Bromwasserstoff oder Tetrabrommethan/Diphenylphosphan in inerten Lösungsmitteln wie Ether, beispielsweise Diethylether oder Tetrahydrofuran, oder Kohlenwasserstoffen wie Benzol oder Toluol oder besonders bevorzugt chlorierten Kohlenwasserstoffen wie Methylenchlorid oder Chloroform, in einem Temperaturbereich von -20°C bis +60°C, vorzugsweise von 0°C bis +40°C bromiert. Es können aber auch die entsprechenden Chlorverbindungen verwendet werden, die beispielsweise durch Umsetzung der Verbindungen VIIIa mit SOCl₂ erhältlich sind.

Im fünften Reaktionsschritt [E] werden die bromierten oder chlorierten Verbindungen (IX) mit Triphenylphosphan in inerten Lösungsmitteln wie Acetonitril oder Kohlenwasserstoffen wie Benzol, Toluol oder Xylol, oder Benzonitril oder Dimethylformamid oder Dimethylsulfoxid oder in einem Alkohol wie Methanol, Ethanol, Propanol, Butanol oder Isopropanol oder ohne Lösungsmittel in einem Temperaturbereich von 0°C bis +200°C, vorzugsweise von +20°C bis +180°C unter Darstellung der Phosphoniumsalze (X) umgesetzt.

Über dieses Verfahren sind die erfindungsgemäßen Verbindungen der Formel (I) zugänglich, bei denen V fehlt und T fehlt oder O bedeutet. Bei den Verbindungen der Formeln (IV) bis (X) haben die Reste R¹, R² und T die gleichen Bedeutungen wie in Anspruch 3 definiert.

Die Acetylenverbindungen der Formel (IV) sind beispielsweise durch Umsetzung entsprechender Phenolverbindungen mit ω-Halogenalkinen in Gegenwart von Basen erhältlich. Besonders bevorzugt sind hierbei ω-Chloralkine wie beispielsweise 5-Chlor-1-pentin. Als Basen können beispielsweise Metallhydride wie Natriumhydrid verwendet werden. Die als Ausgangsverbindungen einzusetzenden Phenole sind käuflich erhältlich oder durch dem Fachmann bekannte Standardreaktionen darstellbar (vgl. z.B. J. March, Advanced Organic Chemistry, 3.Auflage, Wiley, S. 1170 f.). Die Umsetzung zu den Acetylenverbindungen der Formel (IV) kann in organischen Lösungsmitteln wie beispielsweise Ethern, insbesondere Tetrahydrofuran, bei Temperaturen von +20°C bis +80°C unter Inertgasatmosphäre, beispielsweise Argon durchgeführt werden. In einigen Fällen kann es vorteilhaft sein, Komplexierungsmittel wie Hexaphosphorsäuretriamid zuzugeben. Alternativ können die Acetylenverbindungen (IV) durch Umsetzung entsprechender ω-Halogenalkylphenylverbindungen, vorzugsweise ω-Chloralkylphenylverbindungen, mit Acetyliden wie beispielsweise Natriumacetylid oder Lithiumacetylid unter dem Fachmann bekannten herkömmlichen Bedingungen erhalten werden (vgl. z.B. J. March, Advanced Organic Chemistry, 3.Auflage, Wiley, S. 429).

### Verfahren A - 2. Variante

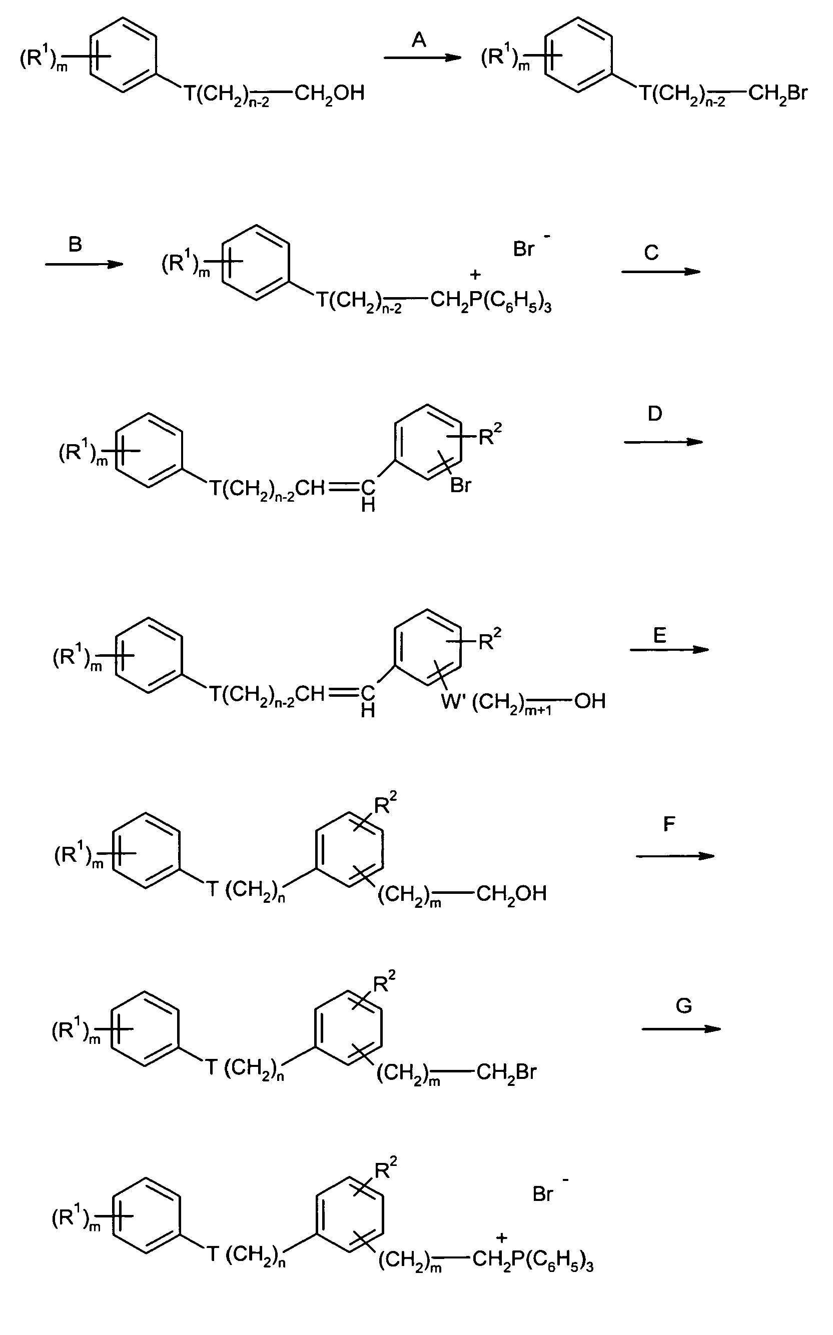

Im ersten Reaktionsschritt werden die als Ausgangsverbindungen verwendeten Alkohole bromiert, wobei als Bromierungsmittel beispielsweise die Verbindungen eingesetzt werden können, die im Schritt D der 1. Variante des Verfahrens A aufgeführt sind.

Die so erhaltenen Bromide werden wie im Schritt E der 1. Variante von Verfahren A mit Triphenylphosphan umgesetzt.

Im nächsten Reaktionsschritt wird wie vorstehend erläutert das reaktive Ylid erzeugt und dieses mit einem Brombenzaldehyd mit gewünschtem Substitutionsmuster umgesetzt.

Aus der so erhaltenen Verbindung können durch Umsetzung mit einer Base, vorzugsweise t-Butyllithium in einem inerten Lösungsmittel (Tetrahydrofuran), bei tiefen Temperaturen und anschließender Zugabe eines entsprechenden Elektrophils wie Paraformaldehyd oder Ethylenoxid die entsprechenden primären Alkohole (W' ist eine Direktbindung) erhalten werden. Wahlweise können die so erhaltenen Verbindungen mit einem gegebenenfalls geschützten Hydroxyalkin wie dem Tetrahydropyranylether von Propargylalkohol unter den gleichen Bedingungen wie im Verfahrensschritt [A] der 1. Variante von Verfahren A umgesetzt (W' bedeutet C=C) und anschließend durch eine Hydrierung, die analog zu Schritt C der 1. Variante von Verfahren A durchgeführt werden kann, zu den primären Alkoholen umgewandelt werden. Die so erhaltenen primären Alkohole werden analog zur 1. Variante des Verfahrens A in die entsprechenden Phosphoniumsalze überführt.

Über dieses Verfahren sind die erfindungsgemäßen Verbindungen der Formel (I) zugänglich, bei denen V fehlt und T fehlt oder O bedeutet.

Die als Ausgangsverbindungen bei diesem Verfahren verwendeten Hydroxyalkyloxyphenylverbindungen beziehungsweise Hydroxyalkylphenylverbindungen sind entweder käuflich erhältlich oder durch dem Fachmann bekannte herkömmliche Reaktionen darstellbar.

Bei den im vorstehenden Diagramm aufgeführten Verbindungen haben die Reste R¹, R² und T die gleichen Bedeutungen wie in Anspruch 3 definiert.

### Verfahren B - 1. Variante

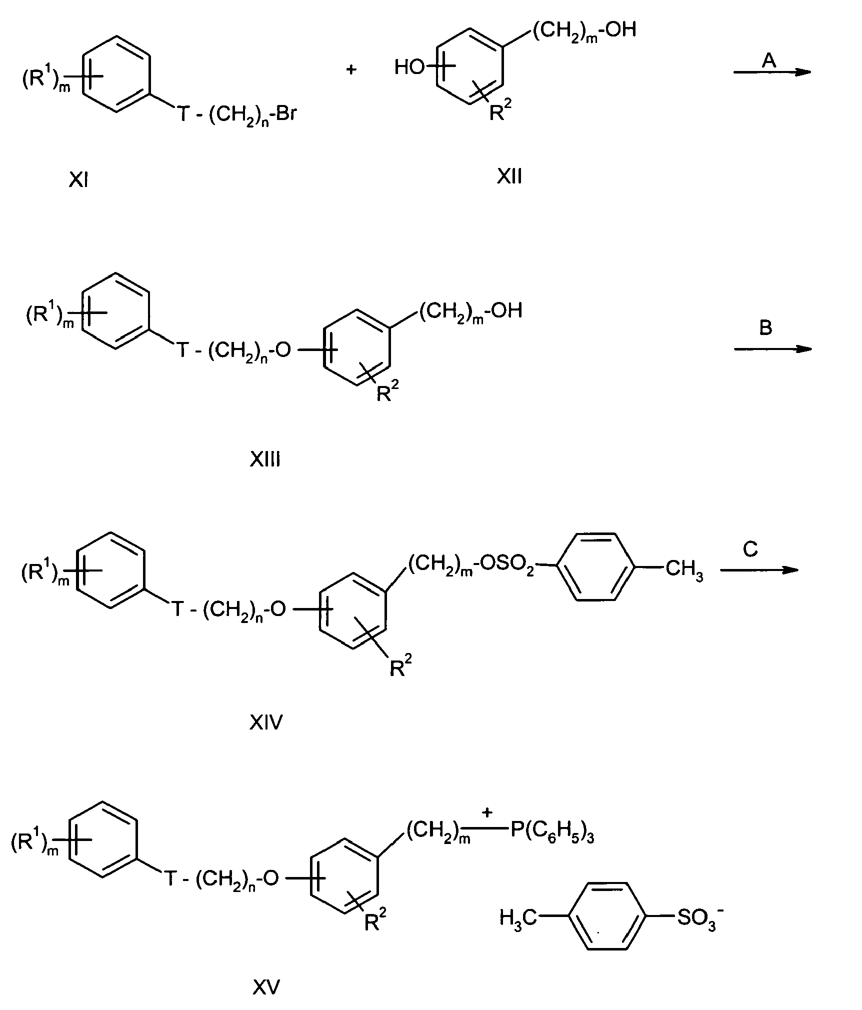

Im ersten Reaktionsschritt dieser Variante werden die Bromverbindungen (XI) mit den Phenolen (XII) in bevorzugten Lösungsmitteln wie Wasser oder Alkoholen wie beispielsweise Methanol, Ethanol, Propanol oder Isopropanol, oder Ethern wie Diethylether, Tetrahydrofuran, Dioxan oder Dimethyloxymethan, oder Dimethylformamid oder Dimethylsulfoxid, oder Acetonitril oder Ketonen wie beispielsweise Aceton, besonders bevorzugt in Isopropanol, in Gegenwart von Basen wie Alkalimetallhydroxiden, Carbonaten oder Alkoholaten wie beispielsweise Natriumcarbonat, Kaliumcarbonat, Cäsiumcarbonat, Natriumhydroxid, Kaliumhydroxid, Natriumethanolat oder Kalium-t.-butylat in einem Temperaturbereich von 0°C bis 200°C, vorzugsweise von +20°C bis +180°C umgesetzt.

Im zweiten Schritt [B] werden die Phenylether (XIII) mit Tosylchlorid in inerten Lösungsmitteln wie Ether, beispielsweise Diethylether, Tetrahydrofuran oder Dioxan, oder Kohlenwasserstoffen wie Benzol oder Toluol, oder chlorierten Kohlenwasserstoffen wie Chloroform oder Methylenchlorid, oder in Ethylacetat, Aceton oder Acetonitril, vorzugsweise in Methylenchlorid, in Gegenwart von Basen wie Triethylamin, Pyridin oder Dimethylaminopyridin, vorzugsweise in Gegenwart von Pyridin, in einem Temperaturbereich von -30°C bis +50°C, vorzugsweise von -10°C bis +30°C umgesetzt.

Im dritten Reaktionsschritt [C] werden die Tosylverbindungen (XIV) mit Triphenylphosphan in bevorzugten Lösungsmitteln wie Kohlenwasserstoffen, beispielsweise Benzol oder Toluol, Benzonitril, Acetonitril, Dimethylformamid oder Dimethylsulfoxid, oder ohne Lösungsmittel, besonders bevorzugt in Acetonitril, in einem Temperaturbereich von 0°C bis +200°C, vorzugsweise von +20°C bis +180°C unter Erhalt der Phosphoniumsalze (XV) umgesetzt.

Bei den Schritten B und C kann die Hydroxyverbindung XIII auch analog zu den Schritten D und E der ersten Variante des Verfahrens A zunächst in das Bromid und anschließend in das Phosphoniumsalz überführt werden.

Über dieses Verfahren sind die erfindungsgemäßen Verbindungen der Formel (I) zugänglich, bei denen V für O steht.

### Verfahren B - 2. Variante

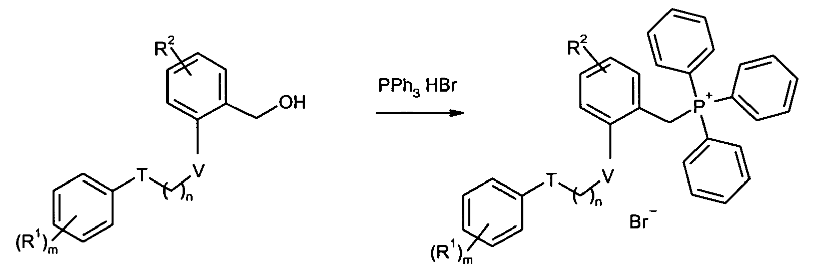

Bei dieser Variante werden die entsprechenden Hydroxyalkylphenylverbindungen mit Triphenylphosphoniumhydrobromid in einem organischen Lösungsmittel wie beispielsweise Acetonitril bei einer Temperatur von +30°C bis +100°C, vorzugsweise von +50C bis +90°C umgesetzt. Die Ausgangsverbindungen können auf herkömmliche Weise erhalten werden. Beispielsweise können für den Fall, dass T fehlt und V gleich O ist, durch Umsetzung einer entsprechenden Halogenalkylphenylverbindung, vorzugsweise einer Chlor- oder Bromalkylphenylverbindung wie beispielsweise Benzylbromid mit einer entsprechenden Phenolverbindung wie beispielsweise 2-Hydroxybenzylalkohol in einem organischen Lösungsmittel wie einem Alkohol, vorzugsweise Isopropanol, in Gegenwart einer Base wie beispielsweise Kaliumcarbonat bei einer Temperatur von +30 bis 100°C, vorzugsweise +50 bis 90°C umgesetzt.

Bei den in den vorstehenden Diagrammen des Verfahrens B aufgeführten Verbindungen haben die Reste R¹, R² und T die gleichen Bedeutungen wie in Anspruch 3 definiert.

### Verfahren B - 3. Variante

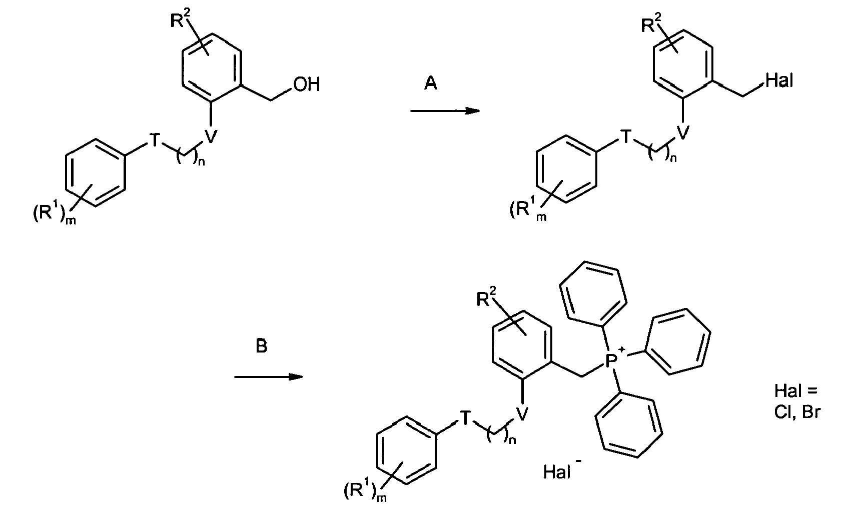

Bei dieser Variante wird der Alkohol zunächst gemäß dem Schritt D des Verfahrens A, Variante I, in ein Halogenid überführt, welches anschließend analog zum Schritt E des Verfahrens A, Variante I, zum gewünschten Phosphoniumsalz umgesetzt werden kann.

Bei dieser Variante haben R¹, R², T, V und n die vorstehend angegebenen Bedeutungen.

Die Aldehyde der allgemeinen Formel (II) können in Abhängigkeit der Bedeutungen von X und Y beispielsweise über folgende Verfahren hergestellt werden.

### Verfahren C

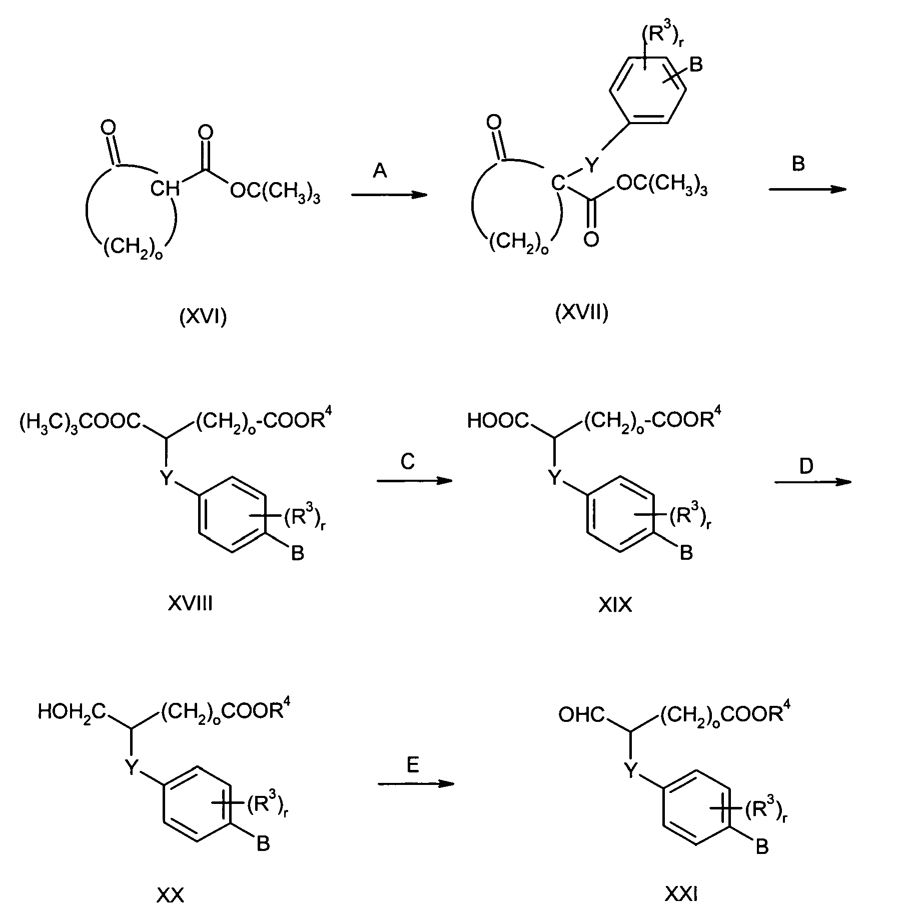

Im ersten Reaktionsschritt [A] dieser Variante wird das Keton (XVI) (wobei o 3, 4 oder 5 bedeutet) mit 4-Halogenmethylbenzoesäureestern oder 4-Halogensulfenylbenzoesäureestern, wobei der Halogenrest vorzugsweise Chlor oder Brom ist, beziehungsweise den entsprechenden Nitrilen in inerten Lösungsmitteln wie einem Ether, beispielsweise Diethylether, Tetrahydrofuran oder Dioxan, oder Dimethylformamid, oder Dimethylsulfoxid, oder in Gemischen davon, besonders bevorzugt in Dimethylformamid, in Gegenwart von Basen wie Alkalimetallhydriden, Amiden oder Alkolaten wie Natriumhydrid, Kaliumhydrid, Lithiumdiisopropylamid, Kaliumethylat, Natriumethylat, Kaliummethylat oder Kalium-t.-butylat, besonders bevorzugt in Gegenwart von Natriumhydrid, in einem Temperaturbereich von -40°C bis +60°C, besonders bevorzugt von -20°C bis +30°C umgesetzt.

Im zweiten Reaktionsschritt [B] werden die Ketone (XVII) in Lösungsmitteln wie Dimethylformamid oder Alkoholen, beispielsweise Methanol, Ethanol, Propanol oder Isopropanol, oder in Wasser oder in Gemischen davon, besonders bevorzugt in Dimethylformamid oder Ethanol, in Gegenwart von Basen wie Alkalimetallhydroxiden, Alkalimetallcarbonaten oder Alkalimetallalkoholaten wie Natriumhydroxid, Kaliumhydroxid, Natriumcarbonat, Natriummethanolat, Natriumethanolat, Kaliumethanolat oder Kalium-t.-butanolat, besonders bevorzugt in Gegenwart von Kalium-t.-butanolat, in einem Temperaturbereich von 0°C bis +150°C, besonders bevorzugt von +20°C bis +100°C, unter Erhalt der Verbindungen (XVIII) umgesetzt.

Im dritten Reaktionsschritt [C] werden die Verbindungen (XVIII) in Lösungsmitteln wie Alkoholen, beispielsweise Methanol, Ethanol, Propanol oder Isopropanol, oder in Ethern, beispielsweise Methylether, Tetrahydrofuran oder Dioxan, oder in chlorierten Kohlenwasserstoffen wie Methylenchlorid oder Chloroform, oder Carbonsäuren wie Essigsäure oder Trifluoressigsäure, oder in Gemischen davon, besonders bevorzugt in Trifluoressigsäure, in Gegenwart von Säuren wie Mineralsäuren, beispielsweise Salzsäure, Bromwasserstoffsäure oder Schwefelsäure oder Carbonsäuren, beispielsweise Essigsäure oder Trifluoressigsäure, besonders bevorzugt in Gegenwart von Essigsäure, insbesondere bevorzugt in Gegenwart von Trifluoressigsäure, sowohl als Lösungsmittel als auch als Säure, in einem Temperaturbereich von -20°C bis +60°C, besonders bevorzugt von 0°C bis +30°C unter Erhalt der Carbonsäuren (XIX) verseift.

Im vierten Schritt [D] werden die Carbonsäuren (XIX) in Lösungsmitteln wie Ether, beispielsweise Diethylether, Tetrahydrofuran oder Dioxan, oder in chlorierten Kohlenwasserstoffen wie Methylenchlorid oder Chloroform, oder in Gemischen davon, besonders bevorzugt in Tetrahydrofuran, unter Verwendung von Borverbindungen als Reduktionsmittel, beispielsweise Boran oder der Boran-Dimethylsulfid-Komplex, in einem Temperaturbereich von -40 °C bis +60°C, besonders bevorzugt von -20°C bis +30°C, unter Erhalt der Hydroxylverbindungen (XX) reduziert.

Im fünften Reaktionsschritt [E] werden die Hydroxyverbindungen (XX) in Lösungsmitteln wie Ether, beispielsweise Diethylether, Dioxan oder Tetrahydrofuran, oder in chlorierten Wasserstoffen wie Methylenchlorid oder Chloroform, oder in Dimethylsulfoxid oder in Gemischen davon, besonders bevorzugt in Dichlormethan, unter Verwendung von Oxidationsmitteln wie Pyridiniumchlorchromat, Chrom-(VI)-Salzen, Dimethylsulfoxid/ Pyridin/SO₃, katalytischen Mengen von Tetraalkylammoniumperruthenat in Gegenwart von N-Methylmorpholin und Molekularsieb, Dimethylsulfoxid/Oxalylchlorid/Triethylamin, besonders bevorzugt unter Verwendung von Pyridiniumchlorchromat, katalytischen Mengen von Tetraalkylammoniumperruthenat in Gegenwart von N-Methylmorpholinoxid und Molekularsieb oder Dimethylsulfoxid/Oxalylchlorid/Triethylamin, gegebenenfalls in Anwesenheit von Basen wie Triethylamin, Diisopropylamin, Pyridin oder Dimethylaminopyridin, besonders bevorzugt in Gegenwart von Triethylamin, in einem Temperaturbereich von -20°C bis +60°C, besonders bevorzugt von 0°C bis +30°C, unter Erhalt der Aldehyde (XXI) oxidiert.

Die cyclischen Ketone (XVI) sind entweder käuflich oder auf dem Fachmann bekannten herkömmlichen Wegen, beispielsweise durch Dieckmann-Kondensation der entsprechenden Carbonsäuredieester darstellbar.

Die mit den Ketonen (XVI) umzusetzenden 4-Chlormethylbenzoesäureester oder 4-Chlorsulfenylbenzoesäureester bzw. die entsprechenden Nitrile sind entweder käuflich oder auf dem Fachmann bekannten herkömmlichen Wegen darstellbar.

Bei den in dem vorstehenden Diagramm des Verfahrens C aufgeführten Verbindungen haben die Reste R³, R⁴, o, r und Y die gleichen Bedeutungen wie in Anspruch 3 definiert.

Mit dem Verfahren C können Aldehyde (II) hergestellt werden, bei denen X für -CH₂-, Y für -CH₂- oder -S-, o für 3, 4 oder 5, A für COOR⁴ und B für CN, CH₂OOR⁹, CONR¹⁰R¹¹ oder COOR⁸ steht.

### Verfahren D

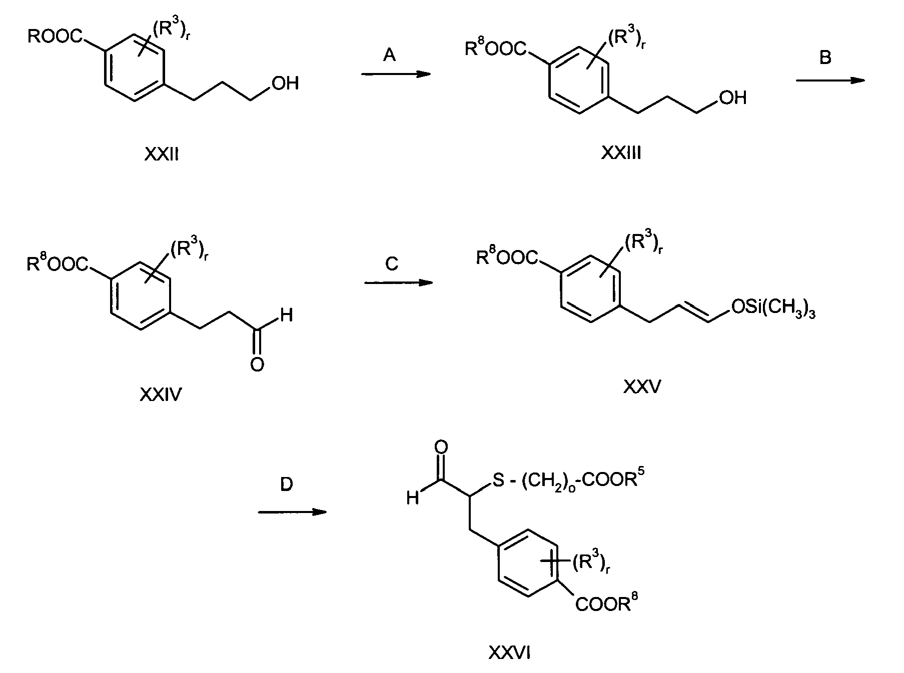

Im ersten Reaktionsschritt [A] dieser Variante wird das Benzoesäure-Gemisch (XXII) in Lösungsmitteln wie Alkoholen, beispielsweise Methanol, Ethanol, Propanol oder Isopropanol, oder in Wasser oder in Gemischen davon, besonders bevorzugt in Methanol, in Gegenwart von Säuren, wie Mineralsäuren, beispielsweise Salzsäure, Bromwasserstoffsäure oder Schwefelsäure, oder in Carbonsäuren, wie Essigsäure oder Trifluoressigsäure, oder besonders bevorzugt in Gegenwart von Thionylchlorid, in einem Temperaturbereich von -40°C bis +60°C, besonders bevorzugt von -20°C bis +40°C, in die Ester (XXIII) überführt.

Im zweiten Reaktionschritt [B] werden die Ester (XXIII) in Lösungsmitteln wie einem Ether, beispielsweise Diethylether, Tetrahydrofuran oder Dioxan, oder in Dimethylsulfoxid, oder in chlorierten Kohlenwasserstoffen wie Methylenchlorid oder Chloroform, oder in Gemischen davon, besonders bevorzugt in Methylenchlorid, unter Verwendung von Oxidationsmitteln wie Brom-(VI)-Salzen, Pyridiniumchlorchromat, Dimethylsulfoxid/Oxalylchlorid oder Dimethylsulfoxid/Pyridin/SO₃, besonders bevorzugt unter Verwendung von Dimethylsulfoxid/Oxalylchlorid, als Oxidationsmittel in Gegenwart von Basen wie Triethylamin, Diisopropylamin, Pyridin, oder Dimethylaminopyridin, besonders bevorzugt in Gegenwart von Triethylamin, in einem Temperaturbereich von -80°C bis +40°C, besonders bevorzugt von -60°C bis +20°C, analog dem Schritt E in Verfahren C zu den Aldehyden (XXIV) oxidiert.

Im dritten Reaktionsschritt [C] werden die Aldehyde (XXIV) in Lösungsmitteln wie Kohlenwasserstoffen, beispielsweise Benzol, Toluol oder Xylol, oder in Dimethylsulfoxid oder in Amiden wie Dimethylformamid oder Hexamethylphosporsäuretriamid, oder in Gemischen davon, besonders bevorzugt in Dimethylformamid, in Gegenwart von Basen wie Triethylamin, Diisopropylamin, Pyridin oder Dimethylaminopyridin, besonders bevorzugt in Gegenwart von Triethylamin, in einem Temperaturbereich von 0°C - +200°C, besonders bevorzugt von +20°C bis +180°C, mit Trimethylsilylchlorid oder -triflat in die Siliciumverbindungen (XXV) überführt.

Im vierten Reaktionsschritt [D] werden diese Siliciumverbindungen (XXV) mit 4,4'- Dithiodibutyrsäuredimethylester oder 3,3'-Dithiodipropansäuredimethylester in Gegenwart von Sulfurylchlorid oder Chlor oder Brom in wie einem Ether, beispielsweise Diethylether, Tetrahydrofuran oder Dioxan, oder in Kohlenwasserstoffen wie Benzol oder Toluol, oder in chlorierten Kohlenwasserstoffen wie Methylenchlorid oder Chloroform oder in Gemischen davon, besonders bevorzugt in Ethylenchlorid, gegebenenfalls in der Gegenwart von Basen wie Triethylamin oder Diisopropylamin oder Pyridin, in einem Temperaturbereich von -80°C bis + 20°C, besonders bevorzugt von -70°C bis +0°C in die Aldehyde (XXVI) überführt.

Mit dieser Variante können Verbindungen der allgemeinen Formel (II) hergestellt werden, bei denen X für S und vorzugsweise Y für CH₂ und o für 2 oder 3 steht.

Bei den in dem vorstehenden Diagramm des Verfahrens D aufgeführten Verbindungen haben die Reste R³, R⁸, r und o die gleichen Bedeutungen wie in Anspruch 3 definiert. Der Rest R bedeutet irgendeine herkömmliche alkoholische Komponente eines Esters.

Die Benzoesäureester der Formel (XXII) sind auf dem Fachmann bekannten herkömmlichen Wegen darstellbar oder käuflich.

### Verfahren E

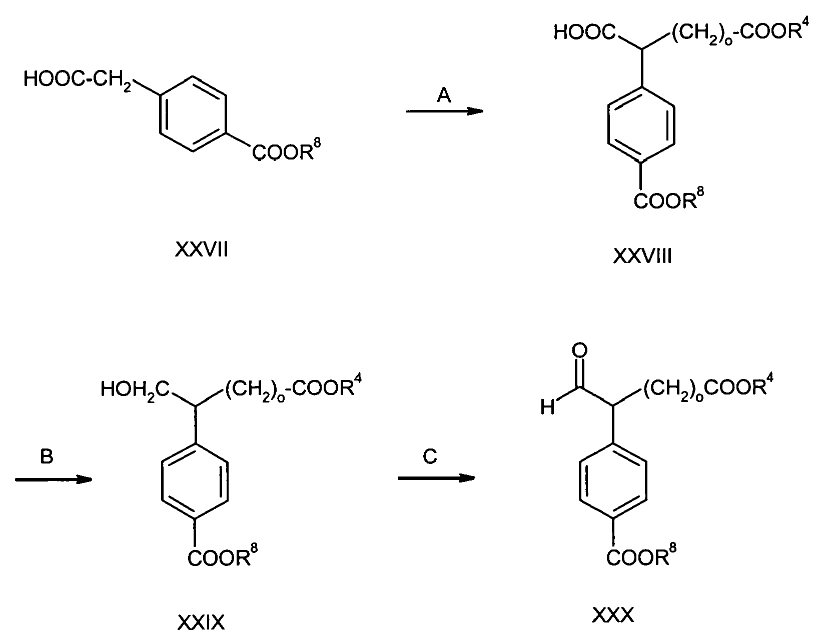

Bei dieser Variante wird das Benzoesäurederivat (XXVII) in Lösungsmitteln wie Ethern, beispielsweise Diethylether, Tetrahydrofuran, Dioxan, Diethylenglykolmonomethylether oder Diethylenglykoldiethylether oder in Amiden wie Dimethylformamid oder Hexamethylphophorsäuretriamid, in 1,3-Dimethylimidazolidin-2-on oder 1,3-Dimethyltetrahydropyridin-2-on oder in Gemischen davon, besonders bevorzugt in Tetrahydrofuran, in Gegenwart von Organometallverbindungen als Base, beispielsweise organischen Lithium-, Natrium- oder Kaliumverbindungen, besonders bevorzugt Butyllithium, Methyllithium, Phenyllithium, Natriumnaphtalid, Kaliumnaphtalid, Lithiumdiisopropylamid oder Lithiumhexamethyldisilazan, insbesondere bevorzugt in Gegenwart von Lithiumdiisopropylamid, in einem Temperaturbereich von -80°C bis +60°C, besonders bevorzugt von -50°C bis +30°C, zu den Verbindungen (XXVIII) umgesetzt, welche anschließend in einem zweiten Reaktionsschritt [B] in Lösungsmitteln wie einem Ether, beispielsweise Dimethylether, Tetrahydrofuran oder Dioxan, oder in chlorierten Kohlenwasserstoffen wie Methylenchlorid oder Chloroform, oder in Gemischen davon, besonders bevorzugt in Tetrahydrofuran, unter Verwendung von Boranen als Reduktionsmittel, vorzugsweise unter Verwendung von Boran oder dem Boran-Dimethylsulfid-Komplex, in einem Temperaturbereich von -40°C bis +60°C, vorzugsweise von -20°C bis +30°C, zu den Hydroxyverbindungen (XXIX) reduziert werden.

Im dritten Reaktionsschritt [C] werden die Hydroxyverbindungen (XXIX) in Lösungsmitteln wie einem Ether, beispielsweise Diethylether, Tetrahydrofuran oder Dioxan, oder in chlorierten Kohlenwasserstoffen wie Methylenchlorid oder Chloroform, oder Dimethylsulfoxid, oder in Gemischen davon, besonders bevorzugt in Dichlormethan unter Verwendung von Oxidationsmitteln wie Chrom-(VI)-Salzen, Pyridiniumchlorchromat, Dimethylsulfoxid/Oxalylchlorid oder Dimethylsulfoxid/ Pyridin/SO₃, besonders bevorzugt Pyridiniumchlorchromat, gegebenenfalls in Anwesenheit von Basen wie Triethylamin, Diisopropylamin oder Pyridin, besonders bevorzugt in Gegenwart von Triethylamin, in einem Temperaturbereich von -80°C bis +60°C, vorzugsweise von -60°C bis +30°C, analog dem Schritt E in Verfahren C zu den Aldehyden (XXX) oxidiert. Die Benzoesäurederivate der Formel (XXVII) sind käuflich oder auf dem Fachmann bekannte herkömmliche Weise erhältlich.

Mit dieser Variante können Verbindungen der allgemeinen Formel (II) hergestellt werden, bei denen X für CH₂ und vorzugsweise Y für eine Direktbindung und o für 3 oder 4 steht.

Bei den in dem vorstehenden Diagramm des Verfahrens E aufgeführten Verbindungen haben die Reste R⁴, R⁸ und o die gleichen Bedeutungen wie in Anspruch 3 definiert, wobei jedoch R⁴ und R⁸ nicht COOH sein dürfen.

### Verfahren F und G

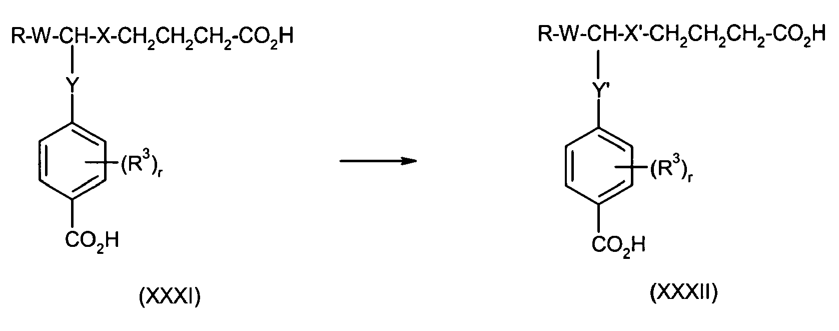

Bei dieser Variante wird die Säure (XXXI) in Lösungsmitteln wie Alkoholen, Wasser, Aceton oder Acetonitril mit einem Oxidationsmittel wie Wasserstoffperoxid, Salpetersäure, Persäuren, Sauerstoff, Ozon, organischen Persäuren, Kaliumpermanganat, Kaliumpersulfat, Natriumhypochlorit, hypochlorigen Säuren, Rutheniumtetroxid, Stickoxiden, anodischer Oxidation oder mit einem speziellen Gemisch wie Ozon in einem normalen Temperaturbereich von -20°C bis +30°C umgesetzt, obwohl für wenig reaktive Substanzen sogar tiefere Temperaturbereiche (-78°C) notwendig sein können. Das Produkt dieses Verfahrens ist das Sulfon (XXXII).

Mit dieser Variante können Verbindungen der allgemeinen Formel (I) hergestellt werden, bei denen X für CH₂ oder eine Direktbindung und Y für SO oder SO₂ oder X für SO oder SO₂ und Y für CH₂ oder eine Direktbindung steht.

Bei den in dem vorstehenden Diagramm des Verfahrens F aufgeführten Verbindungen haben die Reste R³, W, X und Y sowie r die gleichen Bedeutungen wie in Anspruch 3 definiert. X' und Y' stehen für beim dem Verfahren F gegebenenfalls veränderte Reste X und Y (d.h. für SO₂). R steht für den Rest der Verbindungen der allgemeinen Formel (I).

### Verfahren G

Bei dieser Variante wird die Säure (XXXI) wie in Variante F/G umgesetzt, jedoch unter Einsatz geringerer Mengen an Oxidationsmitteln und/oder bei tieferer Temperatur oder mit Oxidationsmitteln wie Hydroperoxiden, Mangandioxid, Selendioxid, Persäuren, Chromsäure oder Iodosobenzol. Das Produkt dieses Verfahrens ist das Sulfoxid (XXXII).

Bei den in dem vorstehenden Diagramm des Verfahrens F aufgeführten Verbindungen haben die Reste R³, W, X und Y sowie r die gleichen Bedeutungen wie in Anspruch 3 definiert. X' und Y' stehen für beim dem Verfahren G gegebenenfalls veränderte Reste X und Y (d.h. für SO). R steht für den Rest der Verbindungen der allgemeinen Formel (I).

### Verfahren H

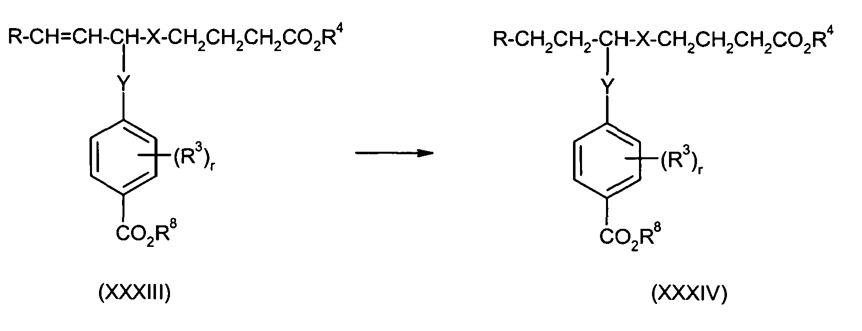

Bei diesem Verfahren wird die Säure (XXXIII) in Lösungsmitteln wie Alkoholen, Wasser, Benzol, Toluol, Ethern wie Dimethylether, Tetrahydrofuran, Dioxan, Estern wie Ethylacetat, oder in Kohlenwasserstoffen wie Hexan, oder in Aminen wie Triethylamin oder in Ammoniak mit einem Reduktionsmittel wie Wasserstoff in Gegenwart eines Metallkatalysators wie den Oxiden oder löslichen Komplexen von Palladium, Platin, Ruthenium oder Nickel, oder mit einem Metall wie Lithium oder Natrium, oder mit Hydrazin oder Arylaralkoxy-substituierten Hydrazinen umgesetzt. Das Produkt dieser Reaktion ist die Säure (XXXIV), worin W der allgemeinen Formel (I) - CH₂CH₂- oder -CH₂CH₂CH₂- bedeutet. Der normale Temperaturbereich für dieses Verfahrens beträgt -20°C bis +30°C.

Bei den in dem vorstehenden Diagramm des Verfahrens H aufgeführten Verbindungen haben R³, R⁴, R⁸, X, r und Y die gleichen Bedeutungen wie in Anspruch 3 definiert. R steht für den Rest der Verbindungen der allgemeinen Formel (I), wobei R einen Arylrest, aber keine Doppelbindung enthalten darf.

### Verfahren I

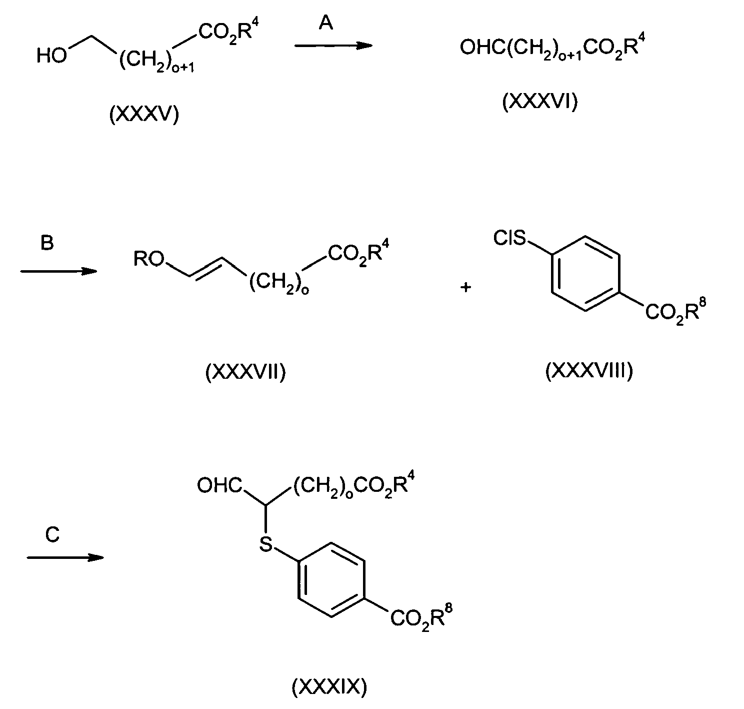

Diese Verfahrensvariante ist analog zu Verfahren D und stellt eine Alternative zum Verfahren C für den Fall dar, dass Y=S ist. Jedoch ist es im Gegensatz zum Verfahren C auch für Verbindungen anwendbar, bei denen o nicht 3, 4 oder 5 ist, sondern für eine ganze Zahl von 1 bis 6 steht.

Die drei Schritte sind wie folgt:
- [A]: entspricht Schritt E des Verfahrens C.
- [B]: entspricht Schritt C des Verfahrens D, wobei R Trimethylsilyl bedeutet. Wahlweise kann R für Alkyl, beispielsweise Methyl, stehen und wird Schritt B durch Zugabe des Aldehyds zu einer Lösung des Alkoxymethylenylids durchgeführt (hierbei wird o um 1 erhöht). Letzteres wird wie vorstehend beschrieben aus einem Alkoxymethylentriphenylphosphoniumsalz erzeugt.
- [C]: entspricht Schritt D des Verfahrens D.

### Verfahren J und Verfahren K

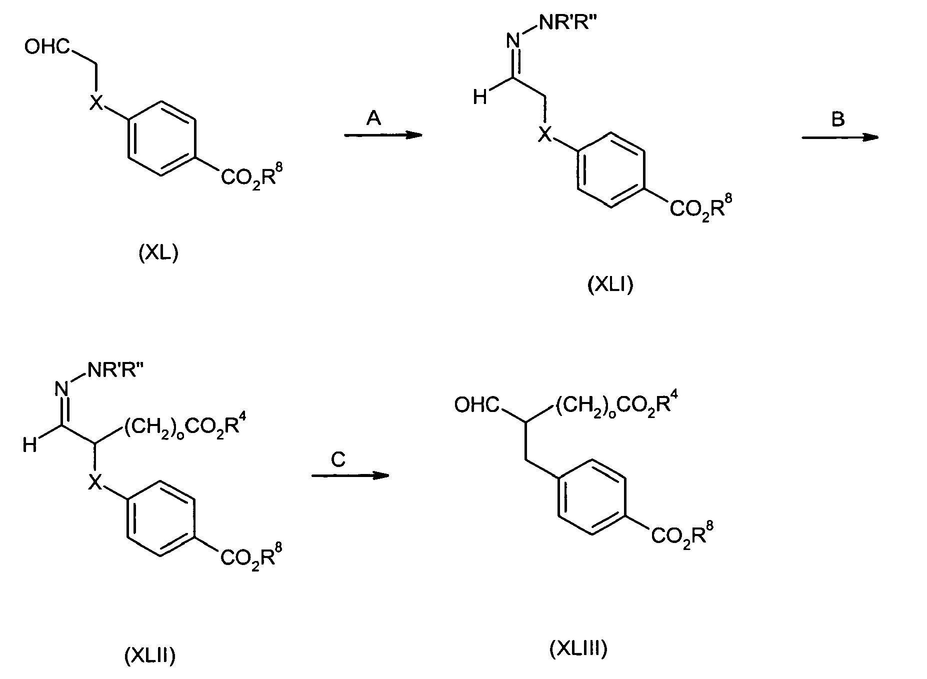

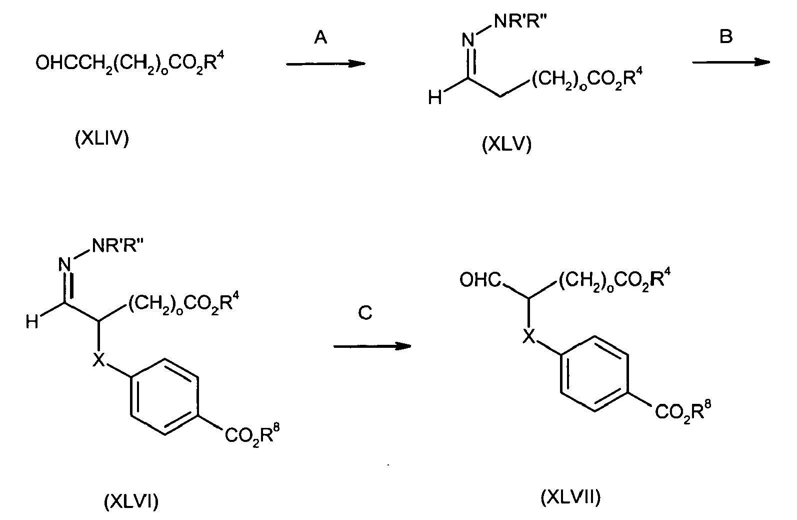

Diese zwei Varianten eines Verfahrens ermöglichen zwei Wege zu den Aldehyden XLIII oder XLVII.

Der Schritt A ist bei beiden Verfahren identisch und besteht in der Umsetzung eines Aldehyds XL oder XLIV mit einem Dialkylaminohydrazin wie Dimethylhydrazin (R'=R"= Alkyl) (E.J. Corey und D. Enders, Chem. Ber., 111, 1337, 1363 (1978)) oder (R)- oder (S)-1-Amino-2-methoxymethylpyrrolidin (R' und R" stellen zusammen mit dem Stickstoffatom, an das sie gebunden sind, einen (S)-2-Methoxypyrrolidinrest dar) (D. Enders et al., Org. Syn. 65, 183 (1987)). Die Verwendung dieser chiralen Hydrazine (RAMP oder SAMP), führt dazu, dass der nachfolgende Schritt praktisch vollständig diastereoselektiv durchgeführt werden kann, so dass das Produkt des Schrittes B ein einziges Diastereomer sein kann. Dadurch wird die Notwendigkeit umgangen, die Produkte wie XLIII oder XLVII auf andere Weise aufzutrennen. Schritt A wird am besten durchgeführt, indem der Aldehyd und das Hydrazin in Abwesenheit eines Lösungsmittels vermischt und auf 60 bis 70°C für einen geeigneten Zeitraum (einen Tag) unter einer Inertatmosphäre (z.B. unter Stickstoff oder Argon, vorzugsweise unter Argon) erhitzt werden.

Schritt B wird in inerten Lösungsmitteln wie Diethylether oder Tertrahydrofuran bei verminderter Temperatur, vorzugsweise bei 0°C, mit einer organometallischen Base wie Butyllithium oder Lithiumdiisopropylamid und anschließender Zugabe eines entsprechenden Elektrophils (R⁴OOC(CH₂)ₒHal, R⁸OOCC₆H₄CH₂Hal oder R⁸OOCC₆H₄SCl) durchgeführt, wodurch das alkylierte Produkt XLII oder XLVI erhalten wird.

Schritt C besteht in einer oxidativen Spaltung der Hydrazone zu den Aldehyden XLIII oder XLVII unter Verwendung von beispielsweise Ozon in einem Lösungsmittel (Dichlormethan) bei tiefen Temperaturen (-78°C) (für den Fall, dass die chiralen Hydrazone verwendet wurden). Die Dimethylhydrazone können mit Natriumperiodat in wässriger Lösung oder durch Methylierung mit Methyliodid und anschließender Zugabe einer Säure (beispielsweise einer Mineralsäure wie Salzsäure) gespalten werden.

Mit dieser Variante können Verbindungen der allgemeinen Formel (II) hergestellt werden, bei denen X für S, CH₂ oder im Fall der Variante J eine Direktbindung und Y für eine Direktbindung steht.

Bei den in dem vorstehenden Diagramm der Verfahren J und K aufgeführten Verbindungen haben die Reste R⁴, R⁸, o und X die gleichen Bedeutungen wie in Anspruch 3 definiert, wobei jedoch R⁴ und R⁸ nicht COOH sein dürfen.

### Verfahren L

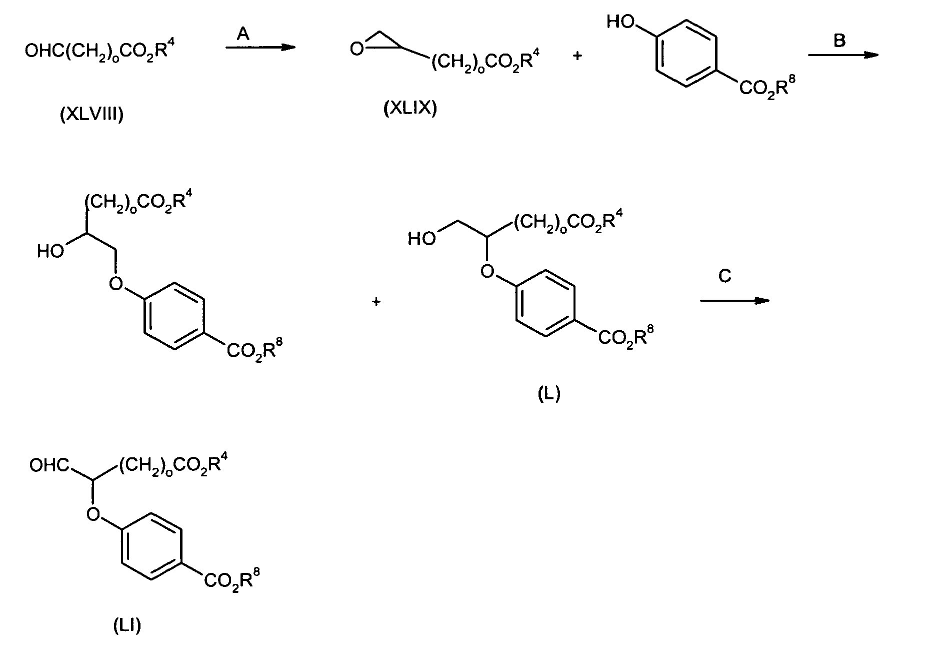

Verfahren M

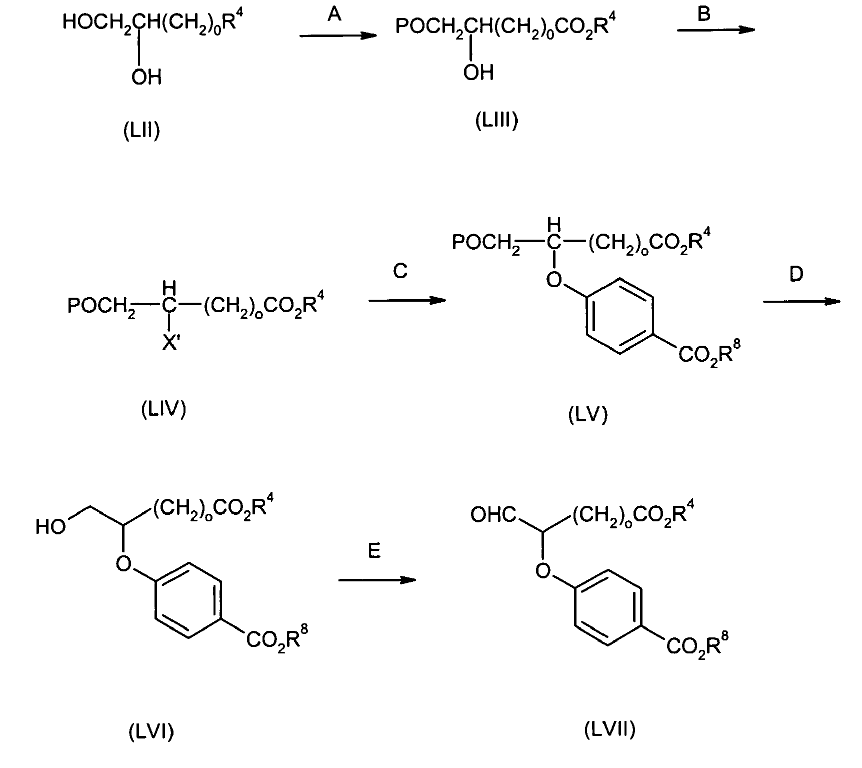

Diese Verfahren veranschaulichen zwei Wege zur Herstellung eines Aldehyds LI oder LVII mit X=O und Y=Direktbindung.

Im ersten Schritt des Verfahrens L wird die Verbindung XLVIII mit Sulfoniumethylid (E.J. Corey et al., J. Am. Chem. Soc. 87, 1353 (1965)) in einem inerten Lösungsmittel unter Erhalt eines Epoxids XLIX umgesetzt.

Das Epoxid erfährt durch Umsetzung mit einem Phenol in einem Lösungsmittel wie Methanol eine nukleophile Ringöffnung, wodurch zwei Regioisomere erhalten werden, von denen das gewünschte Isomer L auf einfache Weise chromatographisch erhalten werden kann. Die Ausbeute und das Verhältnis der zwei Isomeren kann durch Variation des Lösungsmittels und durch Verwendung von Katalysatoren verändert werden.

Schritt C ist eine einfache Oxidation, wie sie bereits im Schritt C des Verfahrens E ausführlich beschrieben wurde.

Wahlweise kann im Verfahren M ein Diol LII durch herkömmliche Schutzgruppentechnik an der primären Hydroxylgruppe geschützt und in einen Tetrahydropyranylether (P=2-Tetrahydropyranyl), t-Butyldimethylsilylether (P=SiMe₂t-Bu) oder t-Butyldiphenylsilylether (P=SiPh₂t-Bu) LIII überführt werden, dessen sekundäre Hydroxylgruppe nicht geschützt ist.

Der Schritt B dieses Verfahrens beinhaltet die Umwandlung der freien Hydroxylgruppe in eine herkömmliche Abgangsgruppe X' wie beispielsweise eine Tosylgruppe oder einen Halogenidrest, vorzugsweise einen Brom- oder Iodrest, auf bereits in vorstehenden Verfahren beschriebenen üblichen Wegen.

Im Schritt C wird im wesentlichen wie in Schritt A des Verfahrens B beschrieben die Abgangsgruppe X' durch eine Phenoxygruppe ersetzt.

In Schritt D wird die Schutzgruppe P selektiv durch ein entsprechendes herkömmliches Verfahren aus dem Stand der Technik entfernt.

Schritt E ist eine einfache, bereits vorstehend beschriebene Oxidation.

Bei den in dem vorstehenden Diagramm der Verfahren L und M aufgeführten Verbindungen haben die Reste R⁴, R⁸ und o die gleichen Bedeutungen wie in Anspruch 3 definiert, wobei jedoch R⁴ und R⁸ nicht COOH sein dürfen.

### Verfahren N

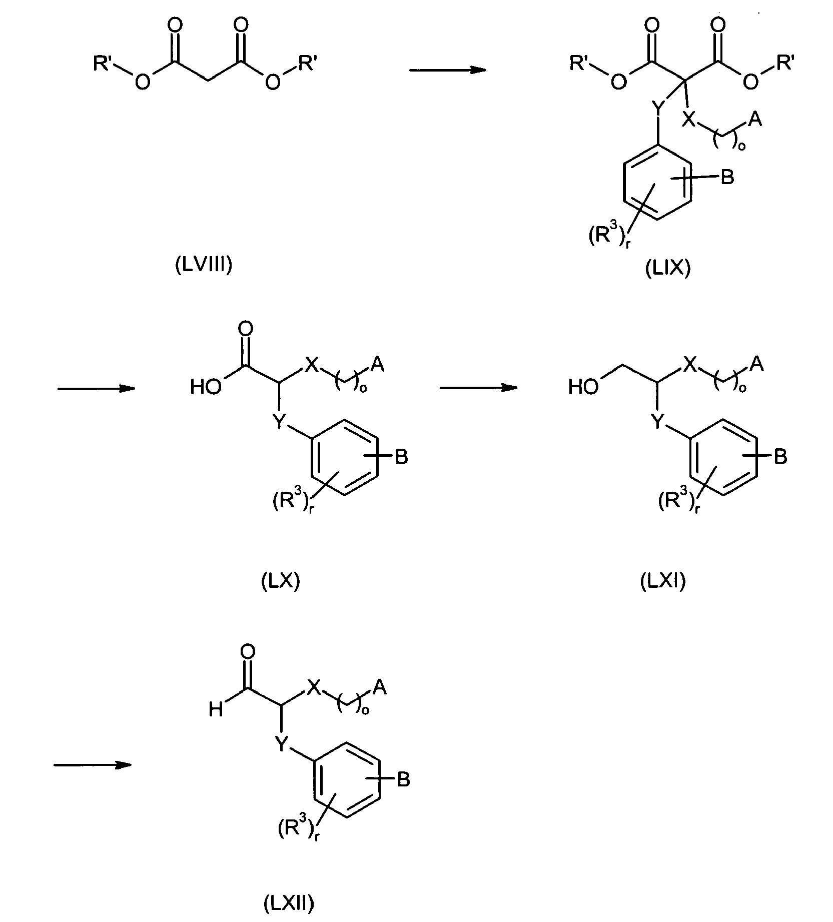

Bei diesem Verfahren wird ein Malonsäurediester (LVIII), wobei als alkholische Komponente R' ein Allylrest oder niedere Alkylreste wie Methyl, Ethyl, t-Bu oder ein Benzylrest eingesetzt werden können) durch zwei aufeinanderfolgende Umsetzungen mit entsprechenden Elektrophilen in einen 2,2-disubstituierten Malonsäurediester (LIX) überführt. Beispielsweise kann der Malonsäurediester (LVIII) zunächst in Gegenwart einer Base wie beispielsweise Natriumhydrid, Triethylamin, Kaliumcarbonat, Natriumhydroxid, DABCO, Kaliumhydroxid, Lithiumdiisopropylamid oder Natriumamid, bevorzugt Natriumhydrid, mit einem entsprechenden Elektrophil wie einem entsprechenden Halogenid, Tosylat, Mesylat oder Triflat, zum Beispiel einem Halogenid wie □-Chlor- oder □-Bromcarbonsäureester, beispielsweise Bromessigsäuremethylester, in einem Lösungsmittel wie Dioxan bei Temperaturen von 0 bis 50°C umgesetzt werden. In einem zweiten Schritt kann das so erhaltene monsubstituierte Malonsäurediesterderivat durch Umsetzung mit einem entsprechenden Elektrophil wie einem entsprechenden Halogenid, Tosylat, Mesylat oder Triflat, zum Beispiel einem 2-Halogenbenzylderivat wie 2-(Bromomethyl)benzoesäuremethylester, in Gegenwart einer Base wie beispielsweise Natriumhydrid, Triethylamin, Kaliumcarbonat, Natriumhydroxid, DABCO, Kaliumhydroxid, Lithiumdiisopropylamid oder Natriumamid, bevorzugt Natriumhydrid, in einem Lösungsmittel wie Dimethylformamid bei Temperaturen von 0 bis 50°C umgesetzt werden. Die Umsetzungen mit den beiden Elektrophilen können jedoch auch in umgekehrter Reihenfolge durchgeführt werden.

Das so erhaltene 2,2-disubstituierte Malonsäurediesterderivat (LIX) kann durch Reaktion mit einer Säure wie beispielsweise Salzsäure, Schwefelsäure oder Trifluoressigsäure, oder durch Reaktion mit einer Base wie Kaliumhydroxid, Natriumhydroxid oder Lithiumhydroxid, oder durch eine Palladium-katalysierte Reaktion wie beispielsweise mit Ameisensäure in Gegenwart eines Pd-Katalysators, vorzugsweise eines Pd(II)-Katalysators wie Palladium-(II)-acetat, und eines Phosphans wie Triphenylphosphan und einer Base wie einem Amin, vorzugsweise Triethylamin, in einem Lösungsmittel wie Dioxan bei Temperaturen von 20 bis 120°C durch Esterspaltung und anschließende Decarboxylierung bei erhöhten Temperaturen in die Carbonsäurederivate (LX) überführt werden.

Die Carbonsäurederivate (LX) können ihrerseits durch eine Reduktion mit herkömmlichen Reduktionsmitteln wie beispielsweise Diisobutylaluminiumhydrid (DIBAL), Lithiumaluminiumhydrid oder Borhydriden wie Boran in Tetrahydrofuran zu den Alkoholen (LXI) umgesetzt werden.

Die Alkohole (LXI) können schließlich mit herkömmlichen milden Oxidationsmitteln wie Cr-(VI)-Verbindungen wie PDC oder PCC, Kaliumpermanganat, Dimethylsulfoxid/Oxalylchlorid/Triethalmin (Swern-Oxidation) oder Tetrapropylammoniumperruthenat (TPAP) in Gegenwart einer Base wie N-Methylmorpholinoxid und Molsieb oder durch die Dess-Martin-Oxidation zu den Aldehyden (LXII) oxidiert werden.

Bei den in dem vorstehenden Diagramm des Verfahrens N aufgeführten Verbindungen haben R³, A, B, X, Y, r und o die gleichen Bedeutungen wie in Anspruch 3 definiert wobei jedoch A und B keine freie Carboxylfunktion und X und Y nicht O sein dürfen.

### Verfahren O

In den vorstehenden Verfahren wurde die Herstellung □-disubstituierter Aldehyde mit einer p-Alkoxycarbonylgruppe als einem der Substituenten in □-Position beschrieben. Es ist aber selbstverständlich auch möglich, Verbindungen der Formel (II) herzustellen, bei denen der Rest B wie in Anspruch 3 definiert ist und in ortho-, meta- oder para-Position zum Rest Y sitzt. In diesen Fällen erfolgen die vorstehend beschriebenen Umsetzungen anstatt mit einer para-disubstituierten Arylverbindung mit einer entsprechend ortho- oder meta-disubstituierten Verbindung. Die Tetrazolylgruppe (wenn A oder B für Tetrazolyl steht) wird hierbei vorzugsweise durch Verwendung eines entsprechenden monosubstituierten Nitrils und anschließende Umsetzung mit Natriumazid in Gegenwart eines Salzes eines tertiären Amins wie Triethylamin oder Morpholin-Hydrochlorid) in einem inerten Lösungsmittel wie Dimethylformamid bei erhöhten Temperaturen eingeführt. Amide oder Sulfonamide werden vorzugsweise aus selektiv verseifbaren Estervorstufen dargestellt. Die selektiv freigesetzte Carbonsäuregruppe kann dann mit einem Aryl-, Alkyl- oder Sulfonamid in Gegenwart eines Diimids wie Dicyclohexancarbodiimid in einem inerten Lösungsmittel umgesetzt werden. Wahlweise kann die selektiv freigesetzte Carbonsäuregruppe beispielsweise durch Umsetzung mit Diphenylposhinsäurechlorid aktiviert und anschließend mit einem entsprechenden Amin zum gewünschten Amid umgesetzt werden.

### Verfahren P

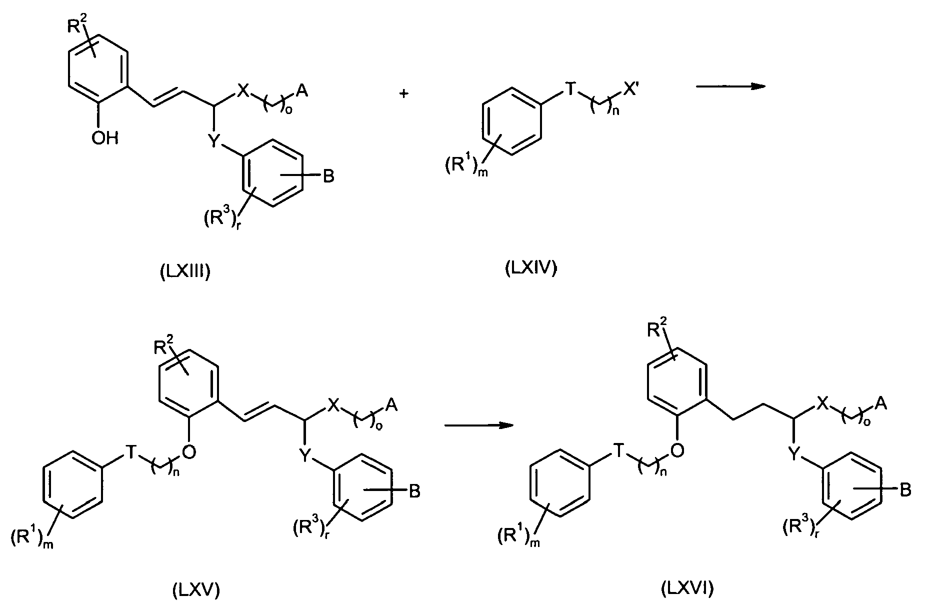

Die Verbindungen der Formel (I) können alternativ auch durch Umsetzung entsprechender Aldehyde (II) mit 2-Hydroxybenzyltriphenylphosphoniumverbindungen zunächst zu den Alkenen (LXIII) und anschließenden Aufbau der Seitenkette dargestellt werden. Die einleitende Wittig-Reaktion kann beispielsweise in einer Inertgasatmosphäre wie Argon in einem Lösungsmittel wie Tetrahydrofuran in Gegenwart einer Base wie n-Butyllithium erfolgen. Die so erhältlichen Verbindungen der Formel (LXIII) können durch Reaktion mit Verbindungen (LXIV), welche eine Abgangsgruppe X' wie beispielsweise ein Halogenatom, vorzugsweise ein Chlor-, Brom- oder Iodatom, oder eine Tosylat-, Mesylat- oder Triflatgruppe enthalten, in Gegenwart einer Base wie Kaliumcarbonat oder Cäsiumcarbonat in einem Lösungsmittel wie Acetonitril zu den Verbindungen der Formel (LXV) umgesetzt werden. Die Verbindungen der Formel (LXV) können analog Verfahren H zu Verbindungen der Formel (LXVI) hydriert werden.

Insbesondere im Fall, dass an die Verbindung der Formel (LXIII) eine Benzylgruppe angeknüpft werden soll, kann vorzugsweise zunächst die Doppelbindung analog dem Verfahren H hydriert und anschließend die Umsetzung an der freien Hydroxygruppe erfolgen.

Durch dieses Verfahren sind Verbindungen der Formel (I) zugänglich, bei denen V für ein Sauerstoffatom steht.

Bei den in dem vorstehenden Diagramm des Verfahrens P aufgeführten Verbindungen haben die Reste R¹, R², R³, A, B, T, X, Y, n, m, r und o die gleichen Bedeutungen wie in Anspruch 3 definiert, wobei jedoch A und B nicht für freie Carboxylfunktionen stehen dürfen.

Die erfindungsgemäßen Verbindungen der Formel (I), bei denen Y für O, S, SO oder SO₂ steht, können über das erfindungsgemäße Verfahren [β] hergestellt werden. Hierbei werden Aldehyde der Formel (i) 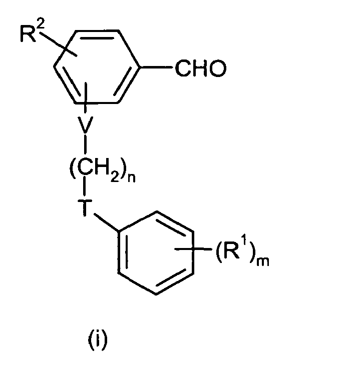 worin
R¹, R², T, V, m und n die vorstehend angegebenen Bedeutungen haben,
mit Phosphorverbindungen der Formel (ii) 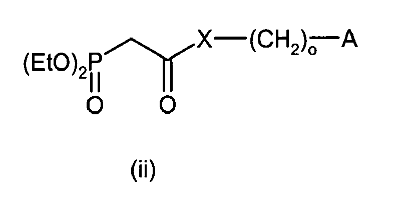 worin
X, o und A die vorstehend angegebenen Bedeutungen haben,
zu Verbindungen der Formel (iii) 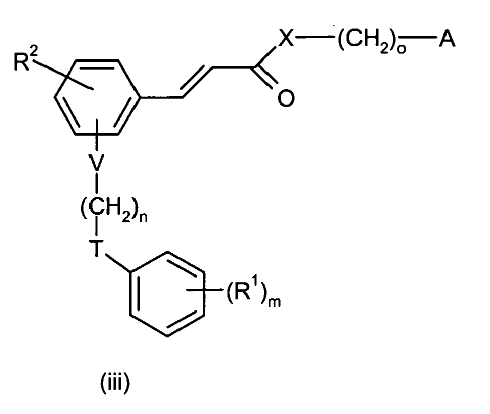 worin
R¹, R², T, V, m, n, X, o und A die vorstehend angegebenen Bedeutungen haben,
umgesetzt und diese anschließend durch aufeinanderfolgende Reduktion der Alkengruppe und der Carbonylgruppe und anschließende Substitution der durch Reduktion der Carbonylgruppe erzeugten Hydroxygruppe mit Alkoholen oder Thiolen sowie gegebenenfalls anschließende Oxidation zu den entsprechenden Sulfoxid- oder Sulfonverbindungen in Verbindungen der Formel (iv) überführt 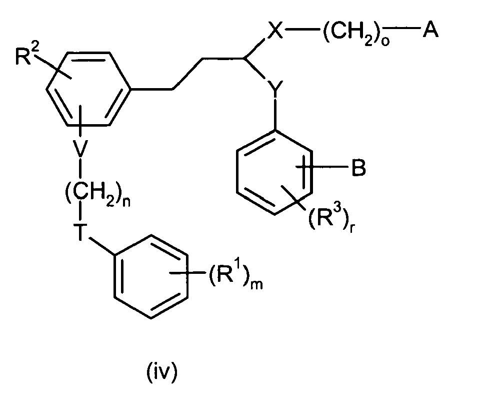 worin
R¹, R², T, V, m, n, X, o, r, A, Y, B und R³ die vorstehend angegebenen Bedeutungen haben.

Die Aldehyde der Formel (i) sind beispielsweise aus den bei den Verfahren A und B als Zwischenprodukte eingesetzten Alkoholen durch dem Fachmann bekannte herkömmliche Oxidationsreaktionen zugänglich (vgl. z.B. J. March, Advanced organic Chemistry, 3^{rd} ed., S. 1057 ff., Wiley).

Die Phosphorverbindungen der Formel (ii) können beispielsweise durch Umsetzung von Alkandicarbonsäurederivaten, beispielsweise den entsprechenden Monoestern, mit Phosphonoessigsäurederivaten, beispielsweise den entsprechenden Diestern, hergestellt werden. Möglich ist aber auch die Synthese aus Phosphiten wie beispielsweise Triethylphosphit mit entsprechenden α-Halogenketonderivaten (Arbuzov-Rkt, vgl. z.B. J. March, Advanced organic Chemistry, 3^{rd} ed., S. 848 ff., Wiley).

Die Umsetzung der Verbindungen der Formel (i) mit Verbindungen der Formel (ii) erfolgt in Gegenwart von Basen wie Alkalimetallhydriden, beispielsweise Natriumhydrid, Alkalimetallalkoholaten, beispielsweise Kalium-t-butylat, oder in Gegenwart von Salzen wie beispielsweise MgCl₂ und Basen wie Aminen, beispielsweise Triethylamin, oder der Hünig-Base. Die Reaktion wird vorzugsweise in organischen Lösungsmitteln, besonders bevorzugt in Tetrahydrofuran, bei Raumtemperatur oder unter leichtem Erhitzen durchgeführt.

Die so erhaltenen Carbonylverbindungen der Formel (iii) werden nach herkömmlichen, dem Fachmann bekannten Verfahren zu den entsprechenden Alkoholen reduziert (vgl. z.B. J. March, Advanced organic Chemistry, 3^{rd} ed., S. 809 ff., Wiley). Besonders bevorzugt ist die Verwendung von komplexen Metallhydriden wie Diisobutylaluminiumhydrid (DIBAL), NaBH₄ oder NaBH₄/CeCl · 7 H₂O. Die Reaktion wird vorzugsweise in organischen Lösungsmitteln wie beispielsweise Alkoholen wie Methanol unter Kühlung durchgeführt.

Die olefinische Doppelbindung der so erhaltenen Hydroxyverbindungen kann nach herkömmlichen, dem Fachmann bekannten Verfahren hydriert werden (vgl. z.B. J. March, Advanced organic Chemistry, 3^{rd} ed., S. 691 ff., Wiley). Bevorzugt ist die Hydrierung mit Wasserstoff in Gegenwart eines Metallkatalysators wie Pd/C oder Raney-Nickel in einem organischen Lösungsmittel wie beispielsweise Ethylacetat.

Die Einführung des Restes Y-Ph(R³)-B kann auf mehreren Wegen erfolgen. Beispielsweise kann die Hydroxyverbindung unter Mitsunobu-Bedingungen (vgl. O. Mitsunobu, Synthesis, 1981, 1-28) mit entsprechenden Alkoholen, Phenolen oder Thiolen umgesetzt werden. Die Hydroxygruppe kann aber auch erst in eine Abgangsgruppe überführt werden, welche anschließend durch entsprechende Alkohole, Phenole oder Thiole in Gegenwart einer Base wie beispielsweise DABCO, Triethylamin, NaH, NaOH, KOH, LDA, Natriumamid oder besonders bevorzugt Kaliumcarbonat substituiert werden kann. Als Abgangsgruppen sind erfindungsgemäß bevorzugt Halogenreste wie Cl, Br oder I, welche durch Umsetzung der Hydroxyverbindung mit beispielsweise SOCl₂, SOBr₂, POCl₃, PCl₃, PCl₅, PBr₃ usw. einführbar sind, der Tosylatrest, welcher beispielsweise durch Umsetzung mit Tosylchlorid einführbar ist, der Mesylatrest, welcher beispielsweise durch Umsetzung mit MsCl einführbar ist, oder der Triflatrest, welcher durch Umsetzung mit beispielsweise Tf₂O oder TfCl einführbar ist.

Die erfindungsgemäßen Verbindungen, insbesondere die Verbindungen der allgemeinen Formel (I), zeigen ein nicht vorhersehbares, wertvolles pharmakologisches Wirkspektrum.

Die erfindungsgemäßen Verbindungen, insbesondere die Verbindungen der allgemeinen Formel (I), führen zu einer Gefäßrelaxation, Thrombozytenaggregationshemmung und zu einer Blutdrucksenkung sowie zu einer Steigerung des koronaren Blutflusses. Diese Wirkungen sind über eine direkte Stimulation der löslichen Guanylatcyclase und einem intrazellulären cGMP-Anstieg vermittelt.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose, fibrotischen Erkrankungen wie Leberfibrose oder Lungenfibrose, asthmatischen Erkrankungen und Krankheiten des Urogenitalsystems wie beispielsweise Prostatahypertrophie, erektile Dysfunktion, weibliche sexuelle Dysfunktion und Inkontinenz sowie zur Behandlung von Glaucoma eingesetzt werden.

Die in der vorliegenden Erfindung beschriebenen Verbindungen, insbesondere die Verbindungen der allgemeinen Formel (I), stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuss- und Suchtmittelaufnahme.

Weiterhin eignen sich die Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (A-poplexia cerebri) wie Schlaganfall, cerebraler Ischämien und des Schädel-Hirn-Traumas. Ebenso können die erfindungsgemäßen Verbindungen, insbesondere die Verbindungen der allgemeinen Formel (I), zur Bekämpfung von Schmerzzuständen eingesetzt werden.

Zudem besitzen die erfindungsgemäßen Verbindungen antiinflammatorische Wirkung und können daher als entzündungshemmende Mittel eingesetzt werden.

### Gefäßrelaxierende Wirkung in vitro

Kaninchen werden durch intravenöse Injektion von Thiopental-Natrium narkotisiert bzw. getötet (ca. 50 mg/kg,) und entblutet. Die Arteria Saphena wird entnommen und in 3 mm breite Ringe geteilt. Die Ringe werden einzeln auf je einem triangelförmigen, am Ende offenen Häkchenpaar aus 0,3 mm starkem Spezialdraht (Remanium®) montiert. Jeder Ring wird unter Vorspannung in 5 ml Organbäder mit 37°C warmer, carbogenbegaster Krebs-Henseleit-Lösung folgender Zusammensetzung (mM) gebracht: NaCl: 119; KCI: 4,8; CaCl₂ x 2 H₂O: 1; MgSO₄ x 7 H₂O: 1,4; KH₂PO₄: 1,2; NaHCO₃: 25; Glucose: 10; Rinderserumalbumin: 0,001 %. Die Kontraktionskraft wird mit Statham UC2-Zellen erfasst, verstärkt und über A/D-Wandler (DAS-1802 HC, Keithley Instruments München) digitalisiert, sowie parallel auf Linienschreibern registriert. Kontraktionen werden durch Zugabe von Phenylephrin induziert.

Nach mehreren (allgemein 4) Kontrollzyklen wird die zu untersuchende Substanz in jedem weiteren Durchgang in steigender Dosierung zugesetzt und die Höhe der unter dem Einfluss der Testsubstanz erzielten Kontraktion mit der Höhe der im letzten Vordurchgang erreichten Kontraktion verglichen. Daraus wird die Konzentration errechnet, die erforderlich ist, um die in der Vorkontrolle erreichte Kontraktion auf 50 % zu reduzieren (IC₅₀). Das Standardapplikationsvolumen beträgt 5 µl. Der DMSO-Anteil in der Badlösung entspricht 0,1 %.

Die Ergebnisse sind in Tabelle 1 gezeigt:

**Tabelle 1: Gefäßrelaxierende Wirkung in vitro**

| **Beispiel** | **IC**_{**50**} **(nM)** |
|---|---|
| 12 | 112 |
| 13 | 2600 |
| 14 | 8,7 |
| 16 | 5 |
| 23 | 26 |
| 24 | 6200 |
| 34 | 0,35 |
| 35 | 1,7 |
| 40 | 41 |
| 41 | 2,8 |
| 44 | 7800 |
| 60 | 608 |

### Stimulation der rekombinanten löslichen Guanylatcyclase (sGC) in vitro

Die Untersuchungen zur Stimulation der rekombinanten löslichen Guanylatcyclase (sGC) und die erfindungsgemäßen Verbindungen mit und ohne Natriumnitroprussid sowie mit und ohne den Häm-abhängigen sGC-Inhibitor 1*H*-1,2,4-Oxadiazol-(4,3a)-chinoxalin-1-on (ODQ) wurden nach der in folgender Literaturstelle im Detail beschriebenen Methode durchgeführt: M. Hoenicka, E.M. Becker, H. Apeler, T. Sirichoke, H. Schroeder, R. Gerzer und J.-P. Stasch: Purified soluble guanylyl cyclase expressed in a baculovirus/Sf9 system: stimulation by YC-1, nitric oxide, and carbon oxide. J. Mol. Med. 77:14-23 (1999).

Die Häm-freie Guanylatcyclase wurde durch Zugabe von Tween 20 zum Probenpuffer (0,5% in der Endkonzentration) erhalten.

Die Aktivierung der sGC durch eine Prüfsubstanz wird als n-fache Stimulation der Basalaktivität angegeben.

Die Ergebnisse sind in Tabelle 2 gezeigt:

**Tabelle 2: Stimulation der rekombinanten löslichen Guanylatcyclase (sGC) in vitro**

| **Bsp. 16 Konzentration (µM)** | **Stimulation (n-fach)** | | | | |
|---|---|---|---|---|---|
| | **Häm-haltige sGC** | | | **Häm-freie sGC** | |
| | **Basal** | **+ SNP (0,1 µM)** | **+ ODQ (10 µM)** | **Basal** | **+ ODQ (10 µM)** |
| 0 | 1 | 15 | 1 | 1 | 1 |
| 0,1 | 4 | 14 | 27 | 11 | 12 |
| 1,0 | 9 | 33 | 52 | 54 | 94 |
| 10 | 19 | 29 | 88 | 204 | 291 |

Aus Tabelle 2 ist ersichtlich, dass eine Stimulation sowohl des Häm-haltigen als auch des Häm-freien Enzyms erreicht wird. Weiterhin zeigt eine Kombination aus sGC-Stimulator und Natriumnitroprussid (SNP), einem NO-Donor, keine synergistischen Effekt, d.h. die Wirkung von SNP wird nicht potenziert, wie dies bei über einem Häm-abhängigen Mechanismus wirkenden sGC-Stimulatoren zu erwarten wäre. Darüber hinaus wird die Wirkung des erfindungsgemäßen sGC-Stimulators durch den Häm-abhängigen Inhibitor der löslichen Guanylatcyclase ODQ nicht blockiert. Die Ergebnisse aus Tabelle 2 belegen somit den neuen Wirkmechanismus der erfindungsgemäßen Stimulatoren der löslichen Guanylatcyclase.

Zur vorliegenden Erfindung gehören pharmazeutische Zubereitungen, die neben nicht-toxischen, inerten pharmazeutisch geeigneten Trägerstoffen die erfindungsgemäßen Verbindungen, insbesondere die Verbindungen der allgemeinen Formel (I), enthält sowie Verfahren zur Herstellung dieser Zubereitungen.

Die Wirkstoff können gegebenenfalls in einem oder mehreren der oben angegebenen Trägerstoffe auch in mikroverkapselter Form vorliegen.

Die therapeutisch wirksamen Verbindungen, insbesondere die Verbindungen der allgemeinen Formel (I), sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen, insbesondere den Verbindungen der allgemeinen Formel (I), auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

### Beispiele

Die vorliegende Erfindung wird nachstehend anhand von nicht einschränkenden bevorzugten Beispielen näher dargestellt. Soweit nicht anderweitig angegeben, beziehen sich alle Mengenangaben auf Gewichtsprozente.

### Abkürzungen:

- RT:: Raumtemperatur
- EE:: Essigsäureethylester
- BABA:: n-Butylacetat/n-Butanol/Eisessig/Phosphatpuffer pH 6 (50:9:25.15; org. Phase)

### Laufmittel für die Dünnschichtchromatographie:

- T1 E1:: Toluol - Essigsäureethylester (1:1)
- T 1 EtOH1:: Toluol - Methanol (1:1)
- C 1 E1:: Cyclohexan - Essigsäureethylester (1:1)
- C1 E2:: Cyclohexan - Essigsäureethylester (1:2)

### Ausgangsverbindungen

### Bsp.I): 5-(4-Methoxycarbonylbenzyl)-6-oxohexansäuremethylester

### Ia) 2-(4-Methoxycarbonylbenzyl)hexan-1,6-disäure-1-tert-butyl-6-methyldiester

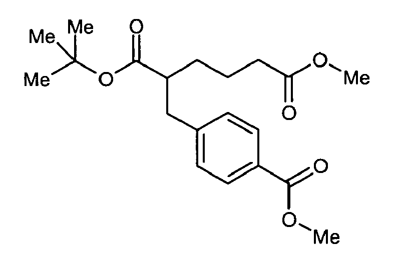

4,83 g (30 mmol) 2-Oxocyclopentancarbonsäuretertbutylester (hergestellt analog D. Henderson et al., Synthesis, 1983, 12, 996) werden unter Argon in 30 ml Dimethylformamid gelöst. 1,15 g (30 mmol) Natriumhydrid (60 %-ig ölige Suspension) werden portionsweise bei 0°C zugegeben. Nach beendeter Wasserstoffentwicklung wird eine Lösung aus 4,84 g (30 mmol) 4-Chlormethylbenzoesäuremethylester und 4,35 g (35 mmol) Kaliumiodid in 8 ml Dimethylformamid zugegeben. Die Mischung wird 30 min. bei 0°C gerührt, und anschließend läßt man auf Raumtemperatur erwärmen. Man gibt 2 ml Methanol zu und rührt bei Raumtemperatur. Nach Zugabe von Essigsäureethylester wird mit Natriumthiosulfatlösung, zweimal mit Wasser und einmal mit Natriumchloridlösung gewaschen, getrocknet und im Vakuum eingedampft.

Ausbeute: 1,24 g (12,9 % d. Th.)
R_{f}: 0,52(C1E1)

### Ib) 2-[4-Methoxycarbonylbenzyl]-hexan-1,6-disäure-6-methylester

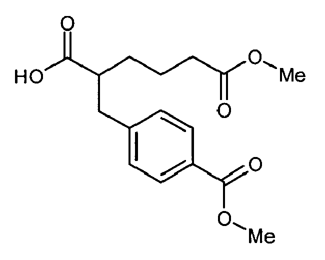

326 mg (0,89 mmol) des t-Butylesters aus Ia) werden in 5 ml Trifluoressigsäure gelöst und eine Stunde bei Raumtemperatur gerührt. Es wird im Vakuum bis zur Trockene eingeengt. Der Rückstand wird in Essigester aufgenommen und zweimal mit Natriumhydrogencarbonatlösung extrahiert. Die wäßrige Phase wird mit 1N Salzsäure auf pH 4 gestellt, zweimal mit Essigester extrahiert, mit Magnesiumsulfat getrocknet und im Vakuum eingedampft.

Ausbeute: 154 mg (54,0 % d. Th.)
¹H-NMR (300 MHz, CDCl₃): 7.90 (d, 2H), 7.20 (d, 2H), 3.90 (s, 3H), 3.65 (s, 3H), 3.10 (dd, 1H), 2.80 (m, 2H), 2.30 (m, 2H), 1.70-1.50 (m, 4H)

### Ic) 6-Hydroxy-5-(4-methoxycarbonylbenzyl)hexansäuremethylester

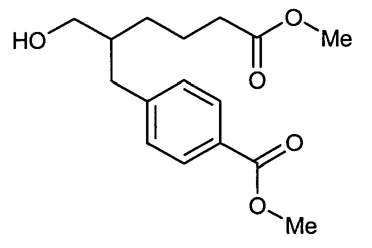

144 mg (0,49 mmol) der Säure aus Ib) werden unter Argon in 2 ml THF gelöst. Bei -10°C werden langsam 0,6 ml (0,6 mmol) Boranlösung in THF (1M) tropfenweise zugegeben. Die Lösung wird 2 Stunden bei 0°C gerührt. Nach einer Stunde werden erneut 0,5 ml der Boranlösung zugegeben. Vorsichtig wird gesättige Natriumbicarbonatlösung zugetropft und zweimal mit Essigester extrahiert, mit Magnesiumsulfat getrocknet und im Vakuum eingedampft.

Ausbeute: 127 mg (92,4% d. Th.)
¹H-NMR (200 MHz, CDCl₃): 7.90 (d, 2H), 7. 20 (d, 2H), 3.90 (s, 3H), 3.65 (s, 3H), 3.50 (m, 2H), 2.70 (m, 2H), 2.30 (m, 2H), 1.80-1.30 (m, 5H)

### I): 5-(4-Methoxycarbonylbenzyl)-6-oxohexansäuremethylester

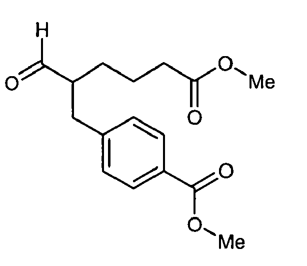

109 mg (0.37 mmol) des Alkohols aus Ic) werden in Dichlormethan gelöst und mit 0,5 g Molsieb 4A (aktiviert 2h bei 125°C im Vakuum) und 65 mg (0,56 mmol) N-Methylmorpholinoxid versetzt. Nach 10 Minuten werden 6,5 mg (0,02 mmol) Tetrapropylammoniumperruthenat (TPAP) zugegeben. Nach 40 Minuten wird das Reaktionsgemisch mit Dichlormethan verdünnt, filtriert, einmal mit Wasser gewaschen, mit Magnesiumsulfat getrocknet und im Vakuum eingedampft. Die Substanz wird mit C2:E1 als Eluent chromatographiert und weiter umgesetzt.

Ausbeute: 34 mg (31,4 % d. Th.)

### Bsp. II): 6-(4-Cyanbenzyl)-7-oxo-heptansäureethylester

### IIa) 2-(4-Cyanbenzyl)heptan-1,7-disäure-1-tert-butyl-7-ethyldiester

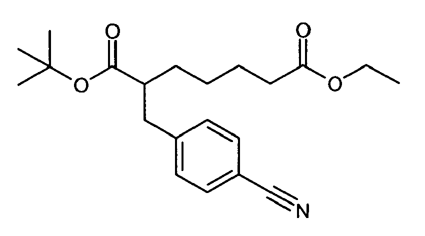

5,33 g (17,1 mmol) 1-(4-Cyanbenzyl)-2-oxocyclohexancarbonsäure-tert.butylester (hergestellt aus 2-Oxocyclohexancarbonsäuretertbutylester und 4-Cyanbenzylbromid mit Natriumhydrid in Benzol) und 1,91 (17,1 mmol) Kalium-tert.-butylat werden in 50 ml Ethanol gelöst und die Lösung 4 Stunden unter Rückfluß erhitzt. Die Lösung wird abgekühlt, mit 10 g Kieselgel versetzt und einrotiert. Die Substanz wird zur Reinigung an 120 g Kieselgel 60 (Korngröße 0,040-0,063mm) mit C1:E1 1:1 bis Essigester als Eluent chromatographiert.

Ausbeute: 3,00 g (49,1 % d. Th.)
R_{f}: 0,69 (EE)

### IIb) 2-(4-Cyanbenzyl)-heptan-1,7-disäure-7-ethylester

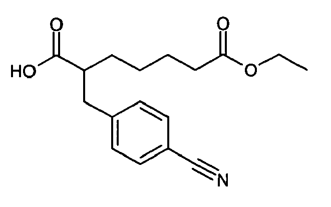

3,00 g (8,34 mmol) des t-Butylesters aus IIa) werden analog Beispiel Ib) umgesetzt. Die Säure wird roh weiter umgesetzt.

Ausbeute: 3,15 g (roh)
¹H-NMR (400 MHz, CDCl₃): 7.70 (d, 2H), 7. 40 (d, 2H), 4.20 (q, J=6Hz, 2H), 3.00 (dd, 1H), 2.90 (dd, 1H), 2.70 (m, 1H), 2.30 (t, 2H), 1.70-1.30 (m, 6H), 1.20 (t, J=6Hz, 3H)

### IIc) 6-(4-Cyanbenzyl)-7-hydroxyheptansäureethylester

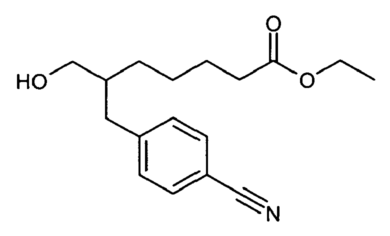

3,15 g der rohen Säure aus IIb) werden analog Beispiel Ic) umgesetzt.

Ausbeute: 1,264 g (42,4 % d.Th. über 2 Stufe)
¹H-NMR (200 MHz, CDCl₃): 7.60 (d, 2H), 7. 20 (d, 2H), 4.10 (q, J=6Hz, 2H), 3.50 (m, 2H), 2.70 (m, 2H), 2.30 (t, 2H), 1.90-1.20 (m, 10H)

### II) 6-(4-Cyanbenzyl)-7-oxo-heptansäureethylester

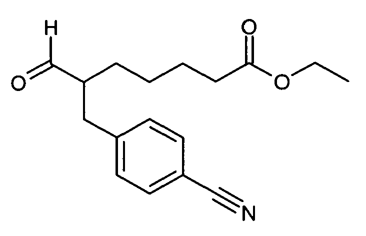

1,13 g (3,90 mmol) des Alkohols aus IIc) werden analog Beispiel Id) umgesetzt.

Ausbeute: 0,79 g (70,4 % d.Th)
¹H-NMR (200 MHz, CDCl₃): 9.60 (d, 1H), 7.60 (d, 2H), 7. 20 (d, 2H), 4.10 (q, J=6Hz, 2H), 3.10 (dd, 1H), 2.70 (m, 2H), 2.30 (t, 2H), 1.90-1.20 (m, 9H).

### Bsp. III-XVI): Triphenylphosphoniumbenzylsalze

### IIIa) 2-Benzyloxybenzylalkohol

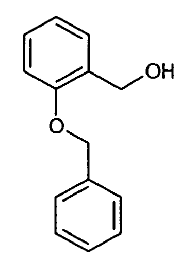

13,78 g (80,5 mmol) Benzylbromid, 10,00 g (80,5 mmol) 2-Hydroxybenzylalkohol und 11,13 g (80,5 mmol) Kaliumcarbonat werden in 270 ml 2-Propanol unter Rückfluß über Nacht erhitzt. Die Suspension wird abgekühlt, in Essigester aufgenommen und mit 1N Natronlauge und Wasser gewaschen, über Magnesiumsulfat getrocknet und im Vakuum eingedampft.

Ausbeute: 15,15 g (87,8 % d. Th.)
R_{f}(SiO₂, C4E1): 0,14

### Analog wurden dargestellt:

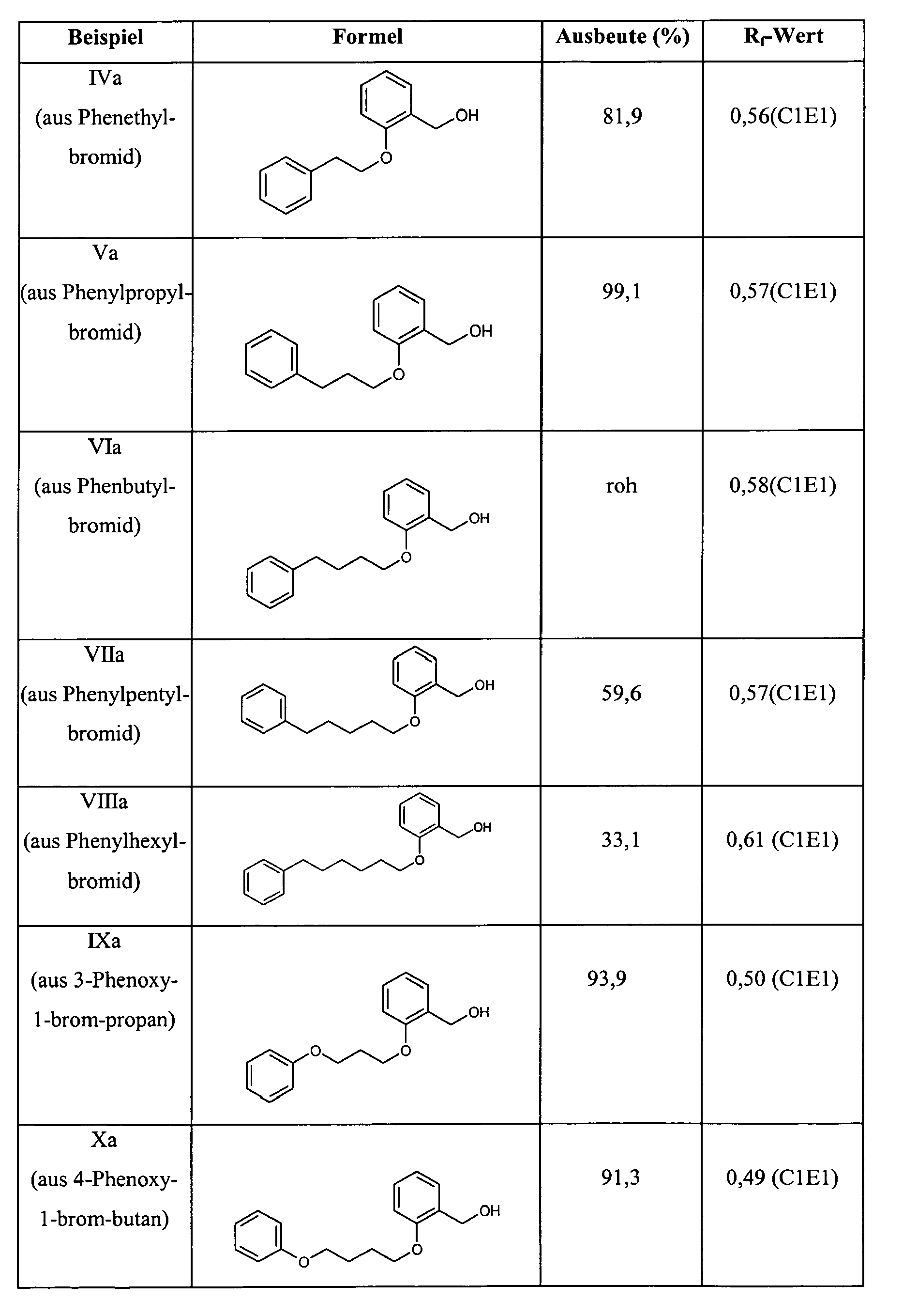

### III) 2-(Benzyloxy)benzyltriphenylphosphoniumbromid

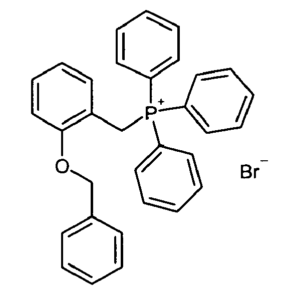

15,15 g (70,7 mmol) des Benzylalkohols IIIa und 21,86 g (63,7 mmol) Triphenylphosphoniumhydrobromid werden in 240 ml Acetonitril unter Rückfluß 5 Stunden erhitzt. Das Lösungsmittel wird im Vakuum eingedampft, und anschließend wird Diethylether zugegeben. Das Feststoff wird filtriert und im Vakuum getrocknet.

Ausbeute: 32,24 g (84,4 % d. Th.)
¹H-NMR (200 MHz, d⁶-DMSO): 7.80-6.60 (m, 24H), 5.20 (d, J=15Hz, 2H), 4.40 (s, 2H)

### Analog wurden dargestellt:

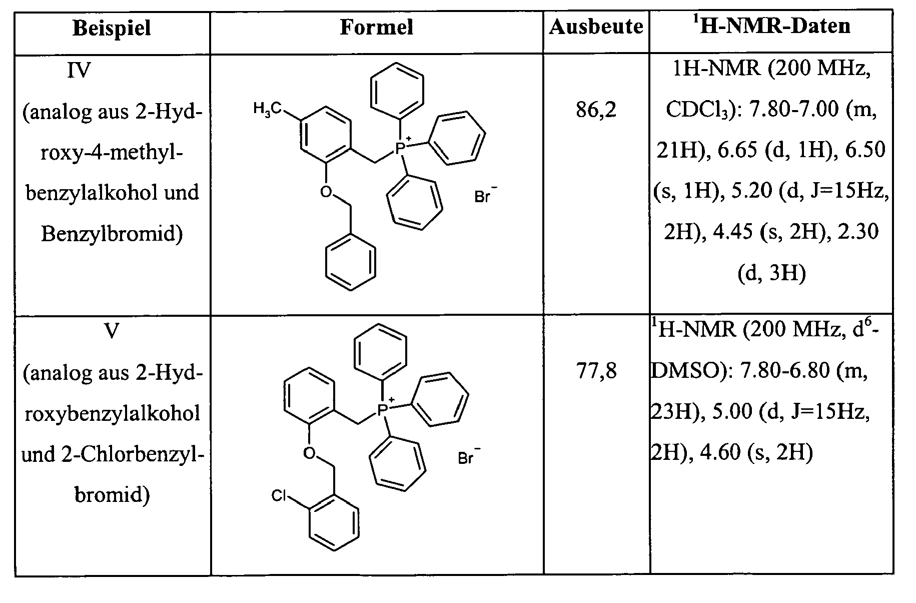

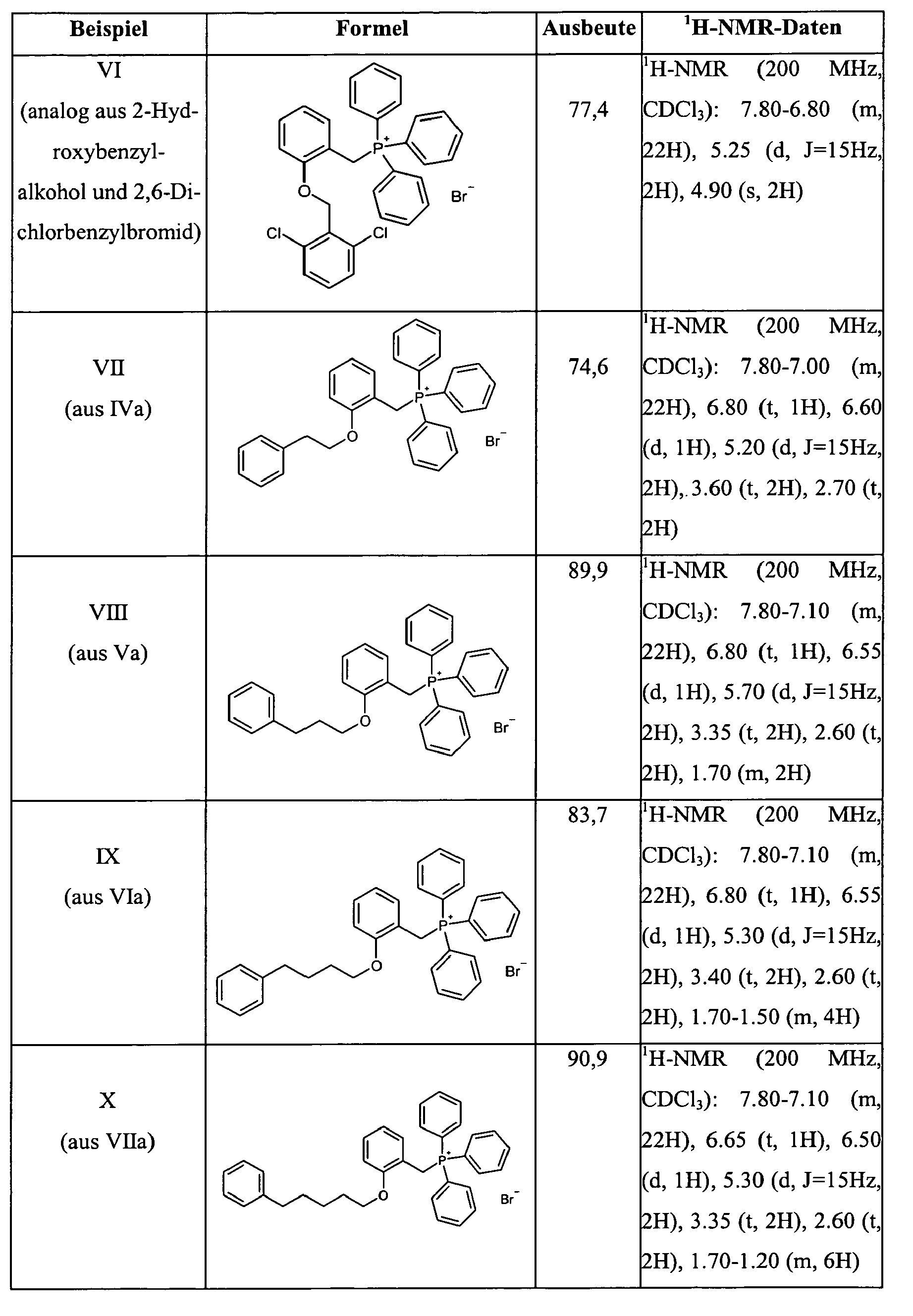

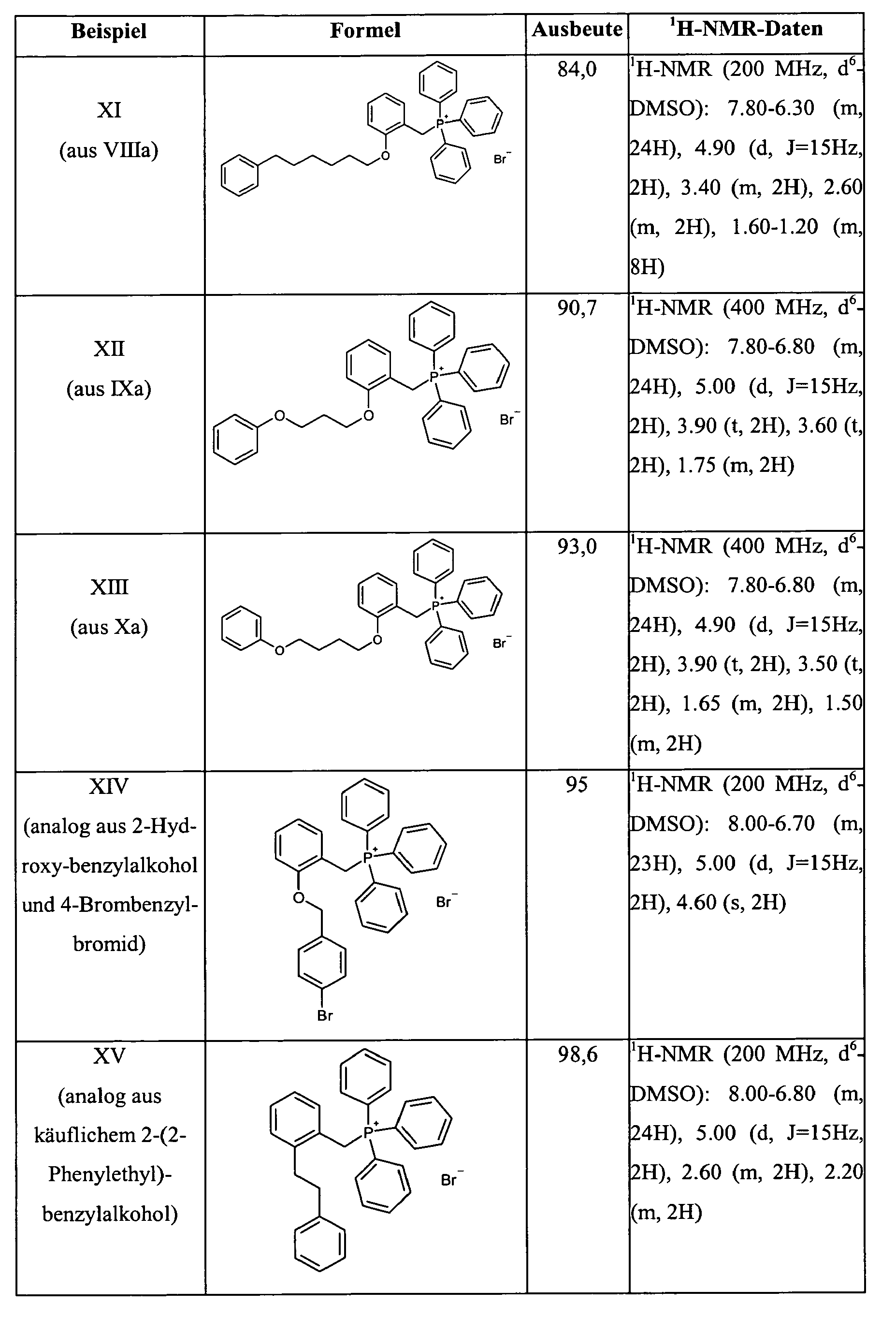

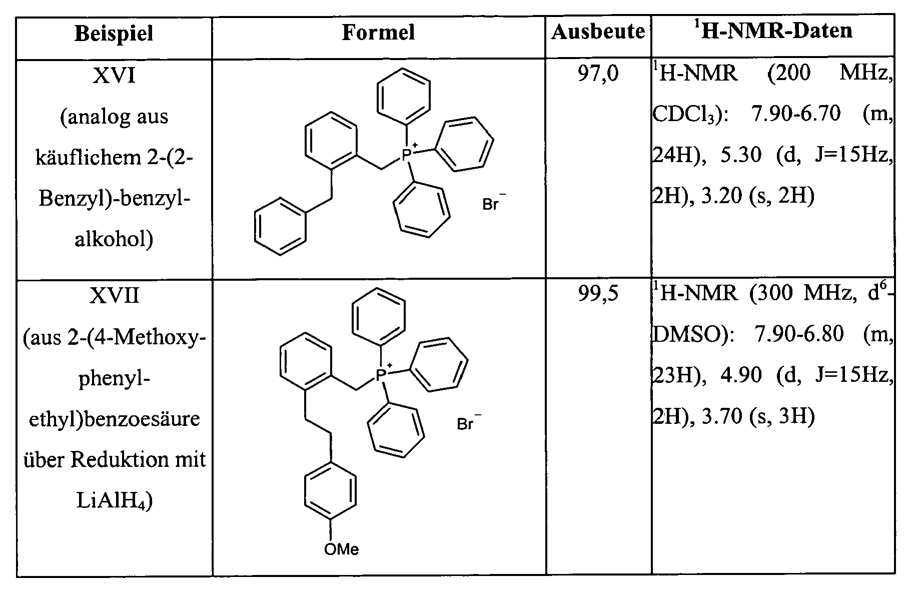

### Bsp. XVIII: 3-((4-Phenylbutoxy)phenethyl)triphenylphosphonium-4-methylbenzolsulfonat

### XVIIIa: 2-(3-(4-Phenylbutoxy)phenyl)ethanol

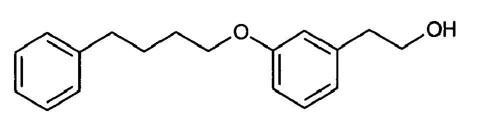

10,00 g (70 mmol) 2-(3-Hydroxyphenyl)ethanol, 15,42 g (70 mmol) 4-Phenylbutylbromid und 10,00g (70 mmol) Kaliumcarbonat werden in 45 ml 2-Propanol unter Rückfluß über Nacht erhitzt. Die Suspension wird abgekühlt, in Essigester aufgenommen und mit 1N Natronlauge und Wasser gewaschen, über Magnesiumsulfat getrocknet und im Vakuum eingedampft.

Ausbeute: 14,8 g (75,7% d. Th.)
R_{f}(SiO₂, C1E1): 0.40

### XVIIIb: 3-(4-Phenylbutoxy)phenethyl-4-methylbenzolsulfonat

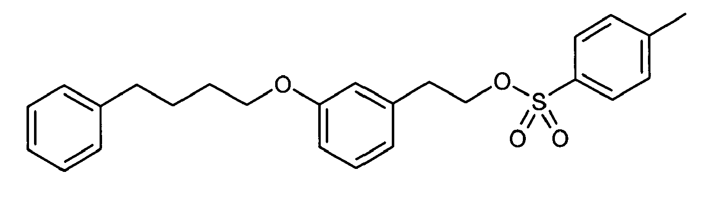

14,47 g (53,5 mmol) 2-(3-(4-Phenylbutoxy))ethanol aus XVIIIa wird in 50 ml und 35 ml Pyridin gelöst und 30 min bei Raumtemperatur gerührt. Die Lösung wird auf -10°C abgekühlt, und 12,20 g (64,2 mmol) 4-Toluolsulfonsäurechlorid werden zugegeben. Es wird 2,5 Stunden bei 0°C gerührt, Essigester und Wasser zugegeben, die Phasen abgetrennt und die wäßrige Phase einmal mit Essigester extrahiert. Die vereinigte organische Phasen werden einmal mit Wasser, zweimal mit 0,5N Salzsäure und einmal mit Natriumbicarbonatlösung gewaschen, über Magnesiumsulfat getrocknet und im Vakuum eingedampft.

Ausbeute: 21,59 g (95,0 % d. Th.)
R_{f}(SiO₂, C1E1): 0,53

### XVIII: 3-((4-Phenylbutoxy)phenethyl)triphenylphosphonium-4-methylbenzolsulfonat

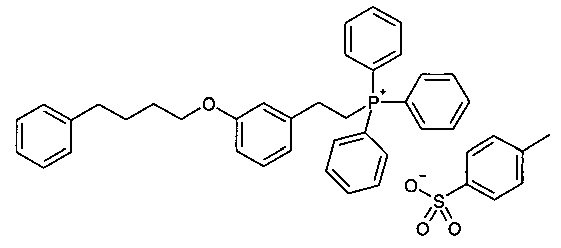

21,42 g (50,5 mmol) des Tosylats aus XVIIIb und 13,20 g (50,5 mmol) Triphenylphosphin werden in 150 ml Acetonitril unter Rückfluß 5 Tage erhitzt. Das Lösungsmittel wird im Vakuum eingedampft.

Ausbeute: 34,8 g (100,0 % d. Th.)
R_{f} (SiO₂, BABA): 0,52

### Bsp. XIX-XXVI : Darstellung von Aldehyden der Formel (II) aus Malonsäureestern

### XIXa: 1,1-Diallyl-2-methyl-1,1,2-ethantricarboxylat

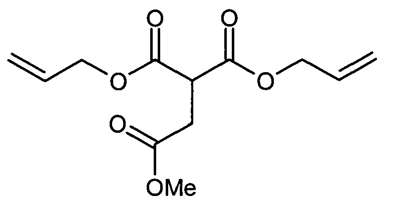

Zu einer Lösung von 12,04 g (65,4 mmol) Malonsäurebisallylester in 700 ml trockenem Dioxan wurde bei 5°C 1,18 g (49,03 mmol) Natriumhydrid portionweise zugegeben. Man rührte bis die Gasentwicklung beendet war, ersetzte das Kühlbad durch warmes Wasser (~40°C) und rührte 30 min. Anschließend wurde eine Lösung von 5,00 g (32,7 mmol) Bromessigsäuremethylester in 100 ml Dioxan zugetropft und man rührte bei RT (DC-Kontrolle, Cyclohexan/EE 20:1). Der gebildete Niederschlag wurde abfiltriert, das Lösungsmittel entfernt und der Rückstand in Wasser aufgenommen. Man extrahierte dreimal mit Diethylether, trocknete die vereinten organischen Phasen über Na₂SO₄ und entfernte das Lösungsmittel. Das Produkt wurde durch Blitzchromatographie an Kieselgel (Cyclohexan/EE 10: 1) gereinigt.

Ausbeute: 6,33 g (75,6 %) einer farblosen Flüssigkeit.
¹H NMR (300 MHz, CDCl₃):
δ = 2.94 (d, J = 7.4 Hz, 2H), 3.68 (s, 3H), 3.90 (t, J = 7.4 Hz, 1H), 4.62 - 4.67 (m, 4H), 5.21 - 5.36 (m, 4H), 5.81 - 5.95 (m, 2H).

Analog wurden unter Verwendung der entsprechenden α-Bromcarbonsäureester hergestellt:

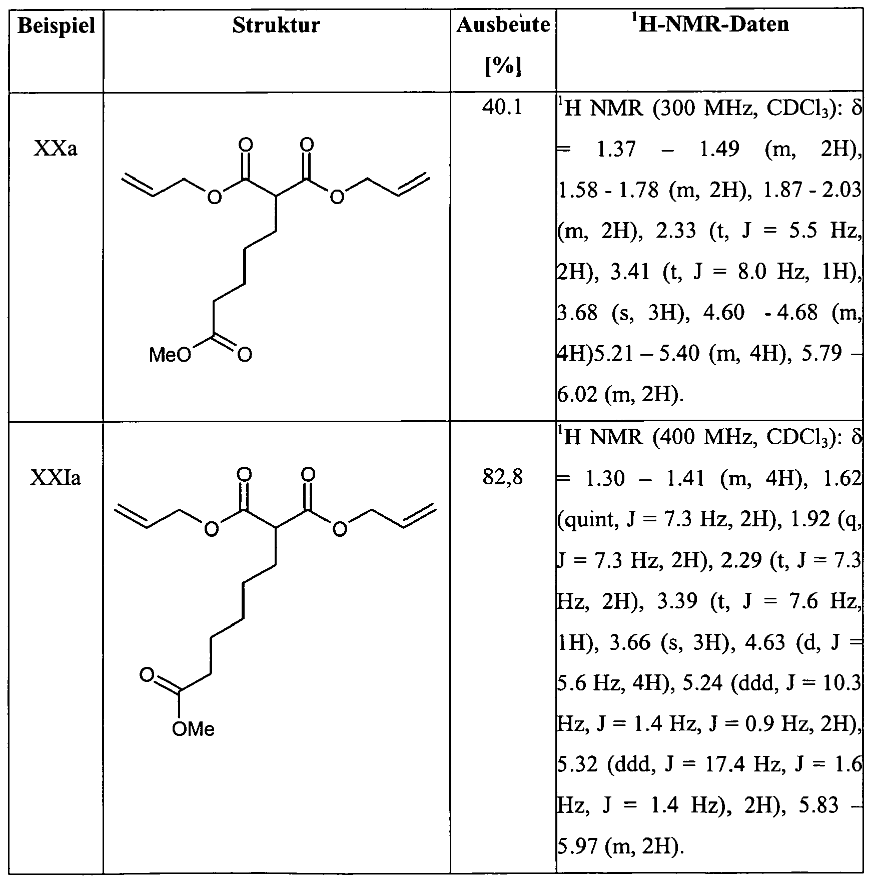

### XIXb: 2,2-Diallyl-1-methyl-3-[2-(methoxycarbonyl)phenyl]-1,1,2-propantricarboxylat

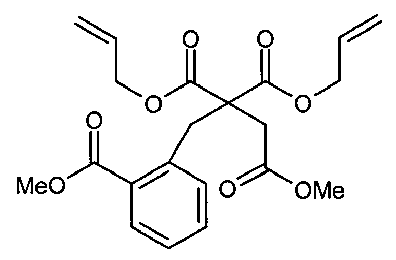

Zu einer Lösung von 510,0 mg (1,99 mmol) 2-Methyl-1,1-di(2-propenyl)-ester-1,1,2-ethanetricarbonsäure aus Bsp. XIXa in 5 ml DMF wurden potionsweise 49,2 mg (1,99 mmol) Natriumhydrid zugegeben. Nach beendeter Gasentwicklung wurde noch 20 min nachgerührt. Danach wurde eine Lösung von 844,3 mg (2,99 mmol) 2-(Bromomethyl)-benzoesäuremethylester in 6 ml DMF zugegeben und man rührte über Nacht. Man versetzte mit Wasser und extrahierte dreimal mit Diethylether, trocknete die vereinten organischen Phasen über Na₂SO₄ und entfernte das Lösungsmittel. Das Produkt wurde chromatographisch (20 g Kieselgel, Cyclohexan/EE 10:1) gereinigt.

Ausbeute: 680,0 mg (84,5 %) einer farblosen Flüssigkeit.
¹H NMR (300 MHz, CDCl₃):
δ = 2.87 (s, 2H), 3.66 (s, 3H), 3.86 (s, 3H), 3.94 (s, 2H), 4.57 - 4.62 (m, 4H), 5.21 (dq, J = 10.5 Hz, J = 2.7 Hz, 2H), 5.28 (dq, J = 17.2 Hz, J = 3.0 Hz, 2H), 5.78 - 5.92 (m, 2H), 7.18 - 7.43 (m, 3H), 7.85 (dd, J = 7.6 Hz, J = 1.6 Hz, 1H).

### Analog wurden hergestellt:

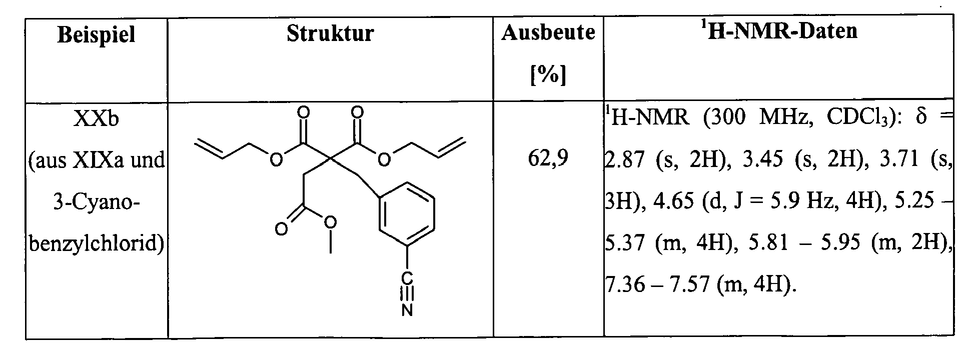

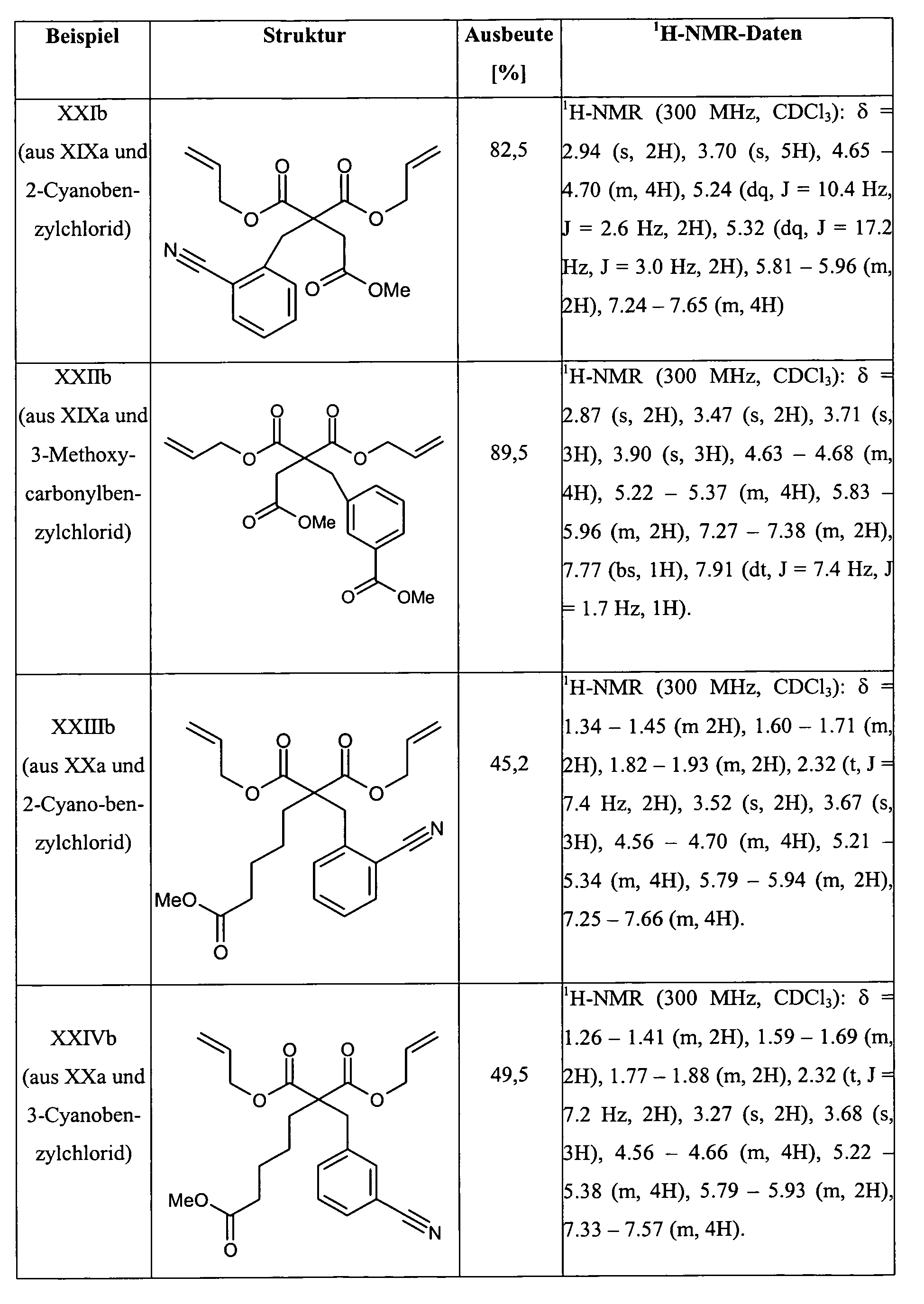

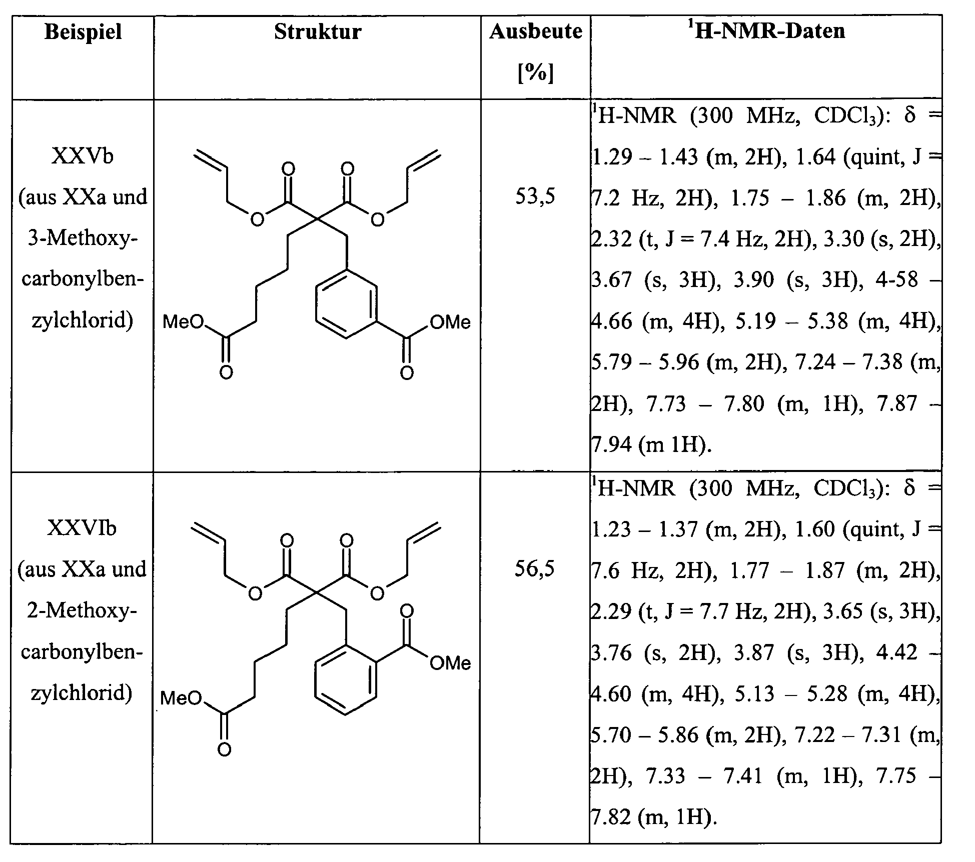

### XIXc: 4-Methoxy-[2-(methoxycarbonyl)benzyl]-4-oxobutansäure

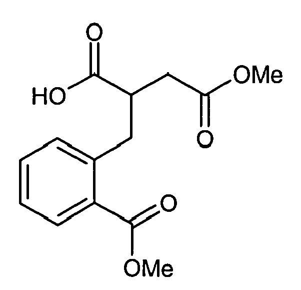

Zu einer Lösung von 610 mg (1,51 mmol) 3-[2-(Methoxycarbonyl)phenyl]-1-methyl-2,2-di(2-propenyl)-ester-1,2,2-propantricarbonsäure aus Bsp. XIXb, 32 mg (0,12 mmol) Triphenylphosphin und 7,0 mg (3,0 mmol) Palladium-(II)-acetat in 6 ml Dioxan wurde eine Lösung von 504 mg (4,98 mmol) Triethylamin und 177 mg (3,77 mmol) Ameisensäure in 2 ml Dioxan zugegeben. Man erhitzt 2 h auf 100°C und rührt anschließend weitere 16 h bei Raumtemperatur. Das Lösungsmittel wurde entfernt und der Rückstand über Kieselgel filtriert (Cyclohexan/Ethylacetat 10:1 zum Eluieren der Nebenprodukte, Cyclohexan/ Ethylacetat/ Essigsäure 5:1:1 zum Eluieren des Produktes).

Ausbeute: 418 mg (98,8 %) eines leicht gelblichen Öls
¹H-NMR (300 MHz, CDCl₃):
δ = 2.48 (dd, J = 16.8 Hz, J = 4.1 Hz, 1H), 2.75 (dd, J = 16.8 Hz, J = 9.3 Hz, 1H), 3.08 (dd, J = 12.9 Hz, J = 8.3 Hz, 1H), 3.18 - 3.28 (m, 1H), 3.51 (dd, J = 12.9 Hz, J = 5.9 Hz, 1H), 3.63 (s, 3H), 3.92 (s, 3H), 7.22 - 7.71 (m, 3H), 7.98 (d, J = 7.7 Hz, 1H).

### Analog wurden hergestellt:

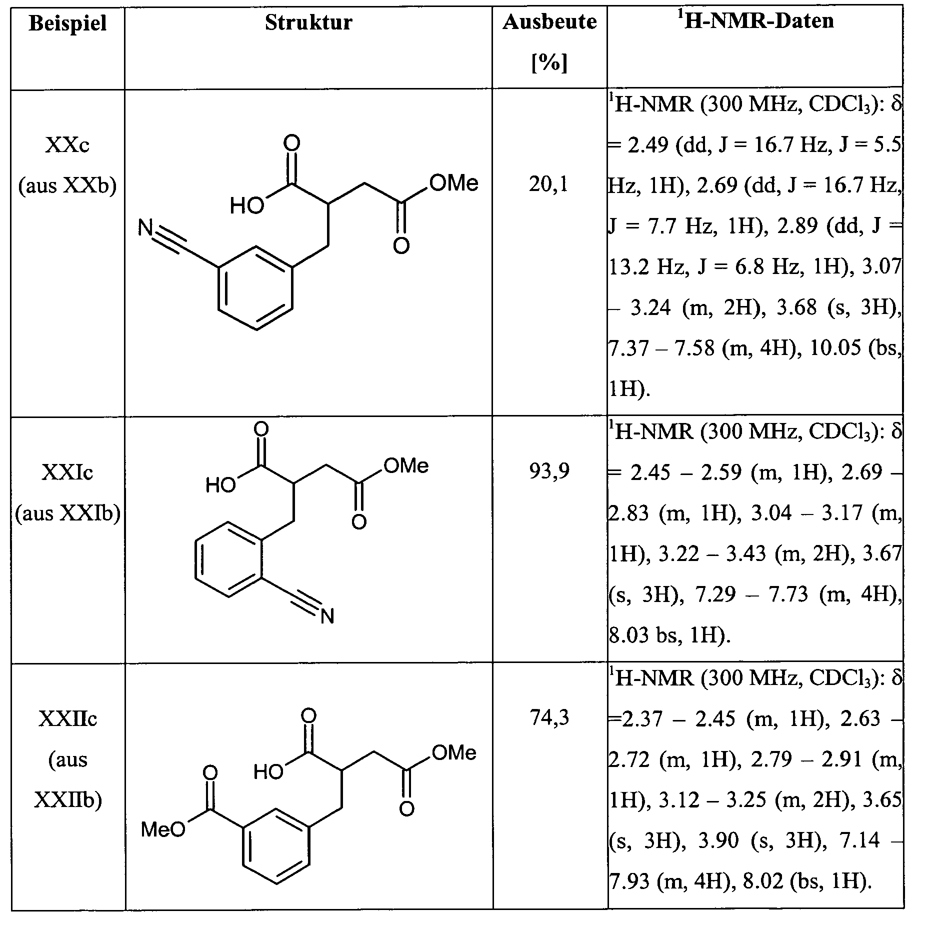

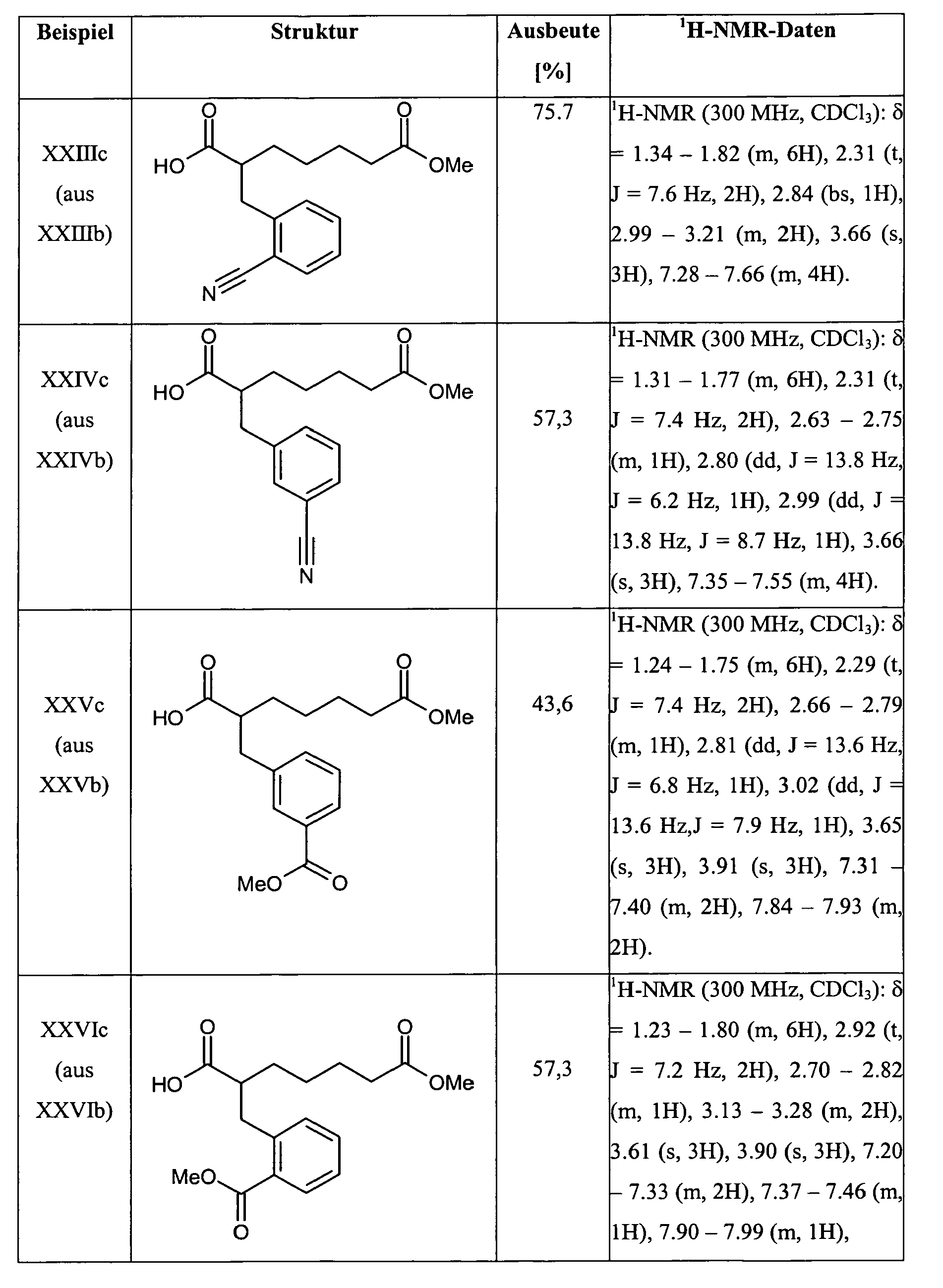

### XIXd: Methyl-2-[2-(hydroxymethyl)-4-methoxy-4-oxobutyl]benzoat

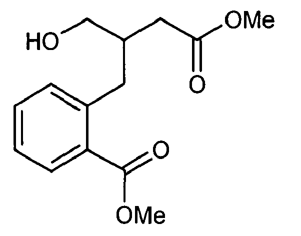

Zu einer Lösung von 470 mg (1,68 mmol) 2-[[2-(Methoxycarbonyl)phenyl]methyl]-butandisäure-4-methylester aus Bsp. XIXc in 5 ml THF wurden bei -10°C 2,16 ml (2,16 mmol) Boran-THF (1 M Lösung in THF) zugegeben. Man ließ die Mischung auf Raumtemperatur erwärmen und rührte 16 h. Die Lösung wurde vorsichtig mit Wasser versetzt und man extrahiert dreimal mit Ethylacetat. Die vereinten organischen Phasen wurden über Na₂SO₄ getrocknet und das Lösungsmittel wurde entfernt. Das Produkt wurde chromatographisch gereinigt (Kieselgel, Cyclohexan/Ethylacetat 1:1).

Ausbeute: 266 mg (39,7 %) eines leicht gelblichen Öls.
¹H-NMR (300 MHz, CDCl₃):
δ = 1.56 (bs, 1H), 2.29 - 2.41 (m, 1H), 2.40 - 2.60 (m, 2H), 2.77 (dd, J = 13.2 Hz, J = 6.1 Hz, 1H), 3.26 (dd, J = 13.2 Hz, J = 8.5 Hz, 1H), 3.45 - 3.57 (m, 1H), 3.66 (s, 3H), 3.90 (s, 3H), 7.23 - 7.35 (m, 2H), 7.45 (dt, J = 7.4 Hz, J = 1.3 Hz, 1H), 7.88 (dd, J = 7.7 Hz, J = 1.3 Hz, 1H).

### Analog wurden hergestellt:

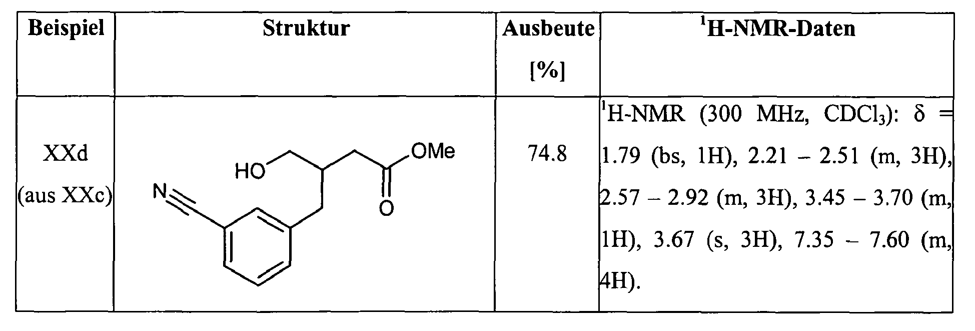

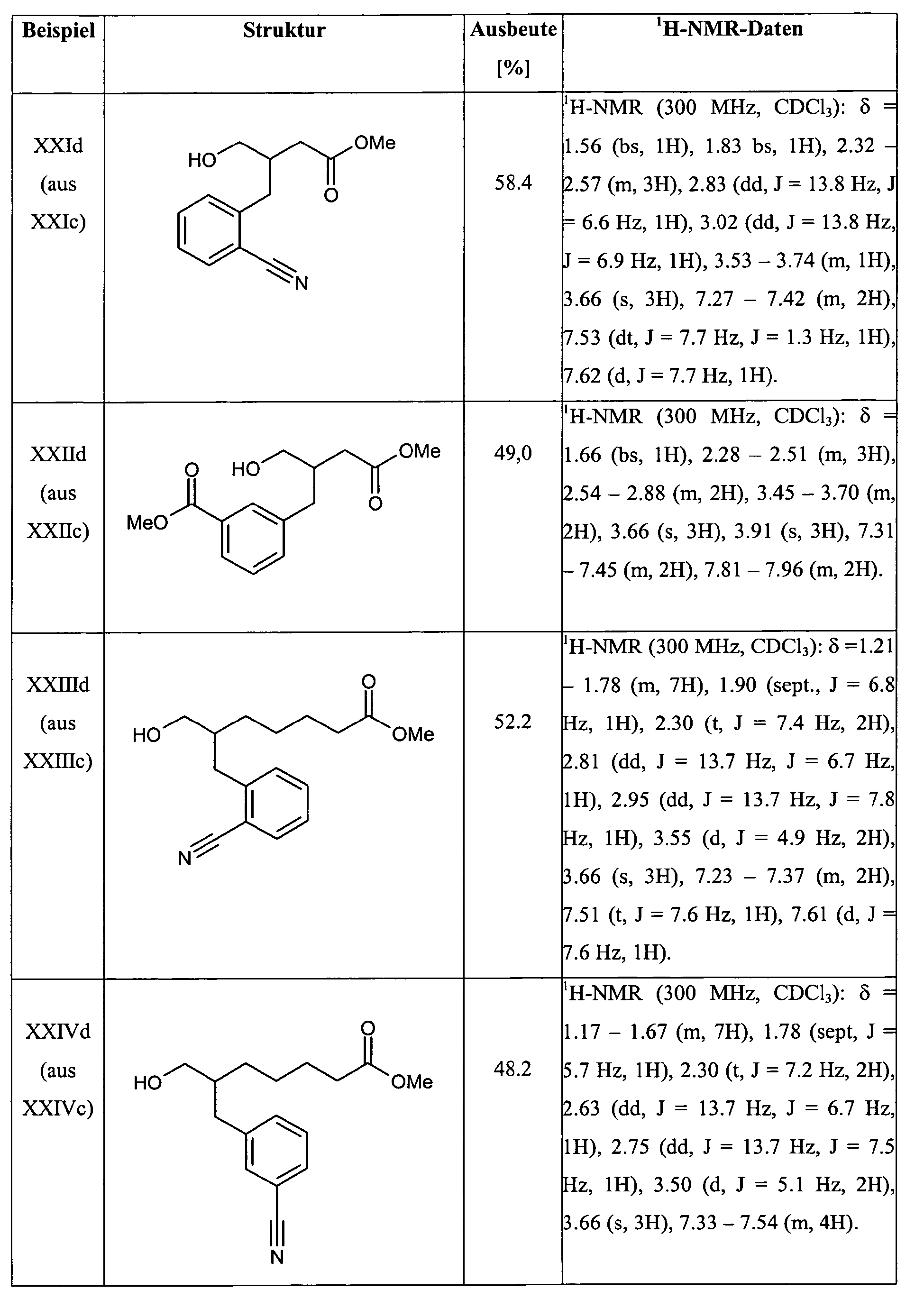

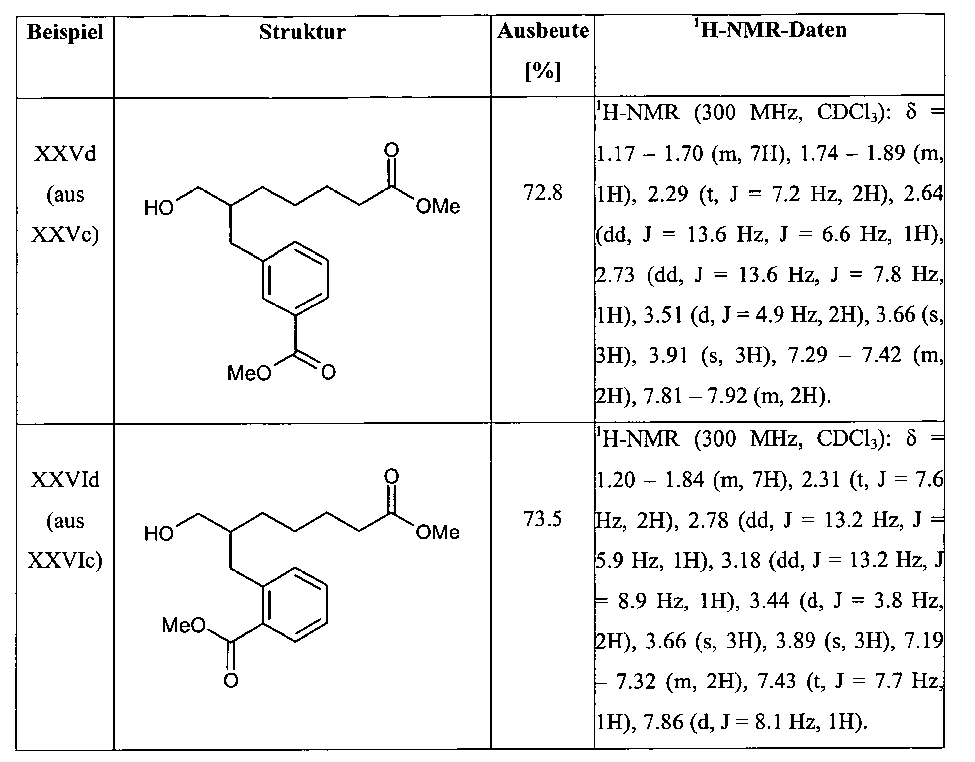

### XXIII: 6-(2-Cyanobenzyl)-7-oxoheptansäuremethylester

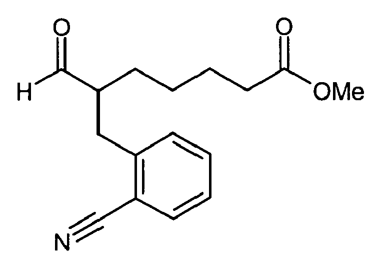

Zu einer Lösung von 40,10 mg (0,32 mmol) Oxalylchlorid in 2 ml Dichlormethan wurde bei -60°C eine Lösung von 49.37 mg (0.63 mmol) Dimethylsulfoxid (DMSO) in 0,5 ml Dichlormethan zugetropft. Nachdem 15 min bei -60°C gerührt wurde, tropfte man eine Lösung von 58,00 mg (0,21 mmol) 6-(2-Cyanobenzyl)-7-hydroxyheptansäuremethylester aus Bsp. XXIIId in 1 ml Dichlormethan zu. Nachdem weitere 15 min bei -60°C gerührt wurde, tropfte man 106,58 mg (1,05 mmol) Triethylamin zu, entfernte das Kühlbad und rührte 2 h. Man versetzte die Mischung mit Wasser, extrahierte mit Dichlormethan, trocknete die vereinten organischen Phasen über Na₂SO₄, entfernte das Lösungsmittel und trocknete das Rohprodukt im Hochvakuum. Der erhaltene Aldehyd konnte ohne weitere Reinigung verwendet werden.

### Analog wurden hergestellt und ohne weitere Reinigung eingesetzt:

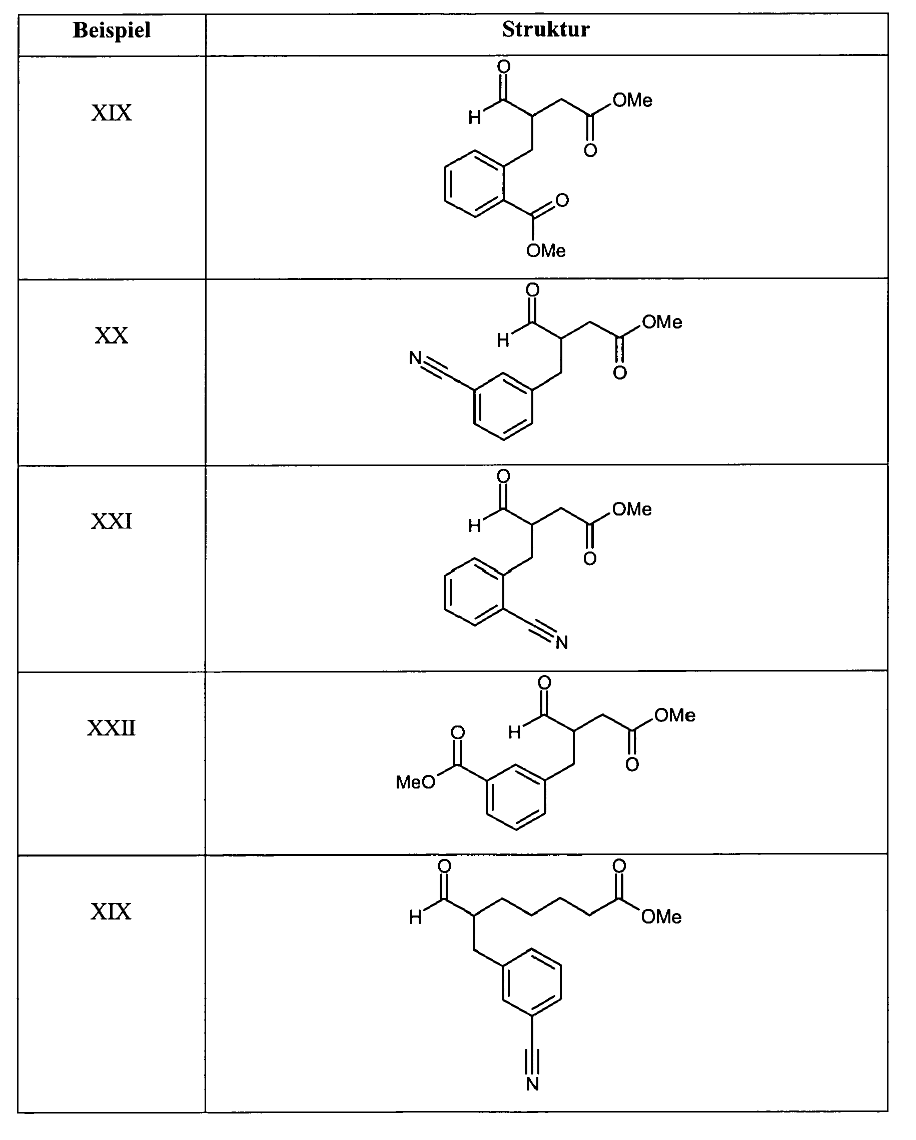

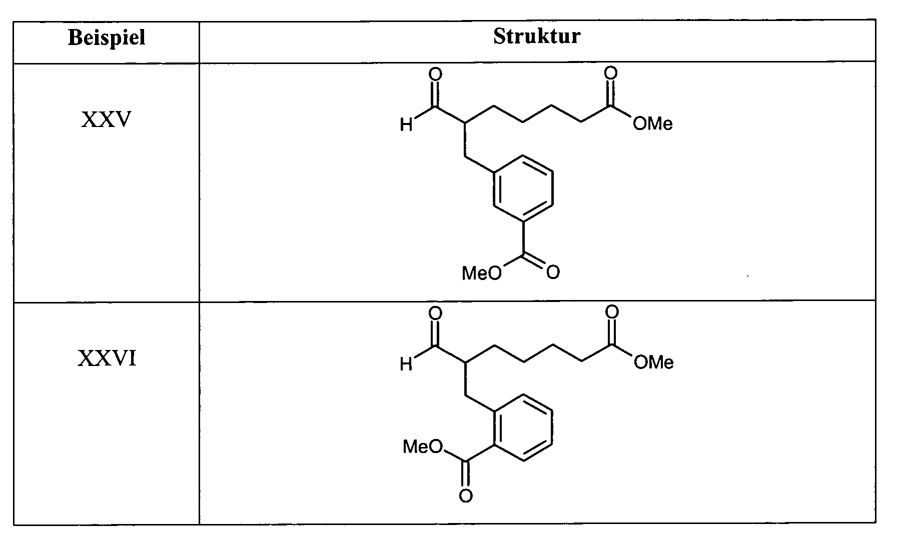

### Beispiel XXVII: Synthese von Benzaldehydderivaten

### XXVIIa: 2-[(5-Phenylpentyl)oxy]benzaldehyd

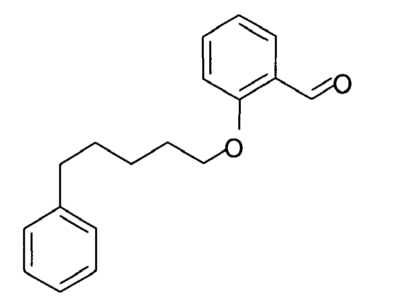

Zu einer Lösung von 7,75 g (61,03 mmol) Oxalychlorid in 100 ml CH₂Cl₂ wurde bei -60°C eine Lösung von 9,54 g (122,05 mmol) Dimethylsulfoxid in 50 ml CH₂Cl₂ zugetropft. Man rührte 15 min bei -60°C und tropfte dann eine Lösung von 11,00 g (40,68 mmol) 2-[(5-Phenylpentyl)oxy]benzylalkohol aus Bsp. VIIa in 50 ml CH₂Cl₂ zu. Nachdem 15 min bei -60°C gerührt wurde, gab man 20,59 ml (203,42 mmol) Triethylamin zu. Man rührte eine Stunde bei -60°C, entfernte das Kühlbad und rührte eine Stunde. Man versetzte mit Wasser, extrahierte mit CH₂Cl₂, trocknete die vereinten organischen Phasen über Na₂SO₄ und entfernte das Lösungsmittel. Das Produkt wurde chromatografisch gereinigt (Kieselgel, Cyclohexan/EE 10:1). Man erhielt 9,68 g (88,7 %) einer farblosen Flüssigkeit.
¹H-NMR (300 MHz, CDCl₃): δ = 1.44 - 1.62 (m, 2H), 1.64 - 1.77 (m, 2H), 1.78 - 1.94 (m, 2H), 2.64 (t, *J* = 7.6 Hz, 2H), 4.05 (t, J = 6.2 Hz, 2H), 6.87 - 7.03 (m, 2H), 7.09 - 7.32 (m, 5H), 7.45 - 7.55 (m, 1H), 7.77 - 7.85 (m, 1H), 10.48 (s, 1H).

### XXVIIb: 2-{[tert-Butyl(dimethyl)silyl]oxy}benzaldehyd

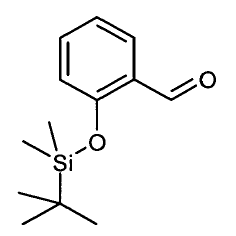

Zu einer Lösung von 10,00 g (81,89 mmol) Salicylaldehyd und 6,13 g (90,07 mmol) Imidazol in 82 ml DMF wurden 13,58 g (90,07 mmol) t-Butyldimethylsilylchlorid (TBDMSC1) zugegeben. Man rührte bei Raumtemperatur und kontrollierte die Reaktion per Dünnschichtchromatographie (Cyclohexan/EE 10:1). Die Mischung wurde mit 1 N NaOH versetzt und mit Petrolether extrahiert. Die vereinten organischen Phasen wurden über Na₂SO₄ getrocknet, das Lösungsmittel entfernt und das Produkt chromatografisch gereinigt (Kieselgel, Cyclohexan/EE 10:1). Man erhielt 16,94 g (87,5 %) einer klaren Flüssigkeit.
¹H-NMR (300 MHz, CDCl₃): δ = 0.18 (s, 6H), 0.92 (s, 9H), 6.78 (d, J = 8.3 Hz, 1H), 6.93 (t, *J* = 7.7 Hz, 1H), 7.36 (dt, *J=* 8.1 Hz, *J* = 1.9 Hz, 1H), 7.71 (dd, *J* = 9.3 Hz, *J=* 1.5 Hz, 1H), 10.37 (s, 1H).

### Bsp. XXVIII: 7-(Diethoxyphosphoryl)-6-oxoheptansäuremethylester

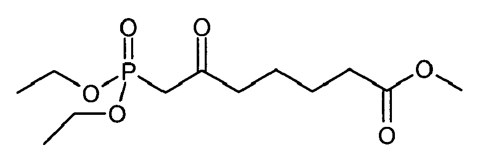

Zu einer Lösung von 15,00 g (74,95 mmol) Phosphonoessigsäurediethylester in 400 ml Toluol wurden bei 0°C 30,34 g (299,79 mmol) Triethylamin sowie 12,21 g (112,42 mmol) Trimethylchlorsilan zugetropft. Man rührte 1 h bei Raumtemperatur und fügte 7,14 g (74,95 mmol) Magnesiumchlorid zu. Man rührte eine Stunde und tropfte 16,56 g (89,94 mmol) Adipinsäuremonomethylesterchlorid zu. Die Mischung wurde 24 h bei Raumtemperatur gerührt. Es wurde mit Wasser versetzt. Man extrahierte mit Diethylether, trocknete die organischen Phasen über Na₂SO₄ und entfernte das Lösungsmittel. Das Produkt wurde chromatografisch gereinigt (Kieselgel, Ethylacetat). Man erhielt 7,83 g (35,5 %) einer klaren Flüssigkeit.
¹H NMR (300 MHz, CDCl₃): δ = 1.34 (t, J = 6.9 Hz, 6H), 1.59 - 1.66 (m, 4H), 2.25 - 2.40 (m, 2H), 2.59 - 2.70 (m, 2H), 3.07 (d, J = 22.9 Hz, 2H), 3.66 (s, 3H), 4.14 (quint, *J* = 7.2 Hz, 4H).

### Bsp. XXIX: Synthese von 6-Oxo-7-octensäurederivaten

### XXIXa: (E)-6-oxo-8-{2-[(5-phenylpentyl)oxy]phenyl}-7-octensäuremethylester

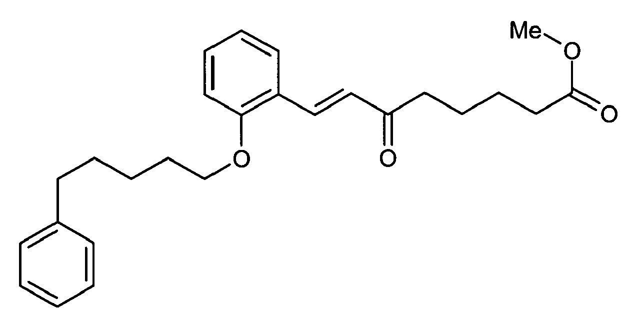

Zu einer Lösung von 3,20 g (10,87 mmol) 7-(Diethoxyphosphoryl)-6-oxoheptansäuremethylester aus Bsp. XXVIII in 53 ml THF wurden unter Argon 0,26 g (10,87 mmol) Natriumhydrid zugegeben. Man rührte 30 min bei Raumtemperatur und tropfte eine Lösung von 2,43 g (9,06 mmol) 2-[(5-phenylpentyl)oxy]benzaldehyd aus Bsp. XVIIa in 20 ml THF zu und rührte 18 h bei Raumtemperatur. Man versetzte mit Wasser, extrahierte mit Ethylacetat, trocknete die vereinten organischen Phasen über Na₂SO₄ und entfernte das Lösungsmittel. Das Produkt wurde chromatografisch gereinigt (Kieselgel, Cyclohexan/EE 10:1). Man erhielt 2,51 g (67,8 %) einer farblosen Flüssigkeit.
¹H-NMR (300 MHz, CDCl₃): δ = 1.49 - 1.60 (m, 2H), 1.63 - 1.79 (m, 6H), 1.84 - 1.95 (m, 2H), 2.35 (t, *J* = 6.8 Hz, 2H), 2.66 (t, *J* = 7.9 Hz, 4H), 3.66 (s, 3H), 4.03 (t, *J* = 6.6 Hz, 2H), 6.79 (d, *J* = 16.3 Hz, 1H), 6.85 - 6.98 (m, 2H), 7.11 - 7.37 (m, 6H), 7.47 - 7.57 (m, 1H), 7.89 (d, *J* = 16.3 Hz, 1H).

### Bsp. XXIXb: (E)-8-(2-{[tert-Butyl(dimethyl)silyl]oxy}phenyl)-6-oxo-7-octensäuremethylester

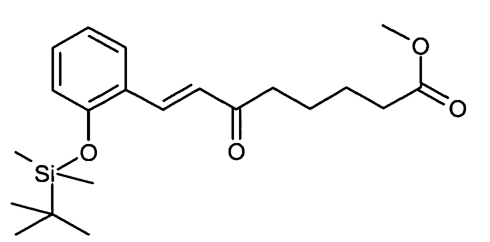

Diese Verbindung wurde analog zu Bsp. XXIXa aus den Verbindungen XVIIb und XXVIII hergestellt.
¹H-NMR (300 MHz, CDCl₃): δ = 0.24 (s, 6H), 1.05 (s, 9H), 1.62 - 1.77 (m, 4H), 2.29 - 2.41 (m, 2H), 2.62 - 2.73 (m, 2H), 3.66 (s, 3H), 6.67 (d, *J* = 16.6 Hz, 1H), 6.84 (m_{c} = 1H), 6.96 (t, *J* = 7.6 Hz, 1H), 7.20 - 7.30 (m, 1H), 7.56 (d, *J* = 7.7 Hz, 1H), 7.96 (d, *J* = 16.6 Hz, 1H).

### Bsp. XXX: Synthese von 6-hydroxy-7-octensäurederivaten

### XXXa: (E)-6-Hydroxy-8-{2-[(5-phenylpentyl)oxy]phenyl}-7-octensäuremethylester

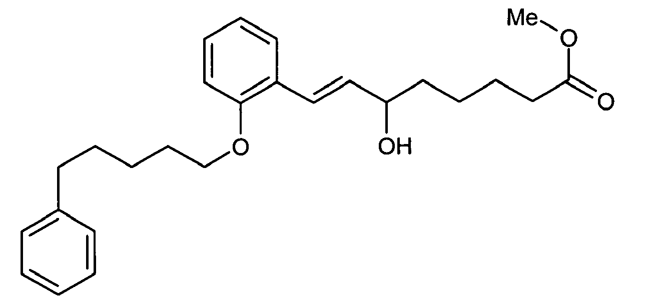

Zu einer Lösung von 1,436 g (3,86 mmol) CeCl₃·7H₂O und 1,50 g (3,67 mmol) (E)-6-oxo-8-{2-[(5-phenylpentyl)oxy]phenyl}-7-octensäuremethylester aus Bsp. XXIXa in 30 ml Methanol wurden bei 0°C 0,146 g (3,86 mmol) Natriumborhydrid zugegeben. Man rührte die Mischung bei 0°C und kontrollierte den Reaktionsverlauf per Dünnschichtchromatografie. Man versetzte mit gesättigter NH₄Cl-Lösung, extrahierte mit Ethylacetat und trocknete die vereinten organischen Phasen über Na₂SO₄. Das Produkt wurde chromatografisch gereinigt (Kieselgel, Cyclohexan/EE 10:2). Man erhielt 1,38 g (91,5 %) einer farblosen Flüssigkeit.
¹H-NMR (400 MHz, CDCl₃): δ = 1.37 - 1.76 (m, 10H), 1.85 (quint, J = 6.6 Hz, 2H), 2.32 (t, *J =* 7.6 Hz, 2H), 2.65 (t, J = 7.6 Hz, 2H), 3.65 (s, 3H), 3.98 (t, J = 6.6 Hz, 2H), 4.25 (q, *J* = 6.4 Hz, 2H), 6.22 (dd, *J* = 15.9 Hz, *J* = 7.1 Hz, 1H), 6.80 (m, 3H), 7.13 - 7.32 (m, 6H), 7.39 - 7.46 (m, 1H).

### Bsp. XXXb: (E)-8-(2-{[tert-Butyl(dimethyl)silyl]oxy}phenyl)-6-hydroxy-7-octensäuremethylester

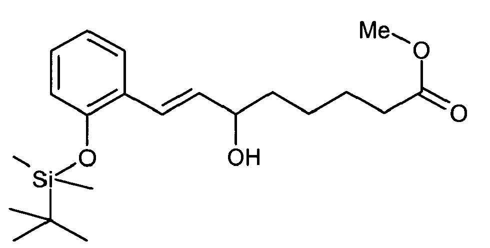

Diese Verbindung wurde analog zu Bsp. XXXa aus der Verbindung XIXb hergestellt.
¹H NMR (400 MHz, CDCl₃): δ = 0.01 (s, 6H), 0.80 (s, 9H), 1.13 - 1.54 (m, 7H), 2.11 (t *J* = 7.3 Hz, 2H), 3.44 (s, 3H), 3.99 - 4.11 (m, 1H), 5.93 (dd, *J=* 15.9 Hz, *J* = 6.9 Hz, 1H), 6.57 (dd, *J* = 8.0 Hz, *J=* 1.0 Hz, 1H), 6.63 - 6.73 (m, 2H), 6.90 (dt, *J* = 8.0 Hz, *J* = 1.7 Hz, 1H), 7.23 (dd, *J =* 7.8 Hz, *J* = 1.7 Hz, 1H).

### Bsp. XXXI: Synthese von 6-Hydroxy-octansäurederivaten

### XXXIa: 6-Hydroxy-8-{2-[(5-phenylpentyl)oxy]phenyl}octansäuremethylester

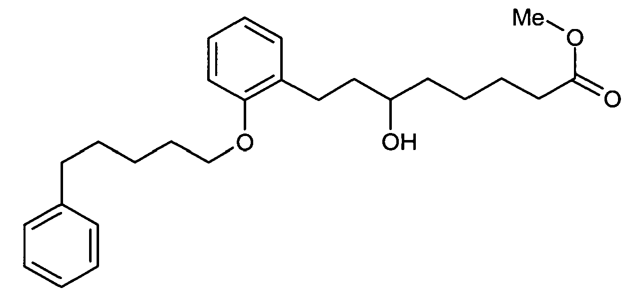

Zu einer Lösung von 1,80 g (4,38 mmol) (E)-6-Hydroxy-8-{2-[(5-phenylpentyl)oxy]phenyl}-7-octensäuremethylester aus Bsp. XXXa in 22,5 ml Ethylacetat wurden 30 mg Palladium auf Kohle (10 %) zugegeben. Man rührte unter einer Wasserstoffatmosphäre bis keine weitere Absorption zu beobachten war, filtrierte über Celite und entfernte das Lösungsmittel. Man erhielt 1,76 g (97,3 %) einer farblosen Flüssigkeit.
¹H-NMR (300 MHz, CDCl₃): δ = 1.21 - 1.90 (m, 15H), 2.29 (t, *J* = 7.4 Hz, 2H), 2.64 (t, *J* = 7.4 Hz, 2H), 2.67 - 2.84 (m, 2H), 3.41 - 3.54 (bs, 1H), 3.64 (s, 3H), 3.88 - 4.08 (m, 2H), 6.78 - 6.92 (m, 2H), 7.07 - 7.21 (m, 5H), 7.21 - 7.32 (m, 2H).

### Bsp. XXXIb: 8-(2-{[tert-Butyl(dimethyl)silyl]oxy}phenyl)-6-hydroxyoctansäuremethylester

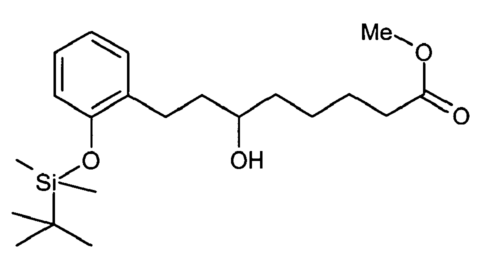

Ausbeute: 82%
¹H NMR (300 MHz, CDCl₃): δ = 0.25 (s, 3H), 0.26 (s, 3H), 1.03 (s, 9H), 1.20 - 1.84 (m, 9H), 2.26 - 2.38 (m, 2H), 2.66 - 2.78 (m, 2H), 3.49 - 3.62 (m, 1H), 3.67 (s, 3H), 6.75 - 6.84 (m, 1H), 6.85 - 6.94 (m, 1H), 7.02 - 7.19 (m, 2H).

### Bsp. XXXII: 6-Bromo-8-{2-[(5-phenylpentyl)oxy]phenyl}octansäuremethylester

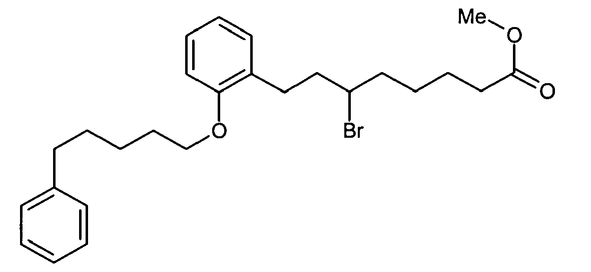

Zu einer Lösung von 1,00 g (2,42 mmol) 6-Hydroxy-8-{2-[(5-phenylpentyl)oxy]-phenyl}octansäuremethylester aus Bsp. XXXIa in 5 ml Diethylether wurden 0,30 g (1,09 mmol) Phosphortribromid bei 0°C zugetropft. Man rührte 1 h bei 0°C und 16 h bei Raumtemperatur. Man versetzte mit Wasser, extrahierte mit Petrolether, trocknete die vereinten organischen Phasen über Na₂SO₄ und entfernte das Lösungsmittel. Das Produkt wurde chromatografisch gereinigt (Kieselgel, Cyclohexan/EE 10:2). Man erhielt 0,62 g (53,7 %) einer farblosen Flüssigkeit.
¹H-NMR (300 MHz, CDCl₃): δ = 1.40 - 1.90 (m, 12 H), 2.00 - 2.14 (m, 2H), 2.30 (t, *J* = 7.2 Hz, 2H), 2.65 (t, *J* = 7.7 Hz, 2H), 2.65 - 2.75 (m, 1H), 2.80 - 2.96 (m, 1H), 3.65 (s, 3H), 3.89 - 4.02 (m, 3H), 6.76 - 6.90 (m, 2H), 7.09 - 7.21 (m, 5H), 7.22 - 7.31 (m, 2H).

### Synthesebeispiele

### Bsp.1: 8-(2-Benzyloxyphenyl)- 6-(4-methoxycarbonylbenzyl)-7-octensäuremethylester

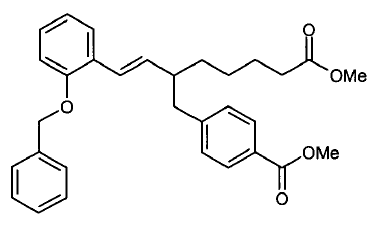

343,4 mg (0,64 mmol) 2-(Benzyloxy)benzyltriphenylphosphoniumbromid aus Bsp. III werden bei 0°C unter Argon in 20 ml THF suspendiert, und 0,48 ml Butyllithium (0,76 mmol, 1,6M Lösung in Hexan) werden hinzugegeben. Die tieforange Lösung wird 30 min. bei 0°C gerührt, und eine Lösung von 195 mg (0,64 mmol) 6-Formyl-7-(4-methoxycarbonylphenyl)heptansäuremethylester (hergestellt analog Beispiel I aus 2-Oxocyclohexancarbonsäure-t-butylester und 4-Chlormethylbenzoesäuremethylester, vgl. EP-A-0 341 551) in 15 ml THF wird bei dieser Temperatur tropfenweise hinzugegeben. Die Mischung wird 30 min. bei 0°C gerührt. Es wird bei 0°C mit Wasser versetzt, anschließend auf Raumtemperatur erwärmt und mit Essigsäureethylester extrahiert. Die organische Phase wird mit Natriumchloridlösung gewaschen, mit Magnesiumsulfat getrocknet und einrotiert. Die Substanz wurde zur Reinigung an 40 g Kieselgel 60 (Korngröße 0,040-0,063mm) mit Petrolether/Ether 4:1 bis 1:1 als Eluent chromatographiert.

Ausbeute: 154 mg (49.7% d.Th.) als eine Mischung: 71.0% Trans / 29.0% Cis.
¹H-NMR (200 MHz, CDCl₃): 7.90 (m, 2H), 7.40-6.80 (m, 11H), 6.60 (d, 0.7H, J=16 Hz), 6.50 (d, 0.3H, J=9 Hz), 5.95 (dd, 0.7H, J=16 Hz, J=9Hz), 5.40 (t, 0.3 H, J=9Hz), 5.00 (s, 1.4H), 4.90 (m, 0.6H), 3.85 (s, 3H), 3.60 (s, 3H), 2.80-2.40 (m, 3H), 2.30-2.10 (m, 2H), 1.60-1.20 (m, 6H)

### Analog wurden die folgenden Verbindungen hergestellt:

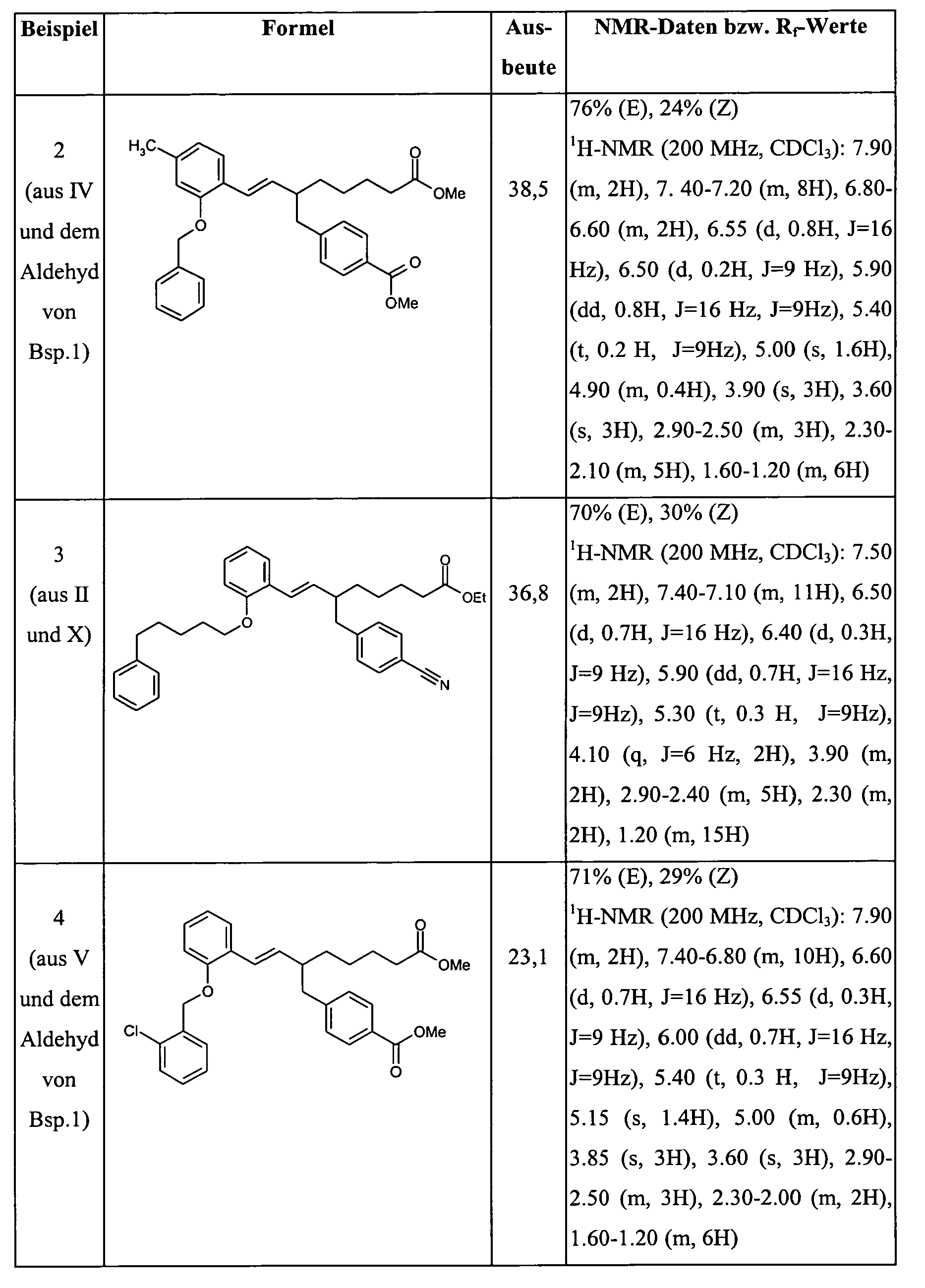

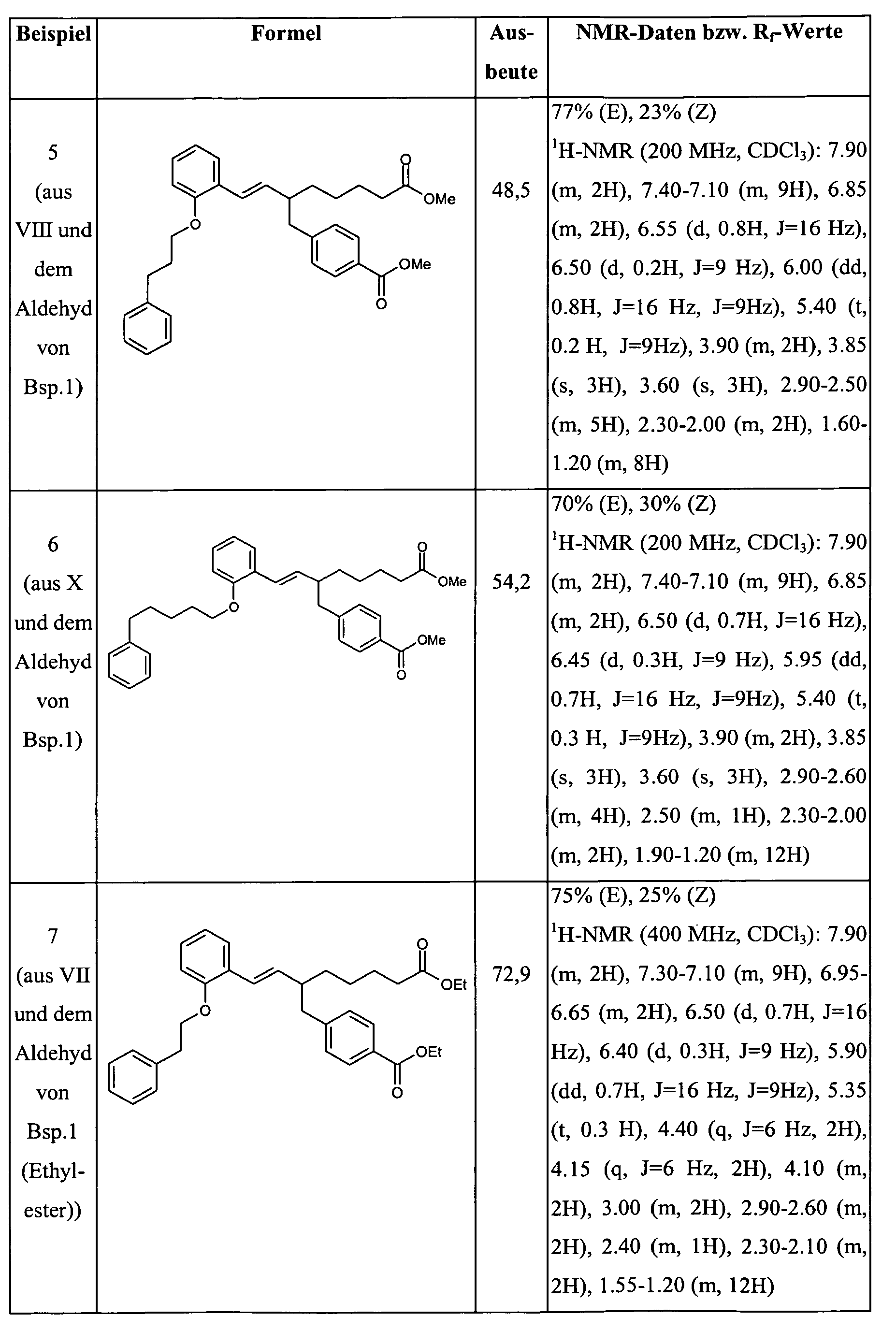

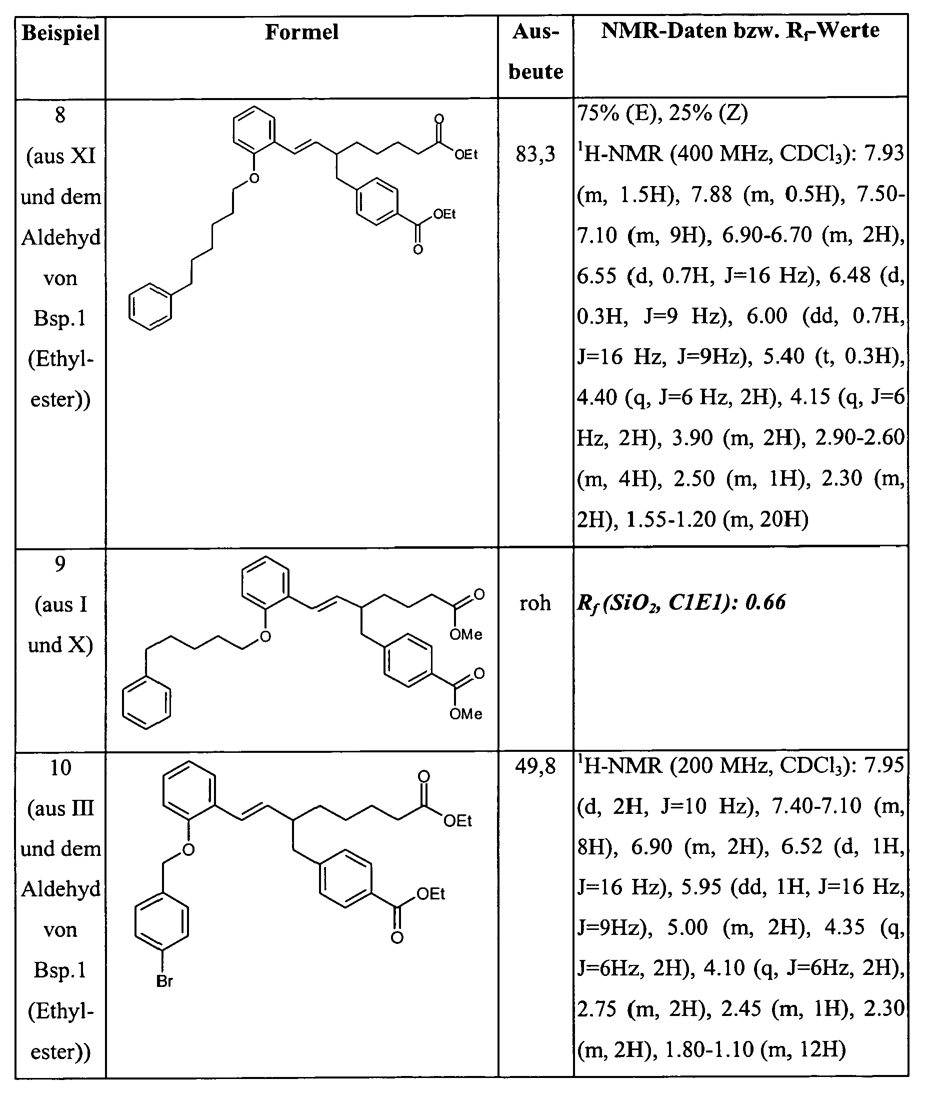

### Bsp. 11: 8-(2-Benzyloxyphenyl)-6-(4-carboxybenzyl)-7-octensäure

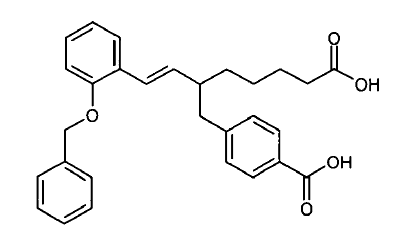

135 mg (0,28 mmol) des Diesters aus Beispiel 1 werden in 5 Methanol gelöst und bei 0°C mit 1,5 ml 45 %iger Natronlauge versetzt. Man läßt auf Raumtemperatur erwärmen und gibt 0,2 ml Dichlormethan hinzu. Die Lösung wird 16 Stunden bei Raumtemperatur gerührt, mit etwas Wasser versetzt und mit Ethylether extrahiert. Die wäßrige Phase wird mit 10 %iger Schwefelsäure auf pH 2-3 gestellt und zweimal mit Essigsäureethylester extrahiert und mit Magnesiumsulfat getrocknet. Das Lösungsmittel wird im Vakuum entfernt.

Ausbeute: 116 mg (91,2 % d.Th.) als eine Mischung: 71.0% Trans / 29.0% Cis.
¹H-NMR (400 MHz, CD₃COCD₃): 10.0 (bs, 2H), 7.90 (m, 2H), 7.40-6.80 (m, 11H), 6.60 (d, 0.7H, J=16 Hz), 6.50 (d, 0.3H, J=9 Hz), 6.10 (dd, 0.7H, J=16 Hz, J=9Hz), 5.50 (t, 0.3 H, J=9Hz), 5.10 (s, 1.4H), 5.00 (m, 0.6H), 2.90-2.50 (m, 3H), 2.30-2.10 (m, 2H), 1.60-1.20 (m, 6H)

### Analog zu Beispiel 11 wurden die folgende Substanzen synthetisiert:

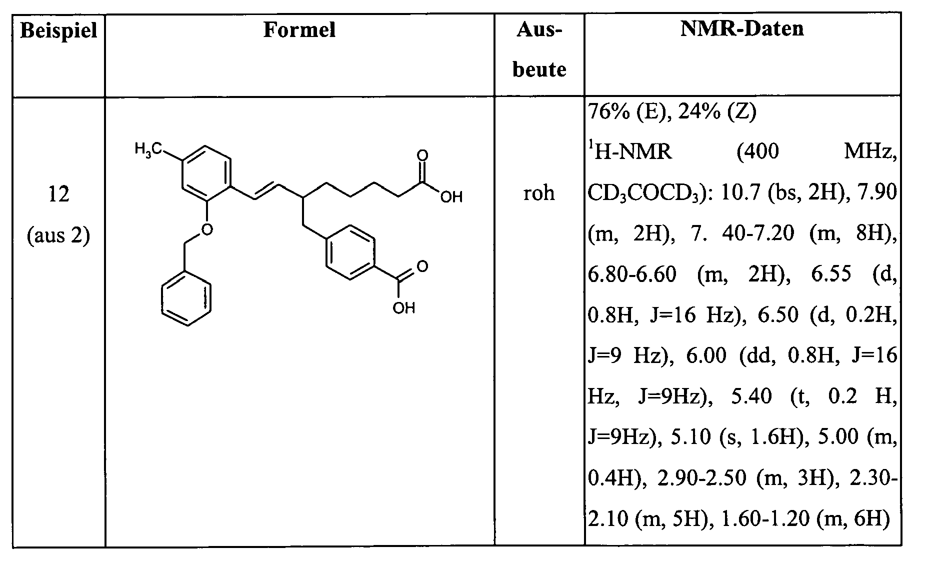

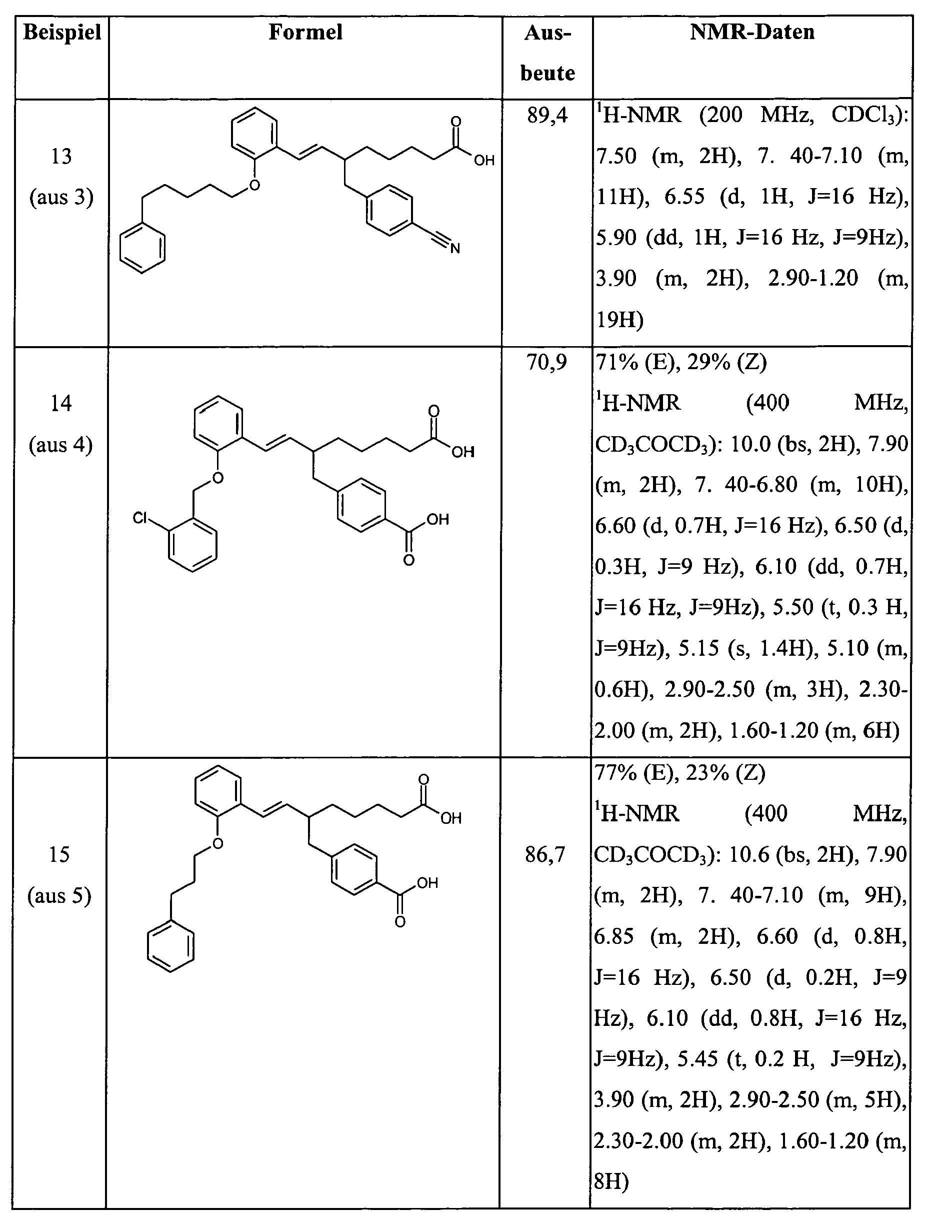

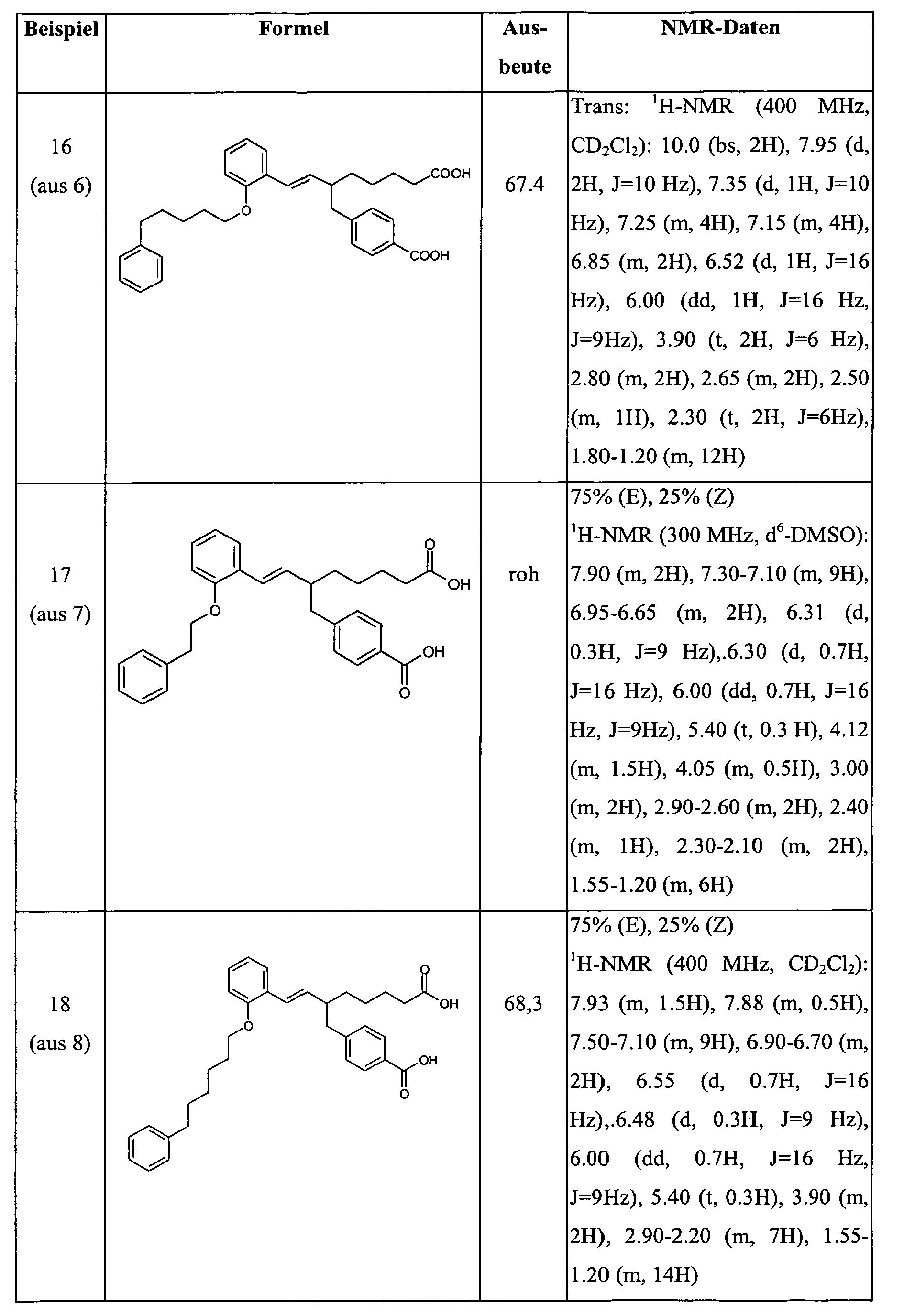

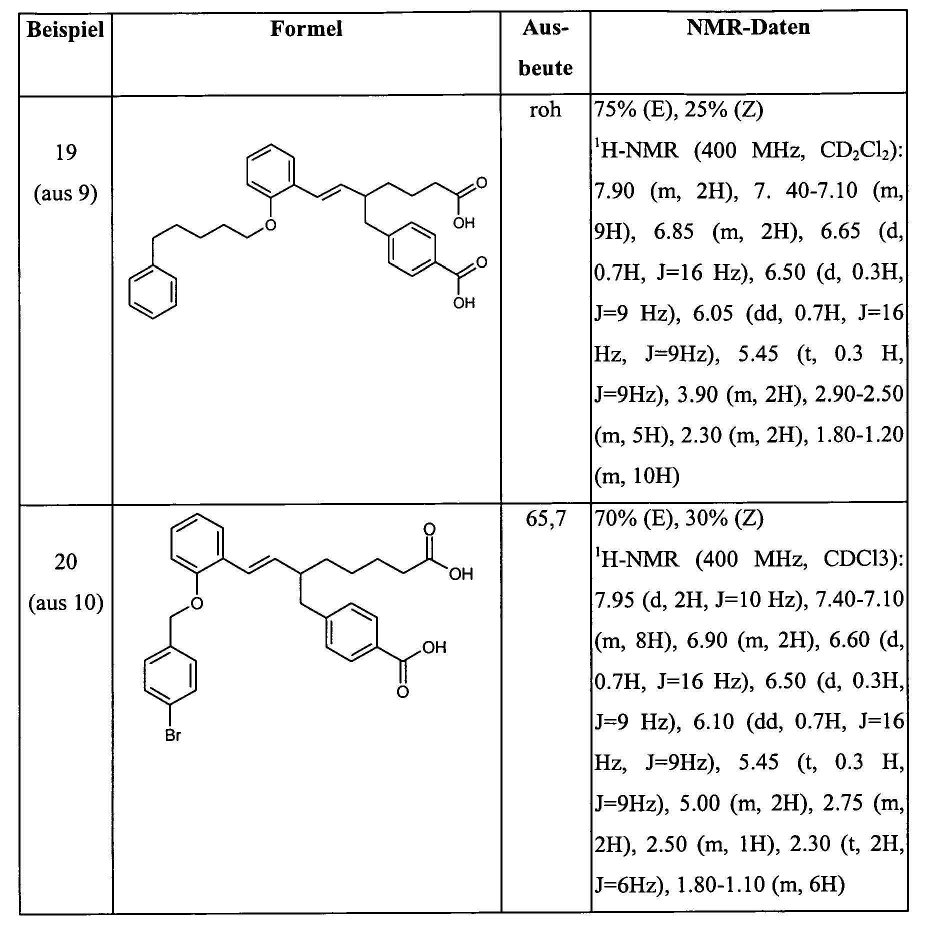

### Bsp. 21: Synthese von 8-(2-Benzylphenyl)-6-(4-carboxybenzyl)-7-octensäure

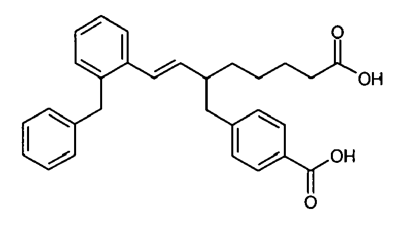

293,5 mg (0,56 mmol) 2-Benzylbenzyltriphenylphosphoniumbromid (XVI) werden bei 0°C unter Argon in 20 mL THF suspendiert und mit 0,42 ml Butyllithium (0,72 mmol, 1,6 M Lösung in Hexan) versetzt. Die tieforange Lösung wird 30 min. bei 0°C gerührt, und eine Lösung von 125 mg (0,37 mmol) 6-Formyl-7-(4-ethoxycarbonylphenyl)heptansäureethylester (hergestellt analog Beispiel I aus 2-Oxocyclohexancarbonsäure-t-butylester und 4-Chlormethylbenzoesäuremethylester, vgl. EP-A-0 341 551) in 15 ml THF wird bei dieser Temperatur tropfenweise hinzugegeben. Die Mischung wird 30 min. bei 0°C gerührt. Es wird bei 0°C mit Wasser versetzt, anschließend auf Raumtemperatur erwärmt und mit Essigsäureethylester extrahiert. Die organische Phase wird mit Natriumchloridlösung gewaschen und mit Magnesiumsulfat getrocknet. Das Lösungsmittel wird im Vakuum entfernt. Das Rohprodukt wird in 5 ml Methanol gelöst und bei 0°C mit 1,5 ml 45 %iger Natronlauge versetzt. Bei Raumtemperatur werden 0,2 ml Dichlormethan hinzugegeben, dabei wird die Lösung klar. Die Lösung wird 16 Stunden bei Raumtemperatur gerührt, mit etwas Wasser versetzt und mit Ethylether extrahiert. Die wäßrige Phase wird mit 10 %ig Schwefelsäure auf pH 2-3 gestellt und zweimal mit Essigsäureethylester extrahiert, mit Magnesiumsulfat getrocknet und einrotiert.

Ausbeute: 184 mg (100 % d.Th.) als eine Mischung: 75.0% Trans / 25.0% Cis.
¹H-NMR (400 MHz, CD₃COCD₃): 10.0 (bs, 2H), 7.90 (m, 2H), 7. 60-7.00 (m, 11H), 6.42 (d, 0.3H, J=9 Hz), 6.40 (d, 0.7H, J=16 Hz), 5.80 (dd, 0.7H, J=16 Hz, J=9Hz), 5.50 (t, 0.3 H), 3.85 (s, 1.5H), 3.70 (s, 0.5H), 2.90-2.30 (m, 5H), 1.70-1.20 (m, 6H)

### Analog wurden dargestellt:

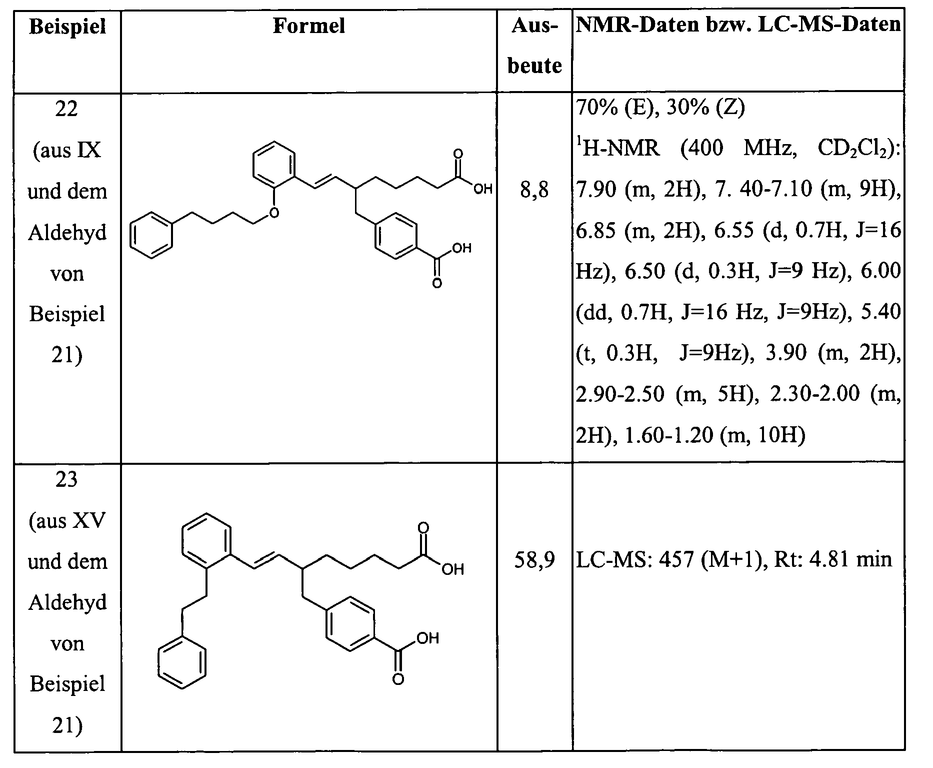

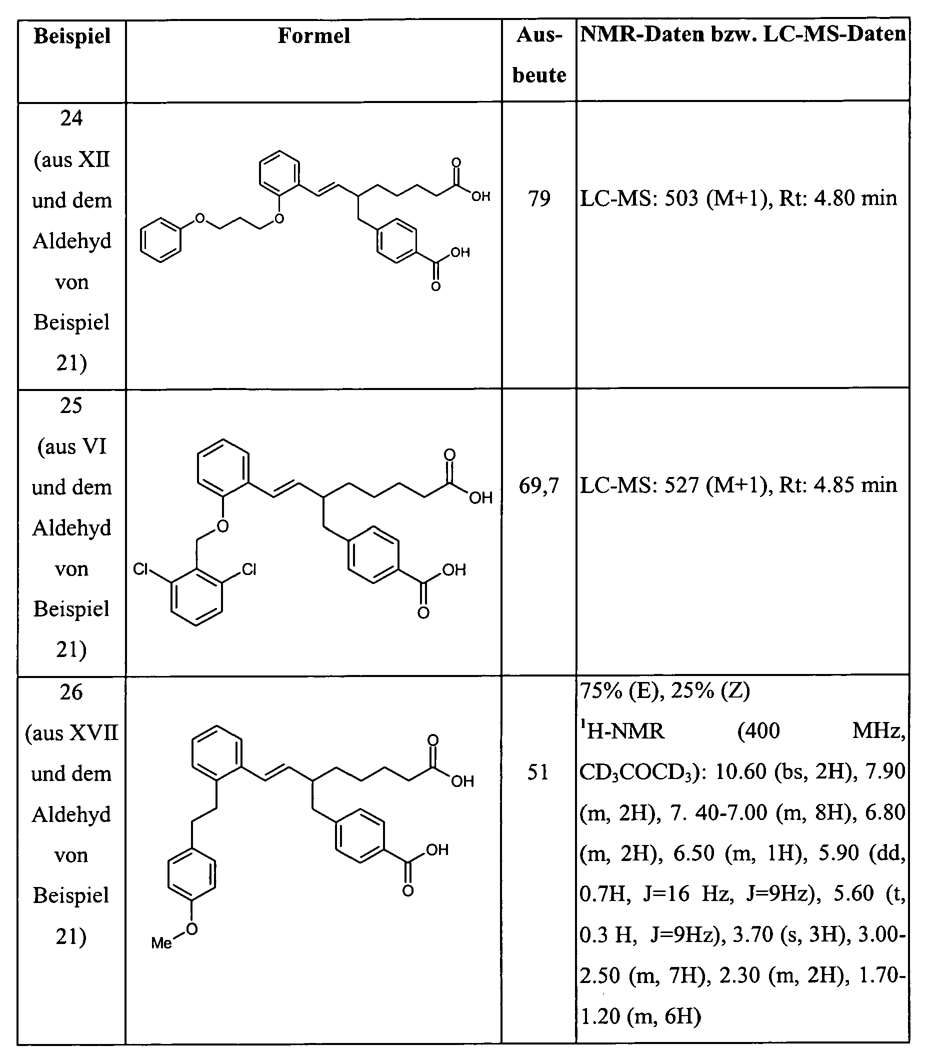

*LC/MS-Bedingungen: Säule: Symmetry C18 2,1 50 mm; Eluent: Acetontril/H₂O; Gradient: 10% Acetonitril bis 90% Acetonitril; Fluß: 0,5 ml/min; Detektor: UV 210 nm.

### Bsp.27.: 8-(2-(4-Chlorobenzyloxy)phenyl)-6-(4-ethoxycarbonylbenzyl)-7-(E)-octensäureethylester

### 27a: 6-(4-Ethoxycarbonylbenzyl)-8-(2-hydroxyphenyl)-7-(E)-octensäureethylester

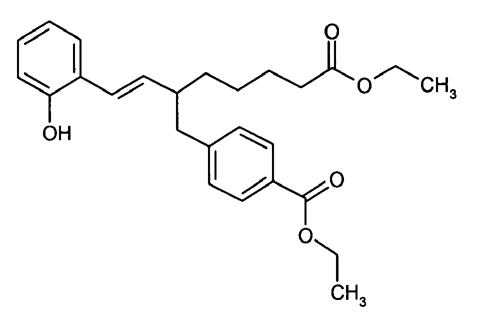

645 mg (1,44 mmol) 2-Hydroxybenzyltriphenylphosphoniumbromid (herstellbar aus 2-Hydroxybenzylalkohol analog zu Bsp. III) werden unter Argon in 25 ml Tetrahydrofuran suspendiert und auf 0°C abgekühlt. Bei dieser Temperatur werden 2,2 ml Butyllithium (1,6M-Lösung in Hexan) zugegeben. Nach 30 Minuten bei dieser Temperatur wird eine Lösung von 436 mg (1,31 mmol) des Ethylesters des Aldehyds aus Beispiel 1 in 2 ml THF zugegeben und noch 30 Minuten bei 0°C verrührt. Es werden 1 ml Wasser und 20 ml Dichlormethan zugegeben, mit HCl sauer eingestellt und über Extrelut filtriert. Das Filtrat wird eingeengt und das Produkt an 30 g Kieselgel mit Cyclohexan/Essigester 2:1 chromatographiert.

Ausbeute: 184 mg (33,2 % d. Th.)
¹H-NMR (200 MHz, CDCl₃): 7.95 (d, 2H, J=10 Hz), 7.25 (d, 2H), 7.10 (m, 2H), 6.80 (m, 2H), 6.40 (d, 1H, J=16 Hz), 5.85 (dd, 1H, J=16 Hz, J=9Hz), 5.10 (s, 1H), 4.35 (q, J=6Hz, 2H), 4.10 (m, 2H), 2.75 (m, 2H), 2.50 (m, 1H), 2.30 (m, 3H), 1.80-1.10 (m, 12H)

### 27: 8-(2-(4-Chlorobenzyloxy)phenyl)-6-(4-ethoxycarbonylbenzyl)-7-(E)-octensäureethylester

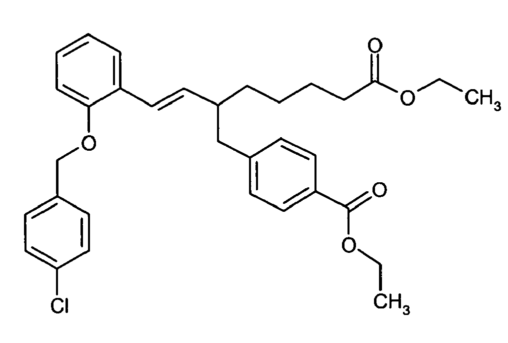

104 mg (0,24 mmol) des Phenols aus Bsp. 27a, 47 mg (0,29 mmol) 4-Chlorbenzylchlorid und 51 mg (0,37 mmol) Kaliumcarbonat werden in 10ml Acetonitril unter Rückfluss 48 Stunden erhitzt. Die Mischung wird abgekühlt, filtriert und eingeengt. Es wird zur Reinigung über Kieselgel chromatographiert.

Ausbeute: 51 mg (37,9 % d. Th.)
¹H-NMR (200 MHz, CDCl₃): 7.95 (d, 2H, J=10 Hz), 7.40-7.10 (m, 8H), 6.90 (m, 2H), 6.52 (d, 1H, J=16 Hz), 5.95 (dd, 1H, J=16 Hz, J=9Hz), 5.00 (s, 2H), 4.35 (q, J=6Hz, 2H), 4.10 (q, J=6Hz, 2H), 2.75 (m, 2H), 2.50 (m, 1H), 2.30 (t, 2H, J=6Hz), 1.80-1.10 (m, 12H)

### Analog wurden dargestellt:

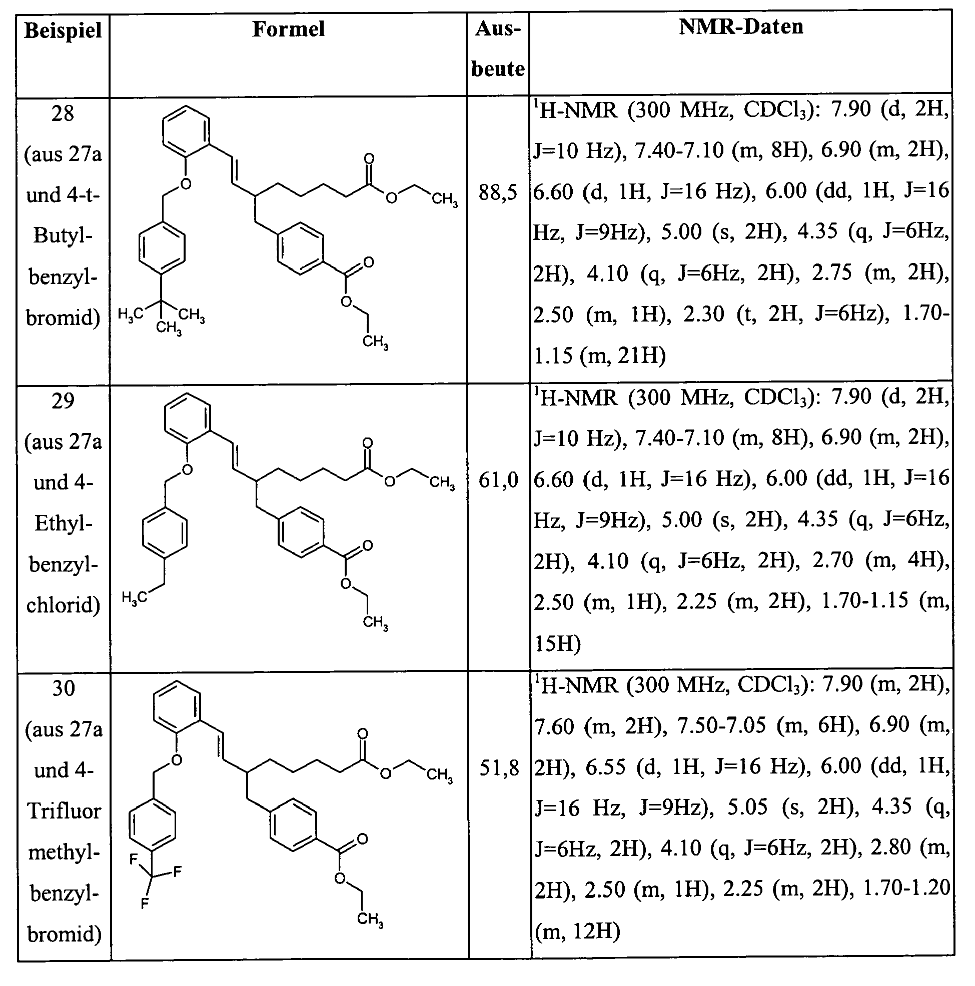

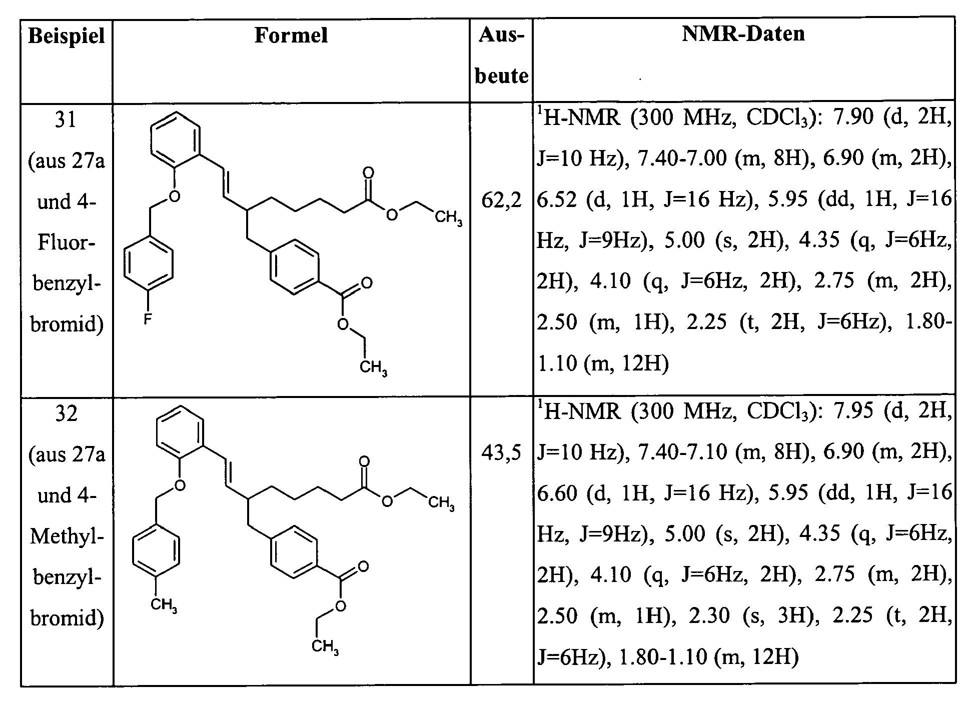

### Bsp. 33: 6-(4-Carboxybenzyl)-8-(2-(4-chlorobenzyloxy)phenyl)-7-(E)-octensäure

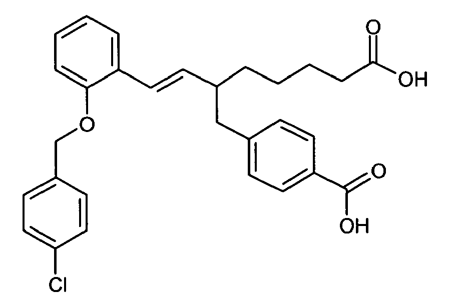

45 mg (0,08 mmol) des Diethylesters aus Bsp. 27 werden in 5ml Methanol gelöst und mit 0,5ml 45 %iger Natronlauge versetzt. Man läßt das Reaktionsgemisch auf Raumtemperatur kommen und gibt 0,3 ml Dichlormethan hinzu. Nach 20 Stunden Rühren bei Raumtemperatur wird die Reaktionslösung einmal mit Ether gewaschen, mit 10 %iger Schwefelsäure angesäuert, zweimal mit Essigester extrahiert, die vereinigten organischen Phasen über Extrelut filtriert und eingeengt.

Ausbeute: 11 mg (23% d. Th.)
LC-MS: 493 (M+1); Rₜ: 4.86 min

### Analog wurden dargestellt:

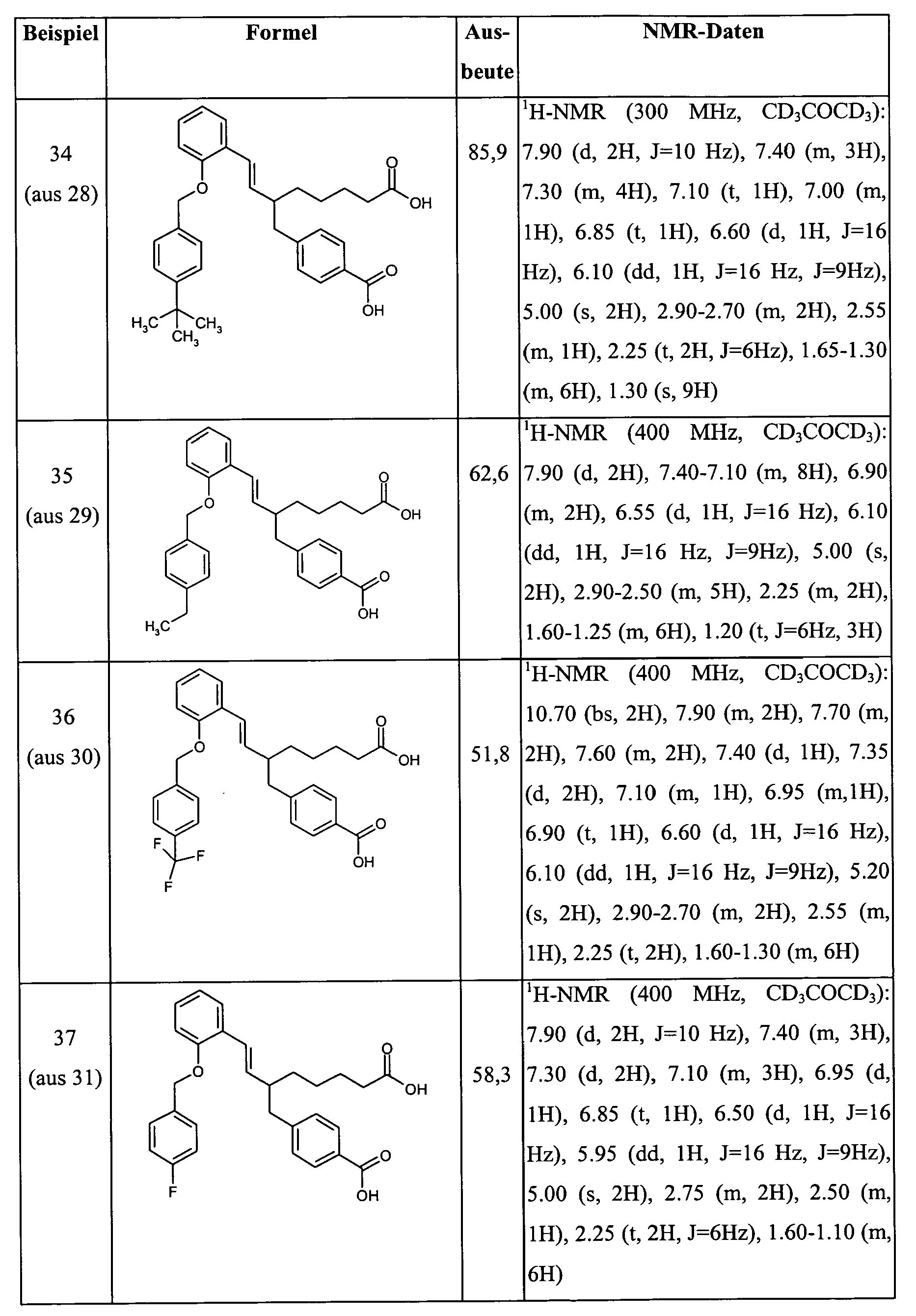

### Bsp. 38: 8-(2-Benzyloxy)phenyl-6-(4-carbonbenzyl)-octansäure

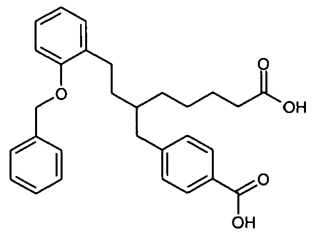

62 mg (0,14 mmol) 8-(2-Benzyloxy)phenyl-6-(4-carboxybenzyl)-7-(E)-octensäure aus Bsp. 11 und 30 mg Palladium/A-Kohle 10 %ig werden in 5 ml Essigsäureethylester gegeben und bei Raumtemperatur unter Normaldruck mit Wasserstoff hydriert. Nach zwei Stunden wird die Mischung über Celite filtriert und das Filtrat eingeengt.

Ausbeute: 59 mg (94,7% d. Th.)
¹H-NMR (400 MHz, CD₂Cl₂): 9.50 (bs, 2H), 7.85 (m, 2H), 7.35-7.00 (m, 9H), 6.80 (m, 2H), 4.95 (s, 2H), 2.70-2.50 (m, 4H), 2.15 (t, 2H, J=6Hz), 1.80-1.20 (m, 9H)

### Analog wurden die folgende Substanzen synthetisiert:

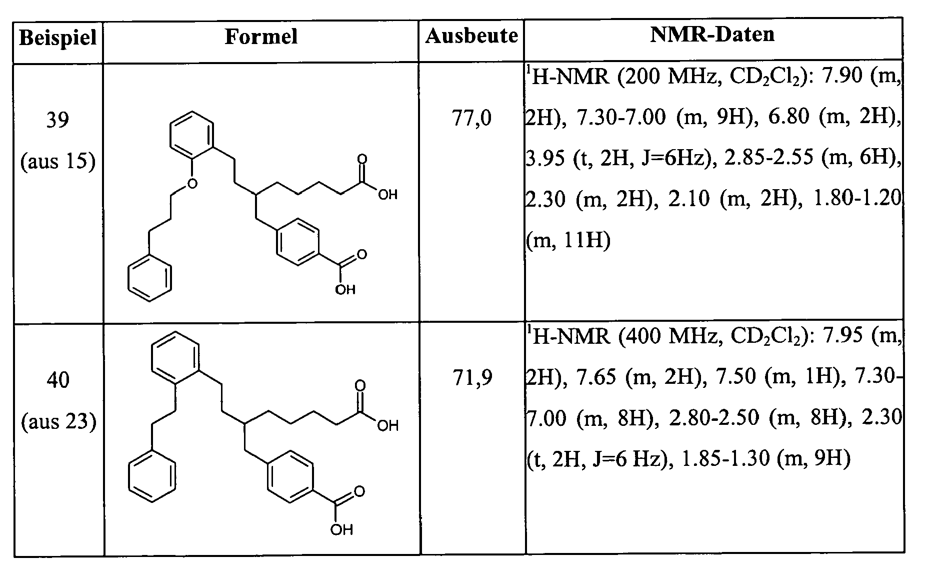

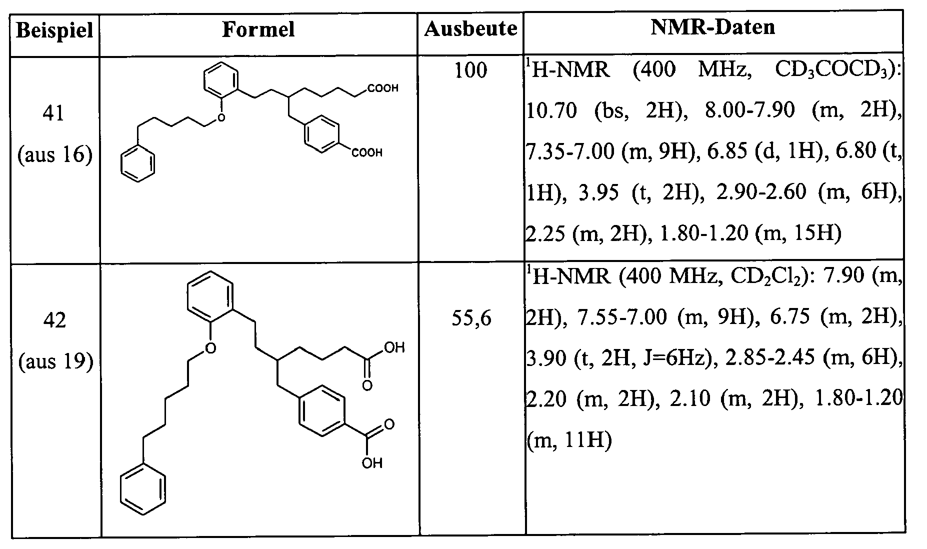

### Bsp. 43: 6-(4-Carboxybenzyl)-9-(3-(4-phenylbutoxy)phenyl)-7-(Z)-nonensäure

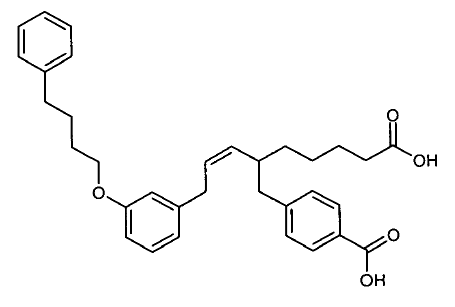

238,2 mg (0,35 mmol) des Phosphoniumsalzes aus Bsp. XVIII werden bei -30°C unter Argon in 5 ml THF suspendiert und mit 0,26 ml Butyllithium (0,76 mmol, 1,6M Lösung in Hexan) versetzt. Die tieforange Lösung wird 30 min. bei -30°C gerührt, und eine Lösung von 106 mg (0,35 mmol) 6-Formyl-7-(4-methoxycarbonylphenyl)heptansäuremethylester (hergestellt analog Beispiel I aus 2-Oxocyclohexancarbonsäure-t-butylester und 4-Chlormethylbenzoesäuremethylester, vgl. EP-A-0 341 551) in 1 ml THF wird bei dieser Temperatur tropfenweise hinzugegeben. Die Mischung wird 30 min. bei 0°C und 30 min. bei Raumtemperatur gerührt. Es wird bei 0°C mit Wasser versetzt, auf Raumtemperatur erwärmt und mit Essigsäureethylester extrahiert. Die organische Phase wird mit Natriumchloridlösung gewaschen, mit Magnesiumsulfat getrocknet und einrotiert. Das Rohmaterial wird in 6 ml Methanol gelöst und bei 0°C werden 1,5 ml 45%ige Natronlauge zugegeben. Bei Raumtemperatur werden 0,2 ml Dichlormethan hinzugegeben, dabei wird die Lösung klar. Die Lösung wird 16 Stunden bei Raumtemperatur gerührt, mit etwas Wasser versetzt und mit Ethylether extrahiert. Die wäßrige Phase wird mit 10 %iger Schwefelsäure auf pH 2-3 gestellt und zweimal mit Essigsäureethylester extrahiert, mit Magnesiumsulfat getrocknet und im Vakuum eingedampft.

Ausbeute: 23 mg (8,2% d.Th.).
¹H-NMR (200 MHz, CD₂Cl₂): 10.50 (bs, 2H), 7.90 (d, 2H), 7.30-7.00 (m, 7H), 7.00 (t, 1H), 6.60 (m, 1H), 6.50 (m, 2H), 5.35 (m, 1H), 5.10 (t, 1H), 3.90 (m, 2H), 3.10 (m, 2H), 2.90-2.50 (m, 5H), 2.30 (m, 4H), 1.20-1.70 (m, 8H).

### Bsp. 44: 6-(4-Carboxybenzyl)-9-(3-(4-phenylbutoxy)phenyl)nonansäure

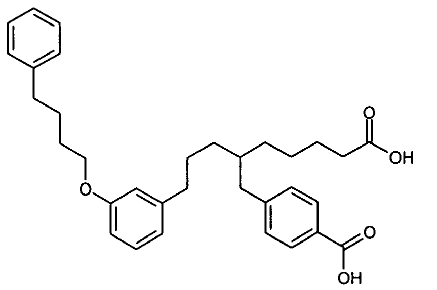

50 mg (0,10 mmol) 6-(4-Carboxybenzyl)-9-(3-(4-phenylbutoxy)phenyl-7-(Z)-nonensäure aus Bsp. 43 und 19,5 mg Palladium/A-Kohle 10%ig werden in 5 ml Essigsäureethylester gegeben und bei Raumtemperatur unter Normaldruck mit Wasserstoff hydriert. Nach zwei Stunden wird die Mischung über Celite filtriert und das Filtrat eingeengt.

Ausbeute: 51 mg (100% d. Th.)
¹H-NMR (200 MHz, CDCl₃): 10.50 (bs, 2H), 7.90 (d, 2H), 7.30-7.00 (m, 8H), 6.60 (m, 3H), 3.90 (m, 2H), 2.70-2.40 (m, 6H), 2.30 (t, 2H), 1.70-1.20 (m, 15H).

### Bsp. 45: 2-Cyano-[(E)-2-[2-[(5-phenylpentyl)oxy]phenyl]ethenyl]-benzolheptansäuremethylester

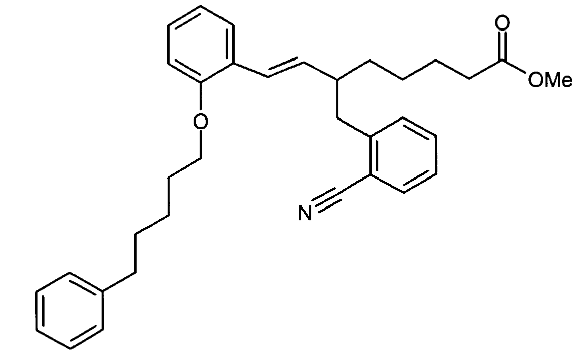

Zu einer Suspension von 117,6 mg (0,20 mmol) Triphenyl[[2-[(5-phenylpentyl)oxy]phenyl]methyl]-phosphoniumbromid aus Bsp. X in 1 ml absolutem THF wurde unter Argon bei -78°C 0,12 ml (0,19 mmol) n-Butyllithium (1,6 M in Hexan) zugetropft. Man rührte 5 min bei -78°C, entfernte das Kühlbad und ließ die Mischung auf Raumtemperatur auftauen. Die Mischung wurde auf 0°C gekühlt und eine Lösung von 45,0 mg (0,16 mmol), 7-(2-Cyanophenyl)-6-formyl-heptansäuremethylester aus Bsp. XXIII in 1 ml absolutem THF zugetropft. Man ließ die Mischung über Nacht auftauen, versetzte mit Wasser und extrahierte zweimal mit Ethylacetat. Die vereinten organischen Phasen wurden über Natriumsulfat getrocknet und das Lösungsmittel entfernt. Das Produkt wurde durch Dünnschichtchromatographie gereinigt.

Ausbeute: 64,8 mg (64,8 %) eines leicht gelblichen Öls.
E/Z = 64: 3 6
¹H-NMR (300 MHz, CDCl₃):
δ = 1.19 - 1.84 (m, 12H), 2.17 - 2.33 (m, 2H), 2.48 - 2.60 (m, 1H), 2.64 (t, J = 7.4 Hz, 2H), 2.72 - 3.08 (m, 2H), 3.63 (s, 3H), 3.75 - 3.94 (m, 2H), 5.42 (dd, J = 10.6 Hz, J = 9.8 Hz, 1H, Z-Isomer), 5.98 (dd, J = 15.9 Hz, J = 9.1Hz, 1H, E-Isomer), 6.38 - 7.62 (m, 14H).

### Analog wurden hergestellt:

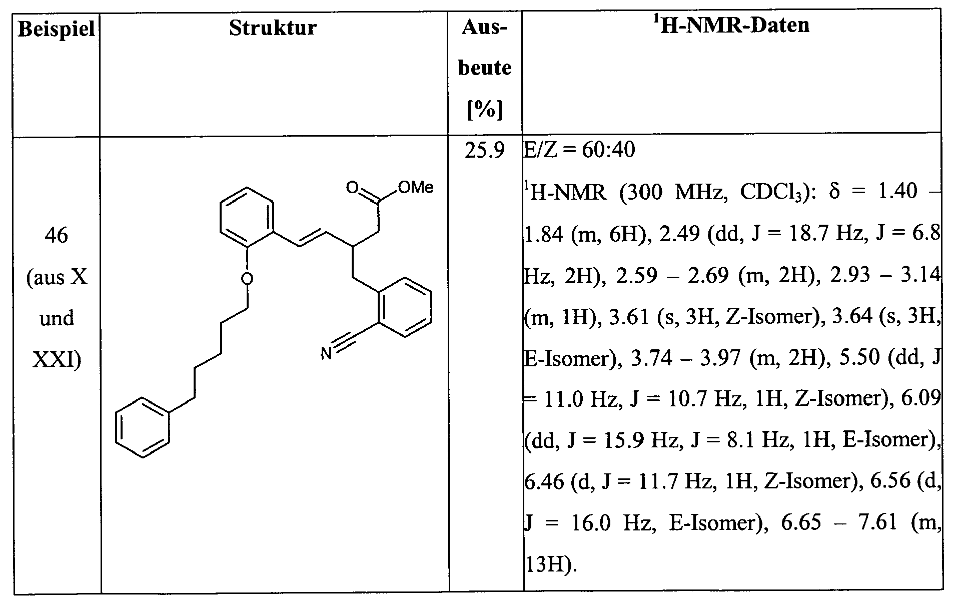

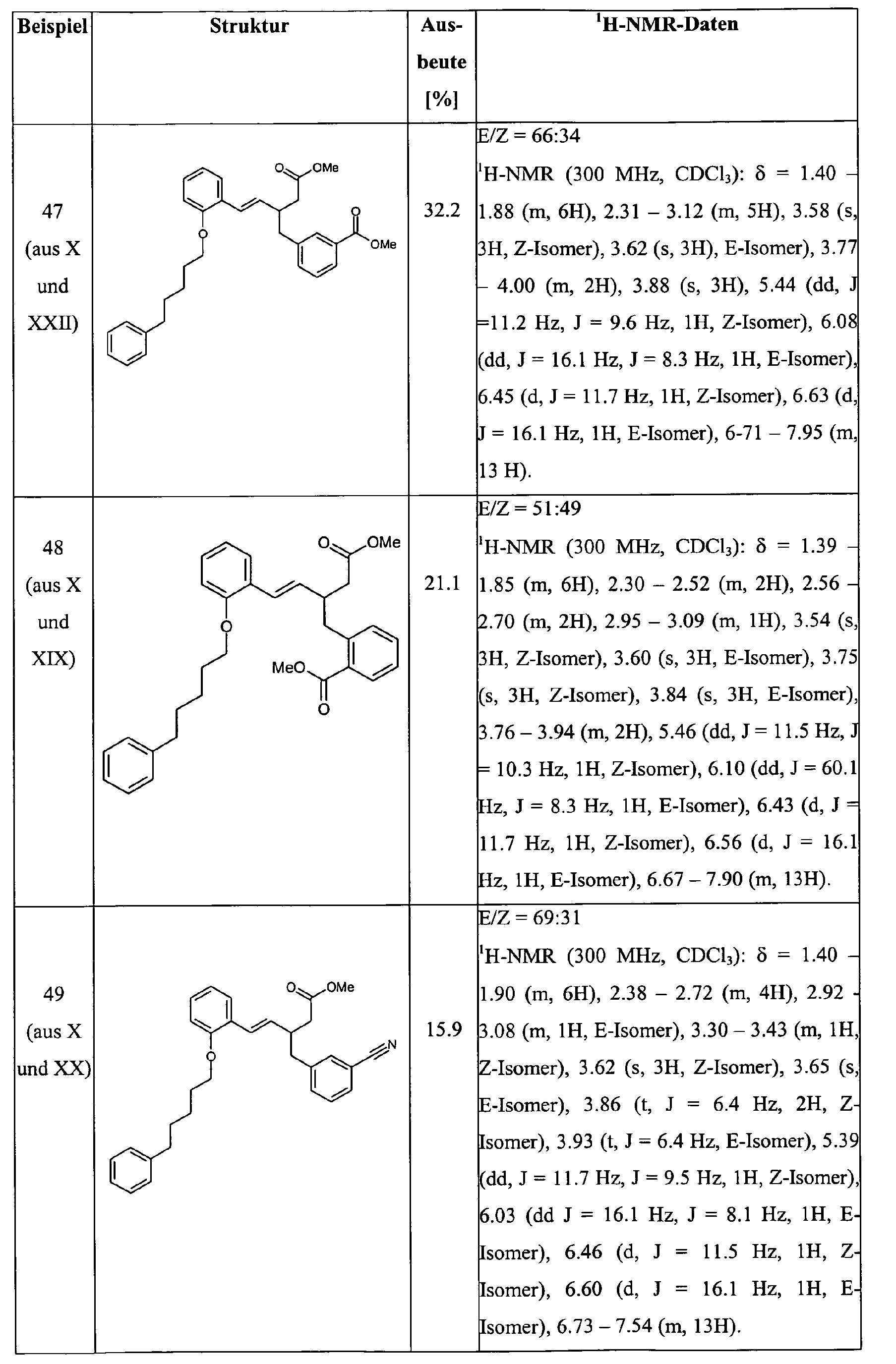

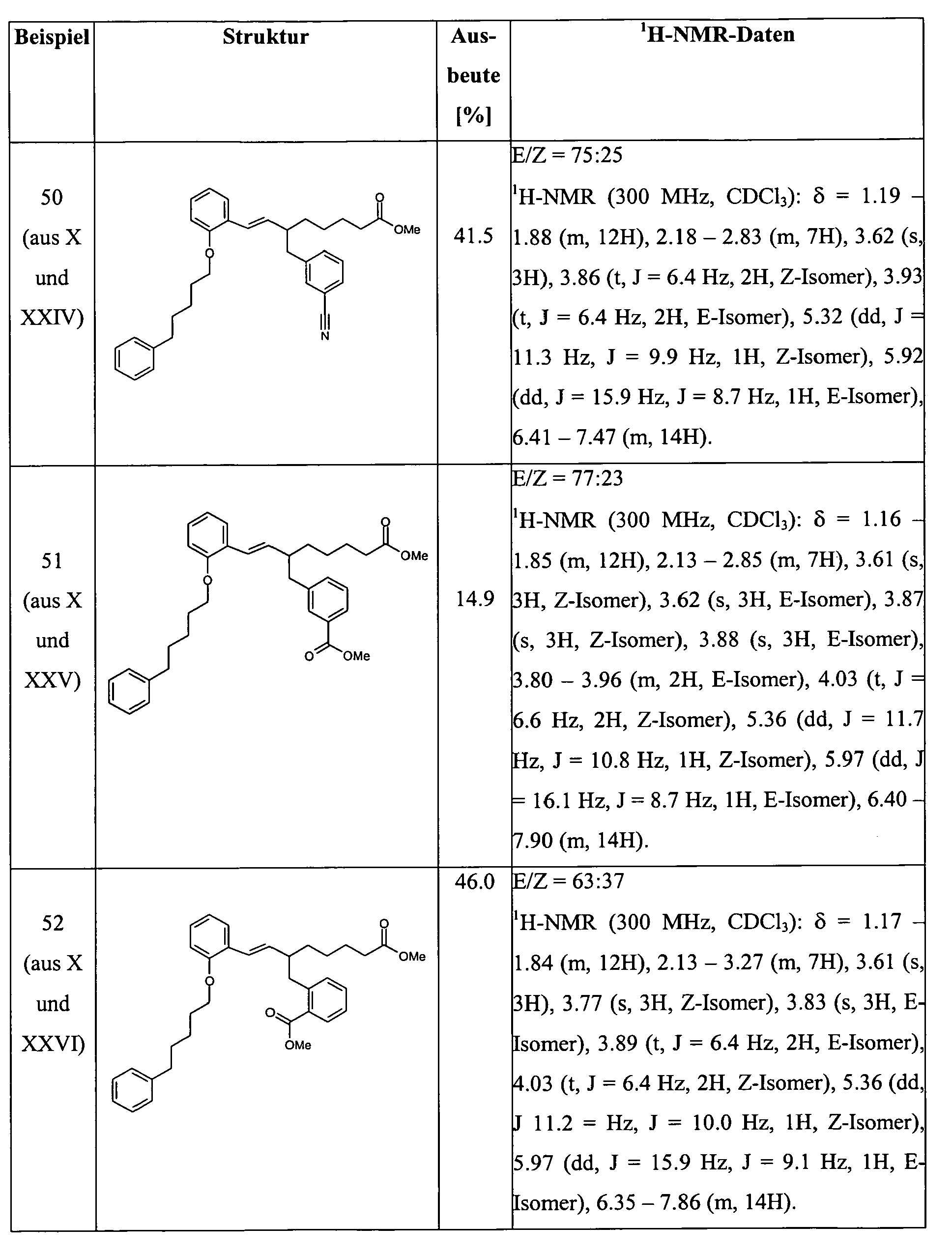

### Bsp. 53.: 3-Carboxy-[(E)-2-[2-[(5-phenylpentyl)oxy]phenyl]ethenyl]-benzolheptansäure

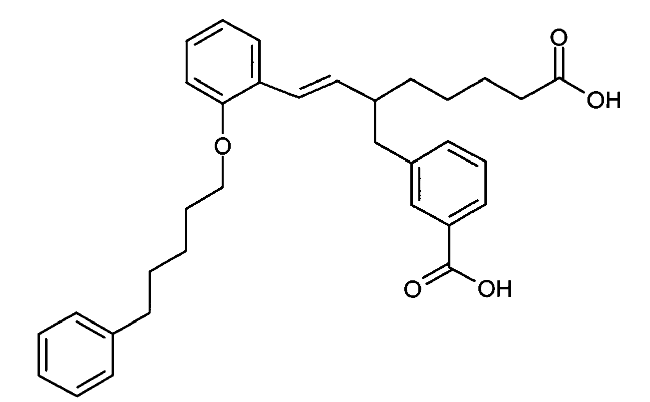

Zu einer Lösung von 9,00 mg (16,6 µmol) 3-(Methoxycarbonyl)-e-[(E)-2-[2-[(5-phenylpentyl)oxy]phenyl]ethenyl]-benzolheptansäuremethylester aus Bsp. 51 in 780 µl THF, 260µl Methanol und 260 µl Wasser wurden 14,18 mg (331,7 µmol Lithiumhydroxid zugegeben. Die Mischung wurde über Nacht gerührt, mit 3 M NaOH versetzt und mit Diethylether extrahiert. Die wässrige Phase wurde mit 6 M HCl angesäuert, zweimal mit Diethylether sowie zweimal mit Ethylacetat extrahiert. Die vereinten organischen Phasen wurden über Natriumsulfat getrocknet und das Lösungsmittel entfernt.

Ausbeute 7,8 mg (91,4%) eines farblosen Feststoffes.
LC/MS: 5.33 min [m/z = 514.4 (M+H), 532.5 (M+NH₄)], 5.40 min [m/z = 514.4 (M+H), 532.5 (M+NH₄)].
E/Z = 77:23
¹H-NMR (300 MHz, CDCl₃):
δ = 1.04 - 2.91 (m, 19 H), 3.88 (t, J = 6.2 Hz, 2H, Z-Isomer), 3.94 (t, J = 6.4 Hz, 2H, E-Isomer), 5.39 (dd, J = 11.2 Hz, J = 10.4 Hz, 1H, Z-Isomer), 6.02 (dd, J = 15.1 Hz, J = 8.7 Hz, 1H, E-Isomer), 6.48 (d, J = 11.7 Hz, 1H, Z-Isomer), 6.59 (d, J = 16.1 Hz, 1H, E-Isomer), 6.73 - 7.99 (m, 13H).

### Analog wurden hergestellt:

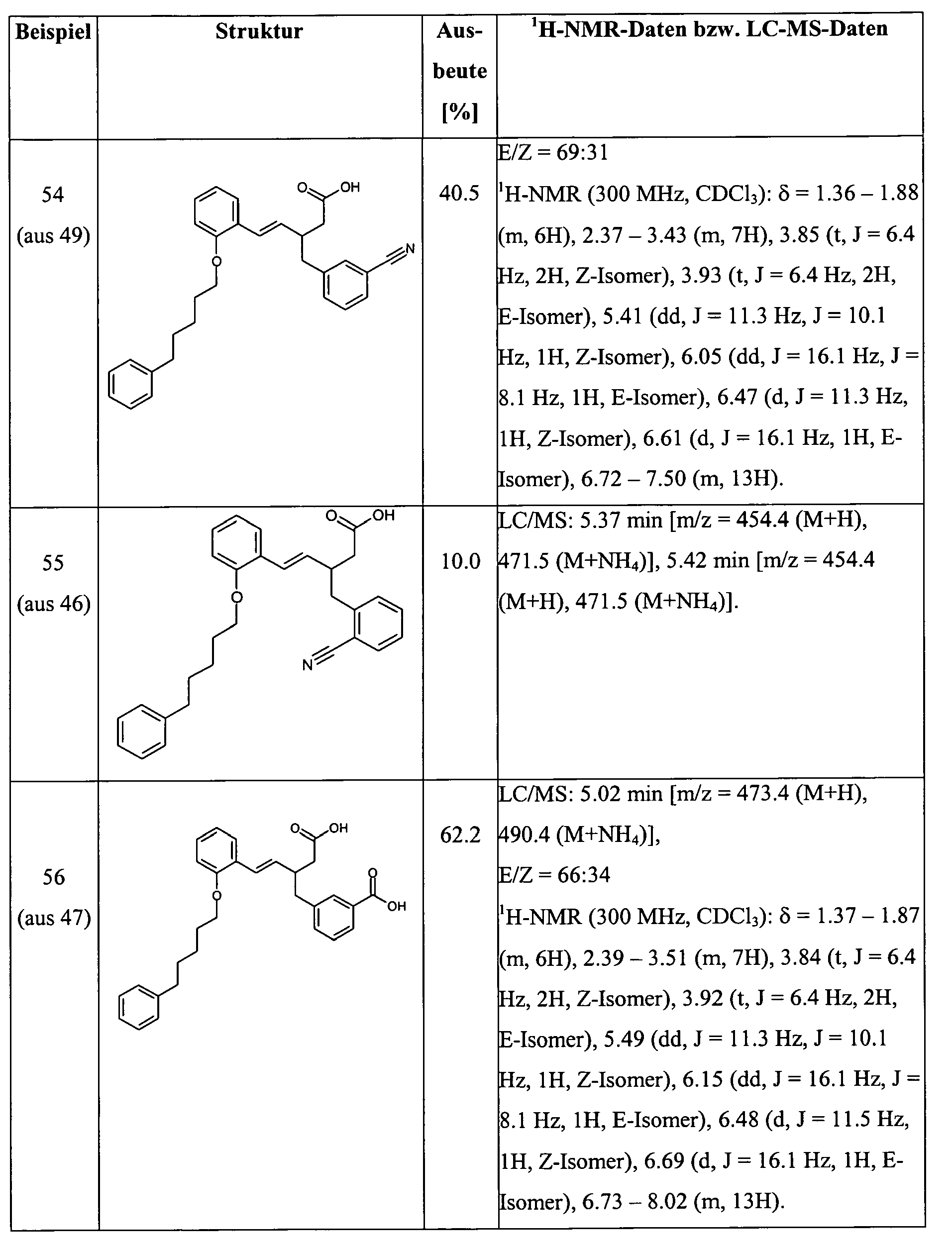

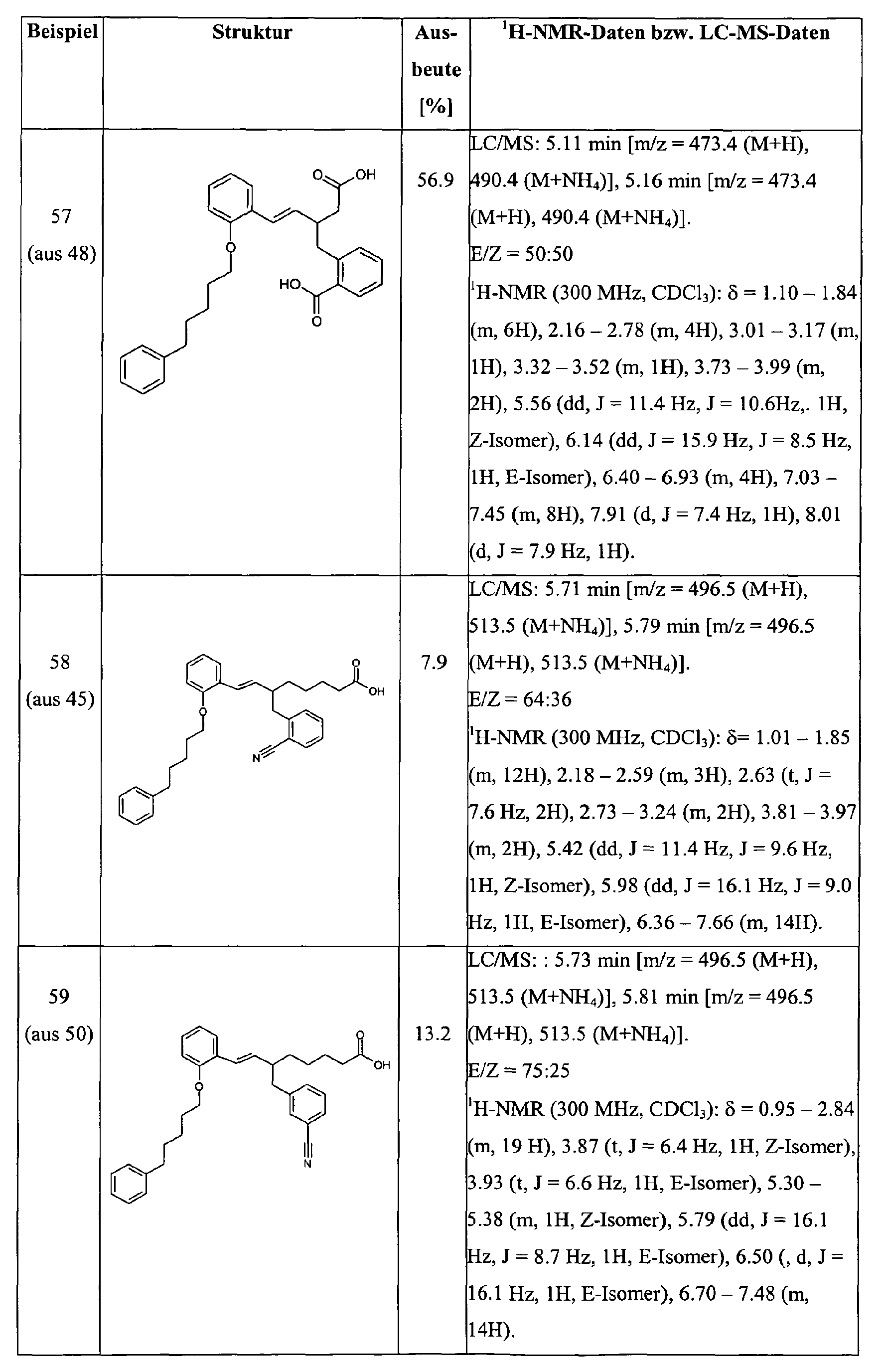

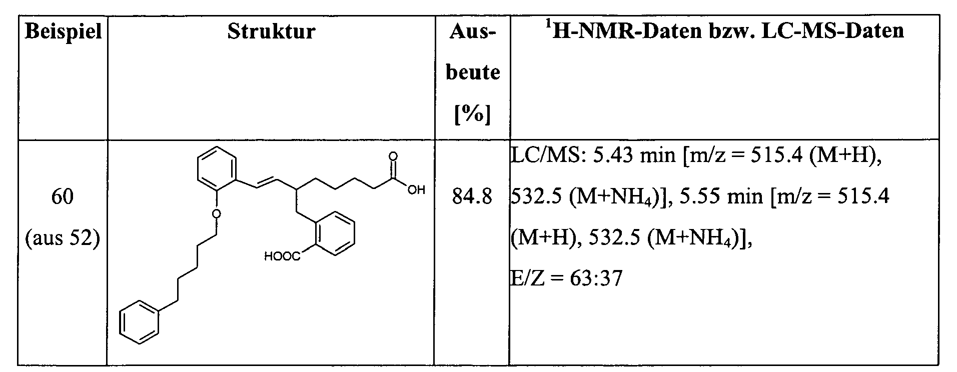

LC/MS-Bedingungen: Säule: Symmetry C18 2,1*50 mm; Eluent: Acetonitril/H₂O (0,1% Ameisensäure); Gradient: 10 % Acetonitril bis 90 % Acetonitril; Fluss: 0,5 ml/min; Detektor: UV 208-400 nm

### Bsp. 61: 6-[4-(Methoxycarbonyl)phenoxy]-8-[2-(4-phenylbutoxy)phenyl]octansäuremethylester

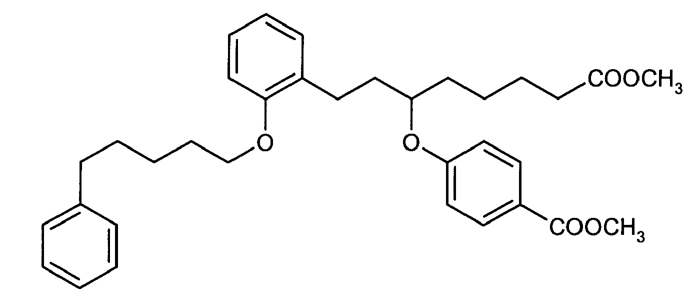

Zu einer Lösung von 55,32 mg (0,36 mmol) 4-Hydroxybenzeosäuremethylester und 95,36 mg (0,36 mmol) Triphenylphosphin in 2.5 ml THF tropfte man innerhalb von 2 h eine Lösung von 100,0 mg (0,24 mmol) 6-Hydroxy-8-{2-[(5-phenylpentyl)oxy]phenyl}octansäuremethylester aus Bsp. XXXIa und 63,32 mg (0,36 mmol) DEAD in 2,5 ml THF zu. Man rührte 18 h bei RT, versetzte mit 40 ml Diethylether, filtrierte und entfernte das Lösungsmittel. Das Produkt wurde chromatografisch gereinigt (Kieselgel, Cyclohexan/EE 10:1). Man erhielt 63,0 mg (47,5 %) eines farblosen Öls.
¹H-NMR (400 MHz, CDCl₃): δ = 0.76 - 1.78 (m, 11H), 1.84 - 2.04 (m, 2H), 2.28 (t, *J* = 7.3 Hz, 2H), 2.61 (t, *J* = 7.6 Hz, 2H), 2.64 - 2.77 (m, 2H), 3.63 (s, 3H), 3.85 (s, 3H), 3.86 - 3.94 (m, 2H), 4.22 - 4.34 (m, 2H), 6.74 - 6.86 (m, 4H), 7.01 - 7.31 (m, 7H), 7.92 (d, *J* = 8.8 Hz, 2H).

### Analog wurden hergestellt:

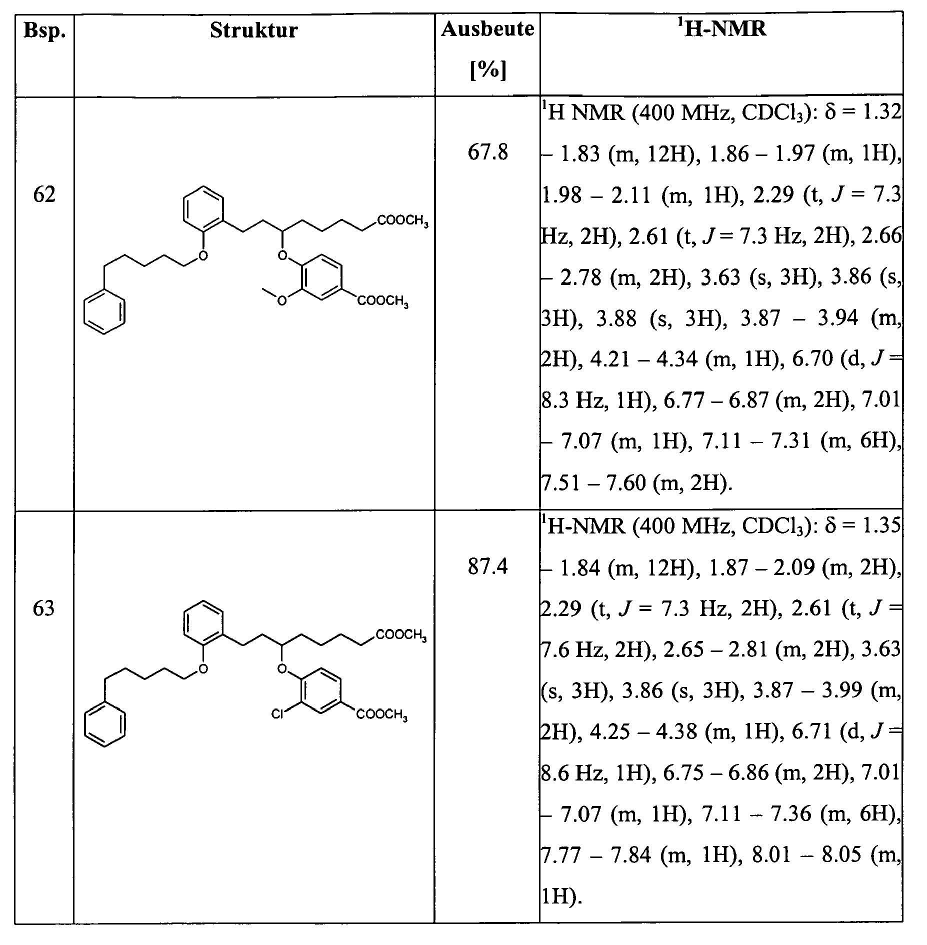

### Bsp. 64: 6-[2,6-Dichloro-4-(methoxycarbonyl)phenoxy]-8-{2-[(5-phenylpentyl)oxylphenyl}-octansäuremethylester

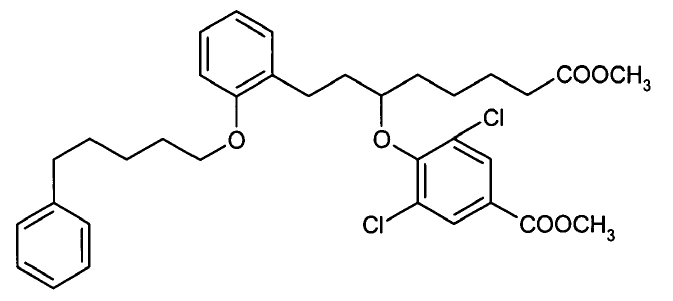

Eine Suspension von 100,00 mg (0,21 mmol) 6-Bromo-8-{2-[(5-phenylpentyl)oxy]phenylloctansäuremethylester aus Bsp. XXXII, 69,73 mg (0,32 mmol) 3,5-Dichlor-4-hydroxy-benzoesäuremethylester und 58,13 mg (0,42 mmol) Kaliumcarbonat in 5 ml DMF wurde über Nacht bei 75°C gerührt. Nachdem die Mischung auf Raumtemperatur abgekühlt war, versetzte man mit 1 N NaOH, extrahierte mit Diethylether, trocknete die vereinten organischen Phasen über Na₂SO₄ und entfernte das Lösungsmittel. Das Produkt wurde chromatografisch gereinigt (Kieselgel, Cyclohexan/EE 10:1). Man erhielt 90,9 mg (70,2 %) eines farblosen Öls.
¹H-NMR (300 MHz, CDCl₃): δ = 1.35 - 1.89 (m, 12H), 1.90 - 2.14 (m, 2H), 2.16 - 2.37 (m, 2H), 2.52 - 2.75 (m, 4H), 3.64 (s, 3H), 3.89 (m, 3H), 3.89 - 4.03 (m, 2H), 4.58 - 4.98 (m, 1H), 6.70 - 6.92 (m, 2H), 7.02 - 7.34 (m, 7H), 7.94 (s, 2H).

### Analog wurden hergestellt:

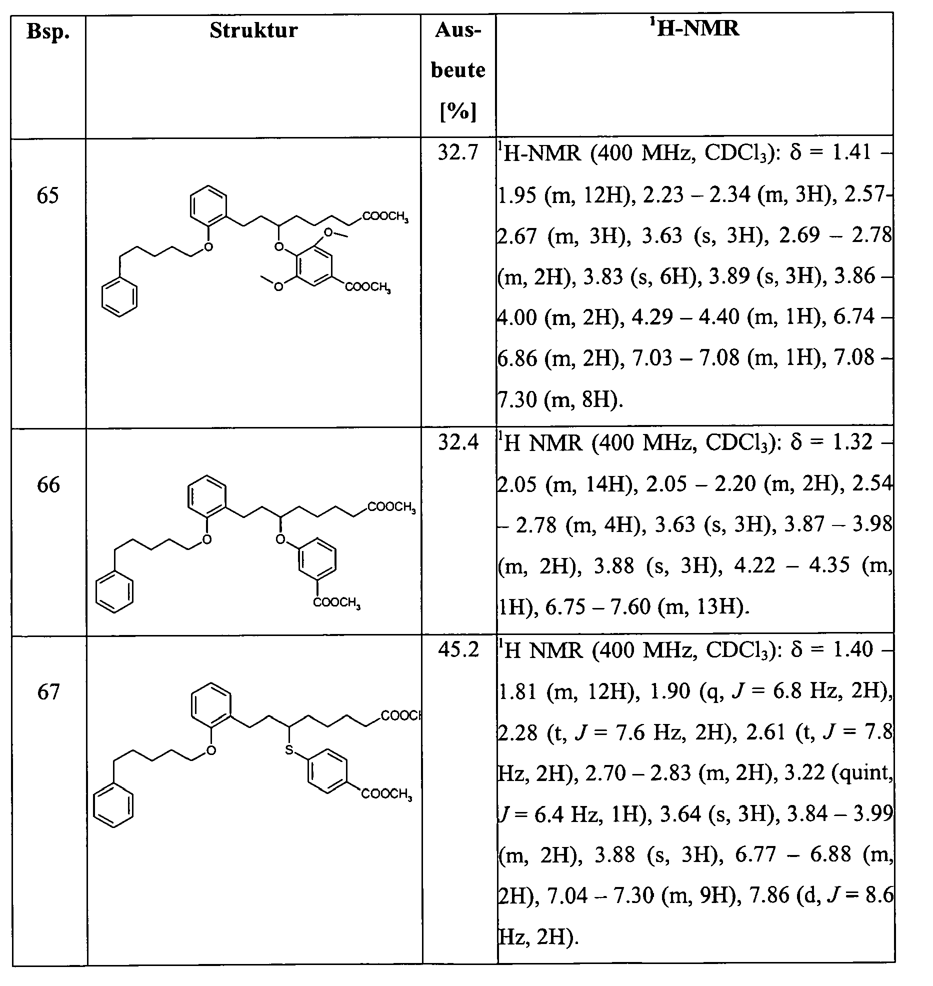

### Analog zu Bsp. 53 wurden hergestellt:

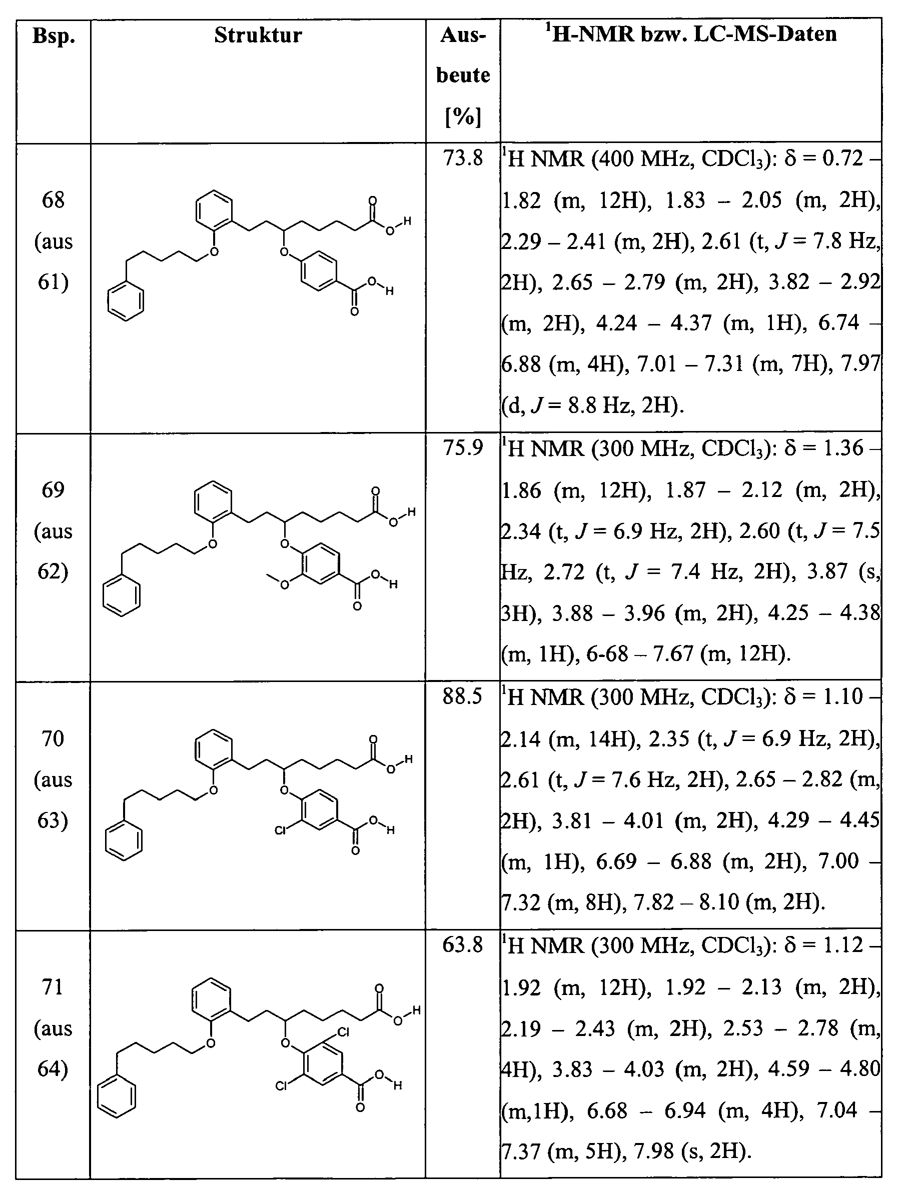

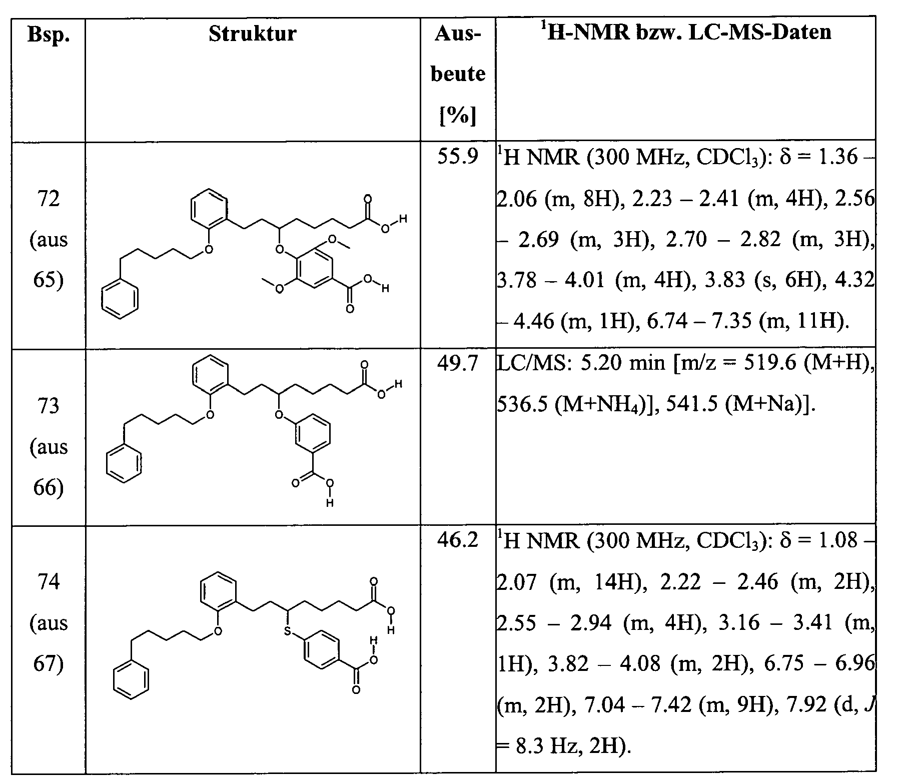

LC/MS-Bedingungen: Säule: Symmetry C18 2,1*50 mm; Eluent: Acetonitril/H₂O (0,1% Ameisensäure); Gradient: 10 % Acetonitril bis 90 % Acetonitril; Fluss: 0,5 ml/min; Detektor: UV 208-400 nm

### Bsp.75: 8-(2-(4-Chlorobenzyloxy)phenyl)-6-(4-ethoxycarbonylbenzyl)-7-(E)-octensäureethylester

### 75a: 6-(4-Ethoxycarbonylbenzyl-8-(2-hydroxyphenyl)octansäureethylester

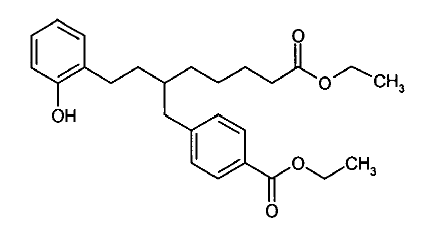

510,2 mg (1,44 mmol) 6-(4-Ethoxycarbonylbenzyl-8-(2-hydroxyphenyl)-7(E)-octensäureethylester aus Bsp. 27a und 250 mg Palladium/A-Kohle 10%ig werden in 20 ml Essigsäureethylester gegeben und bei Raumtemperatur unter Normaldruck mit Wasserstoff hydriert. Nach fünf Stunden wird die Mischung über Celite filtriert und in Vakuum eingedampft.

Ausbeute 507,9 mg (99,1% d. Th.)
¹H-NMR (400 MHz, CDCl₃): 7,95 (d, 2H, J=10 Hz), 7,20 (d, 2H), 7,00 (m, 2H), 6,80 (m, 2H), 4,90 (s, 1H), 4,35 (q, J=6Hz, 2H), 4,10 (m, 2H), 2,65 (m, 4H), 2,30 (m, 2H), 1,80-1,10 (m, 11H)

### 75: 4-[7-Ethoxy-2-(2-{2-[(4-ethylbenzyl)oxy]phenyl}ethyl)-7-oxoheptyl]benzoesäureethylester

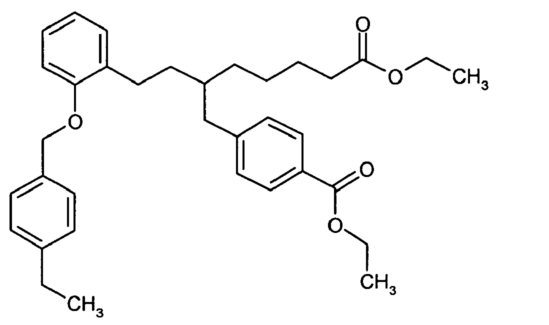

50 mg (0,117 mmol) des Phenols aus Bsp. 75a, 20 mg (0,129 mmol) 4-Ethylbenzylchlorid, 32 mg (0,234 mmol) Kaliumcarbonat und eine katalytische Menge Kaliumjodids werden in 5ml 2-Butanon unter Rückfluß 48 Stunden erhitzt. Die Mischung wird abgekühlt, filtriert und eingeengt. Es wird zur Reinigung über Kieselgel chromatographiert.

Ausbeute: 34 mg (52,8 % d. Th.)
¹H-NMR (200 MHz, CDCl₃): 7.95 (d, 2H, J=10 Hz), 7.40-7.10 (m, 8H), 6.90 (m, 2H), 5.00 (s, 2H), 4.35 (q, J=6Hz, 2H), 4.10 (q, J=6Hz, 2H), 2.75 (m, 6H), 2.30 (m, 2H), 1.80-0.80 (m, 18H)

### Analog wurden dargestellt:

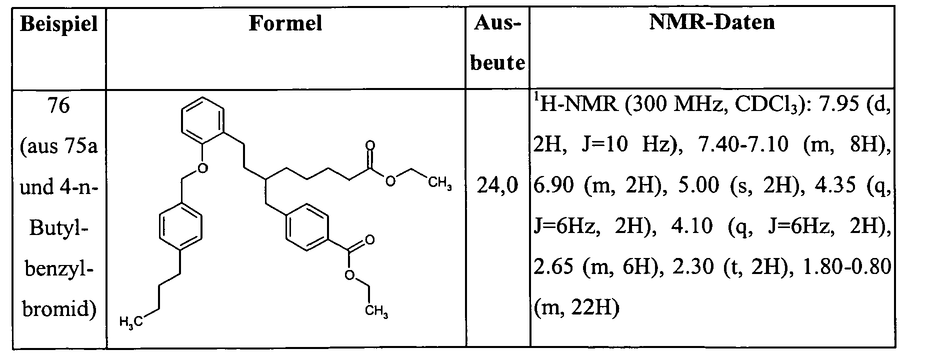

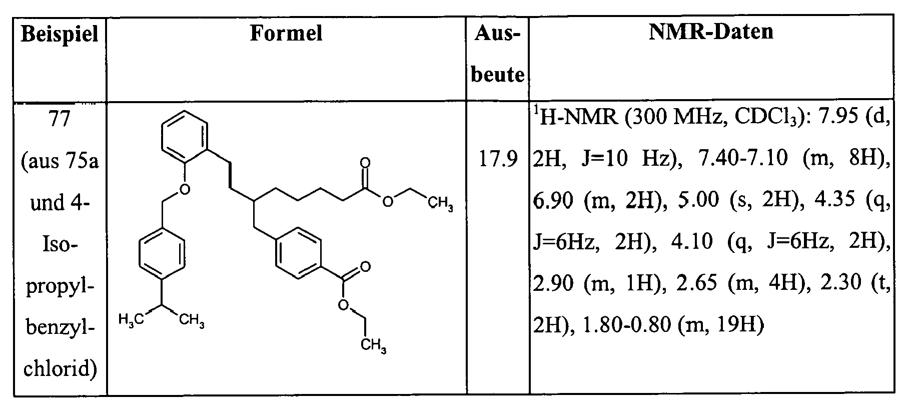

### Bsp. 78: 4-[6-Carboxy-2-(2-{2-[(4-ethylbenzyl)oxy]phenyl}ethyl)hexyl]benzoesäure

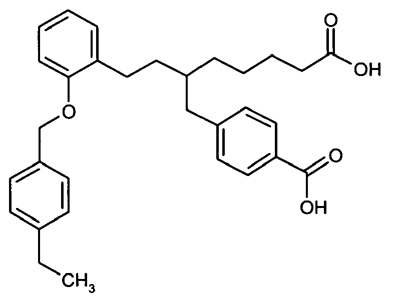

45 mg (0,08 mmol) des Diethylesters aus Bsp. 75 werden in 5ml Methanol gelöst und mit 0,5 ml 45 %iger Natronlauge versetzt. Man läßt das Reaktionsgemisch auf Raumtemperatur kommen und gibt 0,3 ml Dichlormethan hinzu. Nach 20 Stunden Rühren bei Raumtemperatur wird die Reaktionslösung einmal mit Ether gewaschen, mit 10 %iger Schwefelsäure angesäuert, zweimal mit Essigester extrahiert, die vereinigten organischen Phasen über Extrelut filtriert und eingeengt.

Ausbeute: 6,4 mg (21 % d. Th.)
LC-MS: 488 (M); Rₜ: 4.99 min

### Analog wurden dargestellt:

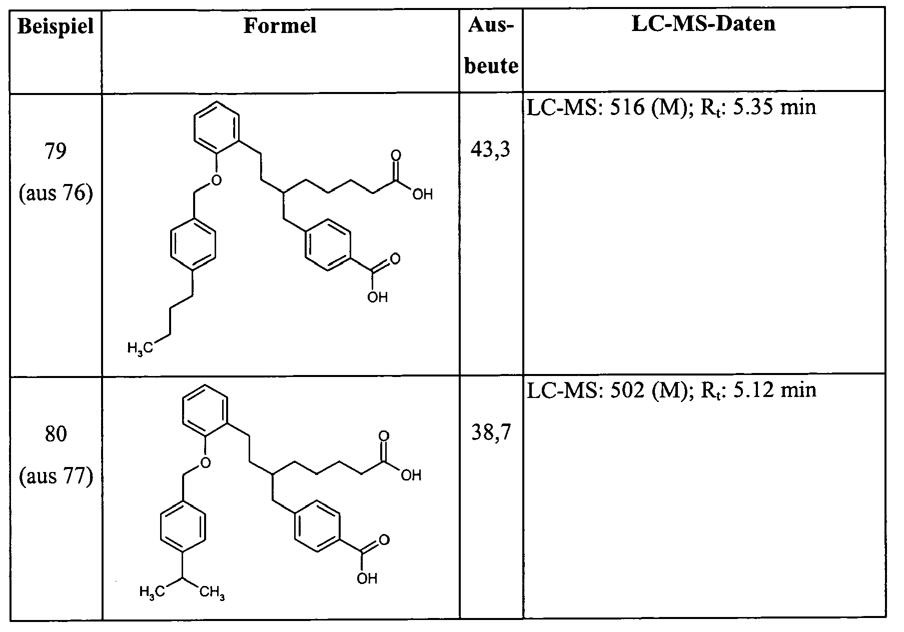

### 1) LC/MS-Bedingungen: Säule: Symmetry C18 2,1*50 mm; Eluent: Acetonitril/H₂O (0,1% Ameisensäure); Gradient: 10 % Acetonitril bis 90 % Acetonitril; Fluss: 0,5 ml/min; Detektor: UV 210 nm

### 81: 8-(2-(4-t-Butylbenzyloxy)phenyl)-6-(4-methoxycarbonylphenyloxy)-octansäuremethylester

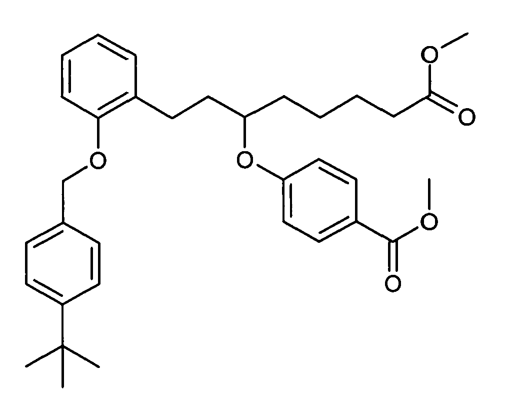

Diese Verbindung wurde analog zu Bsp. 61 erhalten, wobei die zu Bsp. XXXIa analoge Ausgangsverbindung 6-Hydroxy-8-{2-[4-t-butylbenzyloxy]phenyl}octansäuremethylester entsprechend Bsp. XXXIa ausgehend von 2-(4-t-Butylbenzyloxy)benzylalkohol hergestellt wurde.

Ausbeute: 69,5 %
¹H NMR (200 MHz, CDCl₃): δ = 1.19 - 1.78 (m, 6H), 1.33 (s, 9H), 1.81 - 2.09 (m, 2H), 2.26 (t, *J* = 7.6 Hz, 2H), 2.59 - 2.91 (m, 2H), 3.63 (s, 3H), 3.87 (s, 3H), 4.30 (quint, *J* = 5.7 Hz, 1H), 5.01 (s, 2H), 6.77 (d, *J* = 8.9 Hz, 2H), 6.81 - 6.95 (m, 2H), 7.03 - 7.43 (m, 6H), 7.89 (d, *J* = 8.8 Hz, 2H).

### 82: 8-(2-(4-t-Butylbenzyloxy)phenyl)-6-(4-carboxyphenyloxy)-octansäure

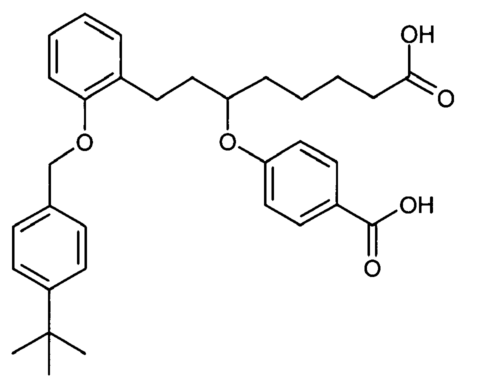

Diese Verbindung wurde analog zu Bsp. 11 aus Bsp. 81 erhalten:

Ausbeute: 83.1 %
¹H NMR (200 MHz, DMSO-d₆): δ = 1.15 - 1.55 (m, 4H), 1.27 (s, 9H), 1.56 - 1.73 (m, 2H), 1.79 - 1.96 (m, 2H), 2.16 (t, *J* = 6.8 Hz, 2H), 2.55 - 2.86 (m, 2H), 4.33 - 4.49 (m, 1H), 5.03 (s, 2H), 6.79 - 7.41 (m, 10H), 7.81 (d, *J* = 8.8 Hz, 2H), 12.26 (bs, 2H).

## Patentansprüche

1. Verbindungen der allgemeinen Formel (I) 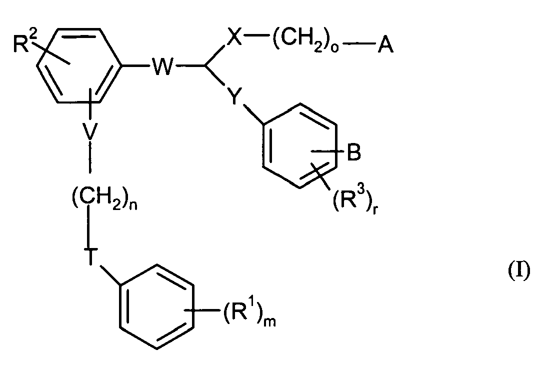 worin
V O bedeutet,
und
n eine ganze Zahl von 1 bis 10 bedeutet,
oder
V fehlt
und
n eine ganze Zahl von 1 bis 3 bedeutet,
T fehlt,
R¹ Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
m 1 oder 2 bedeutet,
R² Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
W CH₂CH₂ oder CH=CH bedeutet, wenn W an dem Phenylring in ortho-Position zu dem Rest V-(CH₂)ₙ-T-Ph-(R¹)ₘ angeordnet ist,
beziehungsweise CH₂CH₂CH₂ oder CH₂CH=CH bedeutet, wenn W an dem Phenylring in meta-Position zu dem Rest V-(CH₂)ₙ-T-Ph-(R¹)ₘ angeordnet ist,
X fehlt, geradkettiges oder verzweigtes Alkylen mit bis zu 6 Kohlenstoffatomen, O, SCH₂ oder S(O)p bedeutet,
worin
p 0, 1 oder 2 bedeutet
o eine ganze Zahl von 1 bis 5 bedeutet
A Tetrazolyl, Tetrazolylmethylen, COOH, CH₂COOH, COOR⁴, CH₂COOR⁵, CONR⁶R⁷ oder CN bedeutet,
worin
R⁴ und R⁵ unabhängig voneinander geradkettiges oder verzweigtes Alkyl mit bis zu 6 Kohlenstoffatomen bedeuten,
R⁶ und R⁷ unabhängig voneinander Wasserstoff, geradkettiges oder verzweigtes Alkyl mit bis zu 6 Kohlenstoffatomen, geradkettiges oder verzweigtes Alkylsulfonyl mit bis zu 12 Kohlenstoffatomen, Arylsulfonyl mit 6 bis 12 Kohlenstoffatomen bedeuten,
oder
R⁶ und R⁷ zusammen mit dem Stickstoffatom, an welches sie gebunden sind, einen 3- bis 8-gliedrigen gesättigten Heterocyclus bilden
Y fehlt, geradkettiges oder verzweigtes Alkylen mit bis zu 6 Kohlenstoffatomen, O, SCH₂ oder S(O)q bedeutet,
worin
q 0, 1 oder 2 bedeutet
B Tetrazolyl, Tetrazolylmethylen, COOH, CH₂COOH, COOR⁸, CH₂COOR⁹, CONR¹⁰R¹¹ oder CN bedeutet,
worin
R⁸ und R⁹ unabhängig voneinander geradkettiges oder verzweigtes Alkyl mit bis zu 6 Kohlenstoffatomen bedeuten,
R¹⁰ und R¹¹ unabhängig voneinander Wasserstoff, geradkettiges oder verzweigtes Alkyl mit bis zu 6 Kohlenstoffatomen, geradkettiges oder verzweigtes Alkylsulfonyl mit bis zu 12 Kohlenstoffatomen, Arylsulfonyl mit 6 bis 12 Kohlenstoffatomen bedeuten,
oder
R¹⁰ und R¹¹ zusammen mit dem Stickstoffatom, an welches sie gebunden sind, einen 3- bis 8-gliedrigen gesättigten Heterocyclus bilden,
R³ Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
r 0, 1 oder 2 bedeutet,
sowie deren Salze und Stereoisomere.

2. Verbindungen nach Anspruch 1,
worin
V O bedeutet,
und
n eine ganze Zahl von 1 bis 10 bedeutet,
oder
V fehlt
und
n eine ganze Zahl von 1 bis 3 bedeutet,
T fehlt,
R¹ Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
m 1 oder 2 bedeutet,
R² Wasserstoff, geradkettiges oder verzweigtes Alkyl oder geradkettiges oder verzweigtes Alkoxy mit jeweils bis zu 12 Kohlenstoffatomen, Halogen, CF₃, OCF₃ oder CN bedeutet,
W CH₂CH₂ oder CH=CH bedeutet und an dem Phenylring in ortho-Position zu dem Rest V-(CH₂)ₙ-T-Ph-(R¹)ₘ angeordnet ist,
beziehungsweise CH₂CH₂CH₂ oder CH₂CH=CH bedeutet, wenn W an dem Phenylring in meta-Position zu dem Rest V-(CH₂)ₙ-T-Ph-(R¹)ₘ angeordnet ist,
X fehlt,
o eine ganze Zahl von 1 bis 4 bedeutet,
A COOH oder COOR⁴ bedeutet,
worin
R⁴ Alkyl mit bis zu 2 Kohlenstoffatomen bedeuten,
Y O, S, SO, SO₂ oder CH₂ bedeutet,
B COOH , COOR⁸ oder CN bedeutet,
worin
R⁸ Alkyl mit bis zu 2 Kohlenstoffatomen bedeutet,
R³ Wasserstoff, geradkettiges oder verzweigtes Alkoxy mit bis zu 6 Kohlenstoffatomen, F, Cl, Br oder I bedeutet,
r 0, 1 oder 2 bedeutet.

3. Verbindungen nach Anspruch 1,
worin
V O bedeutet,
und
n eine ganze Zahl von 1 bis 6 bedeutet,
oder
V fehlt
und
n eine ganze Zahl von 1 bis 3 bedeutet,
T fehlt,
R¹ Wasserstoff, geradkettiges oder verzweigtes Alkyl oder geradkettiges oder verzweigtes Alkoxy mit jeweils bis zu 6 Kohlenstoffatomen, F, Cl, Br oder CF₃ bedeutet,
m 1 oder 2 bedeutet,
R² Wasserstoff oder geradkettiges oder verzweigtes Alkyl mit bis zu 6 Kohlenstoffatomen bedeutet,
W CH₂CH₂ oder CH=CH bedeutet und an dem Phenylring in ortho-Position zu dem Rest V-(CH₂)ₙ-T-Ph-(R¹)ₘ angeordnet ist,
beziehungsweise CH₂CH₂CH₂ oder CH₂CH=CH bedeutet, wenn W an dem Phenylring in meta-Position zu dem Rest V-(CH₂)ₙ-T-Ph-(R¹)ₘ angeordnet ist,
X fehlt,
o eine ganze Zahl von 1 bis 4 bedeutet,
A COOH oder COOR⁴ bedeutet,
worin
R⁴ Alkyl mit bis zu 2 Kohlenstoffatomen bedeuten,
Y O, S oder CH₂ bedeutet,
B COOH , COOR⁸ oder CN bedeutet,
worin
R⁸ Alkyl mit bis zu 2 Kohlenstoffatomen bedeutet,
R³ Wasserstoff, geradkettiges oder verzweigtes Alkoxy mit bis zu 4 Kohlenstoffatomen, Cl oder Br bedeutet,
r 0, 1 oder 2 bedeutet.

4. Verbindungen nach Anspruch 1,
worin
V O bedeutet,
und
n eine ganze Zahl von 1 bis 6 bedeutet,
oder
V fehlt
und
n eine ganze Zahl von 1 bis 3 bedeutet,
T fehlt,
R¹ Wasserstoff, geradkettiges oder verzweigtes Alkyl oder geradkettiges oder verzweigtes Alkoxy mit jeweils bis zu 6 Kohlenstoffatomen, F, Cl, Br oder CF₃ bedeutet,
m 1 oder 2 bedeutet,
R² Wasserstoff oder geradkettiges oder verzweigtes Alkyl mit bis zu 6 Kohlenstoffatomen bedeutet,
W CH₂CH₂ oder CH=CH bedeutet und an dem Phenylring in ortho-Position zu dem Rest V-(CH₂)ₙ-T-Ph-(R¹)ₘ angeordnet ist, beziehungsweise CH₂CH₂CH₂ oder CH₂CH=CH bedeutet, wenn W an dem Phenylring in meta-Position zu dem Rest V-(CH₂)ₙT-Ph-(R¹)ₘ angeordnet ist,
X fehlt,
o eine ganze Zahl von 1 bis 4 bedeutet,
A COOH bedeutet,
Y O, S oder CH₂ bedeutet,
B COOH bedeutet,
R³ Wasserstoff, geradkettiges oder verzweigtes Alkoxy mit bis zu 4 Kohlenstoffatomen, Cl oder Br bedeutet,
r 0, 1 oder 2 bedeutet.

5. Verfahren zur Herstellung der Verbindungen der Formel (I) 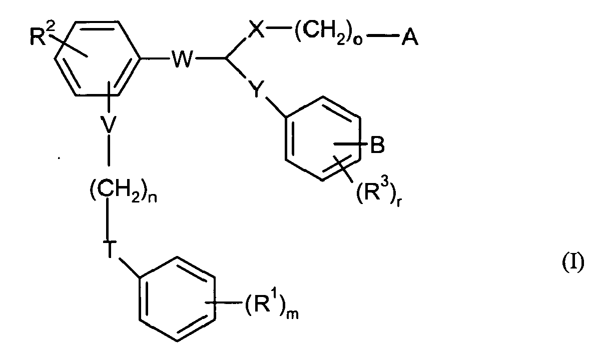 worin
R¹, R², R³, A,B, T, V, W, X, Y, m, n, o und r die in Anspruch 1 angegebene Bedeutung haben,
umfassend
[α] die Umsetzung von Aldehyden der allgemeinen Formel (II) 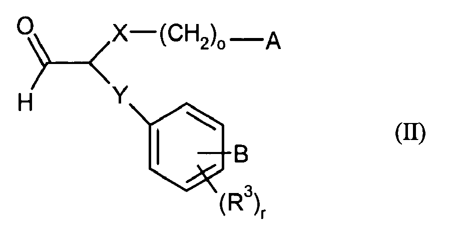 worin
R³, A, B, X, Y, o und r die in Anspruch 1 angegebene Bedeutung haben, mit der Maßgabe, dass A und B nicht für freie Carbonsäuregruppen stehen dürfen,
mit Phosphorverbindungen der allgemeinen Formel (III) 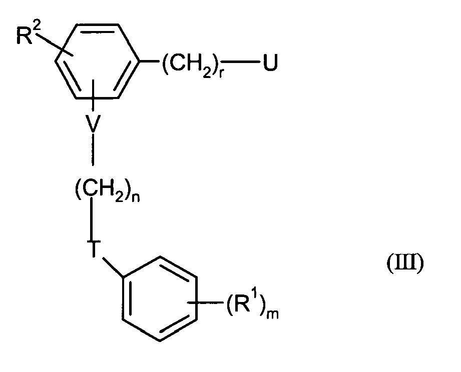 worin
R¹, R², T, V, m und n die in Anspruch 1 angegebenen Bedeutungen haben,
r 1 oder 2 bedeutet, und
U für einen Rest der Formel 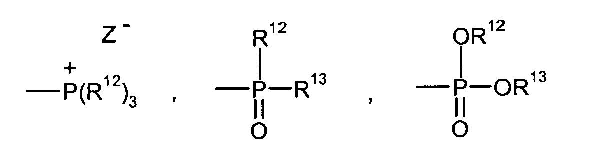 steht, worin
R¹² und R¹³ unabhängig voneinander geradkettiges oder verzweigtes Alkyl mit bis zu 12 Kohlenstoffatomen oder Phenyl bedeuten, und
Z ein Halogenidanion oder Tosylatanion bedeutet,
in inerten Lösungsmitteln in Gegenwart einer Base,
und gegebenenfalls die anschließende teilweise oder vollständige Hydrolyse der Reste A und B zu freien Carbonsäuregruppen;
oder
[β] die Umsetzung von Aldehyden der Formel (i) 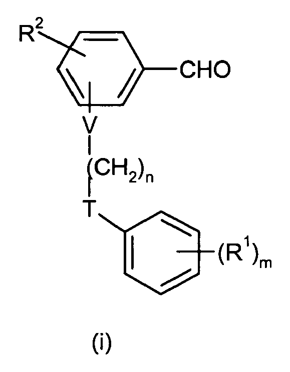 worin
R¹, R², T, V, m und n die in Anspruch 1 angegebenen Bedeutungen haben,
mit Phosphorverbindungen der Formel (ii) 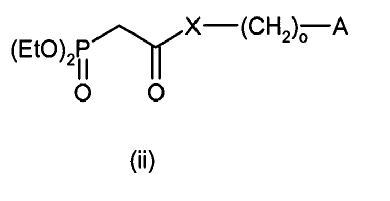 worin
X, o und A die in Anspruch 1 angegebenen Bedeutungen haben,
zu Verbindungen der Formel (iii) 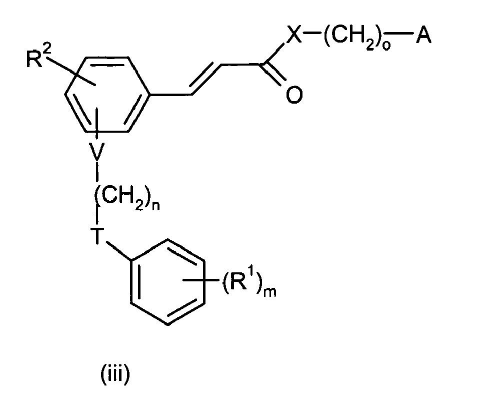 worin
R¹, R², T, V, m, n, X, o und A die in Anspruch 1 angegebenen Bedeutungen haben,
und die anschließende Überführung der Verbindungen der Formel (iii) in Verbindungen der Formel (iv) 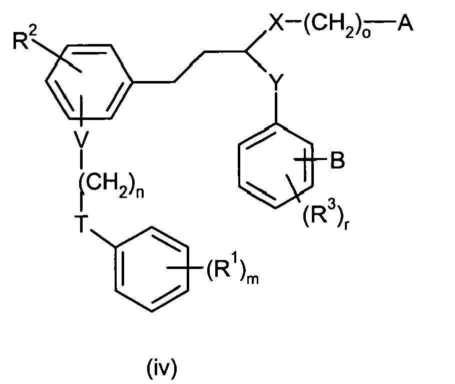 worin
R¹, R², T, V, m, n, X, o, r, A, B und R³ die in Anspruch 1 angegebenen Bedeutungen haben,
Y O, SCH₂ oder S bedeutet,
durch aufeinanderfolgende Reduktion der Carbonylgruppe und der Alkengruppe und anschließende Substitution der durch Reduktion der Carbonylgruppe erzeugten Hydroxygruppe mit Alkoholen oder Thiolen sowie gegebenenfalls anschließende Oxidation zu den entsprechenden Sulfoxid- oder Sulfonverbindungen.

6. Arzneimittel enthaltend mindestens eine Verbindung der allgemeinen Formel (I) gemäß einem der vorhergehenden Ansprüche 1 bis 4.

7. Verwendung von Verbindungen der Formel (I) gemäß einem der vorhergehenden Ansprüche 1 bis 4 zur Herstellung eines Arzneimittels zur Behandlung von Herz-Kreislauf-Erkrankungen.

8. Verwendung von Verbindungen der allgemeinen Formel (I) gemäß einem der vorhergehenden Ansprüche 1 bis 4 zur Herstellung von Arzneimitteln zur Behandlung von Angina pectoris, Ischämien und Herzinsuffizienz.

9. Verwendung von Verbindungen der allgemeinen Formel (I) gemäß einem der vorhergehenden Ansprüche 1 bis 4 zur Herstellung von Arzneimitteln zur Behandlung von Hypertonie, thromboembolischen Erkrankungen, Arteriosklerose und venösen Erkrankungen.

10. Verwendung von Verbindungen der allgemeinen Formel (I) gemäß einem der vorhergehenden Ansprüche 1 bis 4 zur Herstellung von Arzneimitteln zur Behandlung von fibrotischen Erkrankungen.

11. Verwendung nach Anspruch 10, **dadurch gekennzeichnet, dass** die fibrotische Erkrankung Leberfibrose ist.
